# EUROPEAN PATENT SPECIFICATION

(11) **EP 0 323 441 B1**
(45) Date of publication and mention of the grant of the patent: **24.04.1996**
(21) Application number: 89102313.7
(22) Date of filing: 29.10.1985
(51) Int. Cl.: C07H 19/073, A61K 31/70

(54) **5-Fluorouracil derivatives**
5-Fluorouracil-Derivate
Dérivés de 5-fluorouracile

(30) Priority: 30.10.1984 JP 229938/84; 30.11.1984 JP 254587/84; 17.01.1985 JP 7190/85; 25.03.1985 JP 59788/85; 09.05.1985 JP 98295/85; 30.08.1985 JP 192582/85; 03.09.1985 JP 195223/85
(43) Date of publication of application: 05.07.1989
(62) Divisional of application: 85113724.0
(73) Proprietor: OTSUKA PHARMACEUTICAL CO., LTD., Chiyoda-ku Tokyo 101 (JP)
(72) Inventor: Fujii, Setsuro Aig de Blanche 703, Nakagyo-ku, Kyoto-shi Kyoto-fu (JP)
(74) Representative: VOSSIUS & PARTNER

(56) References cited:
- EP-A- 0 009 882
- EP-A- 0 129 984
- FR-A- 2 428 052
- GB-A- 2 066 812
- GB-A- 2 072 164
- CHEMICAL ABSTRACTS, vol. 101, 1984 page 657, abstract no. 55487y, Columbus, Ohio, US; & JP-A-59 29 699 (FUNAI PHARM. IND.) 16-02-1984

## Description

This invention relates to novel 5-fluorouracil derivatives, process for preparing the same and pharmaceutical compositions containing the same.

The 5-fluorouracil derivatives of the invention are novel compounds undisclosed in literature and having antitumor action.

EP-A-0 129 984 relates to 2'-deoxy-5-substituted uridine derivatives wherein the 5-position may be substituted by a fluorine atom, the 3-position may be substituted by a hydrogen atom, a benzoyl group or a tetrahydrofuranyl group and the O-substituents at the sugar ring may be a hydrogen atom, an alkyl, an alkenyl, a substituted benzyl or a phenyl-substituted alkyl group.

The compounds have been used as antitumor agents.

The inventor has carried out research in an attempt to improve the antitumor action of known 5-fluorouracils, 2'-deoxy-5-fluorouridines and related compounds and to render them less toxic, and consequently succeeded in synthesizing a class of novel 5-fluorouracil derivatives and 5-fluorouridine derivatives which are substituted at 3'- or 5'- position with phenyl-lower alkyloxy group optionally having specific substituent(s) and related compounds. The inventor has also found that the compounds thus prepared have an excellent antitumor action and are very useful as antitumor agents.

The present invention provides a compound represented by the formula 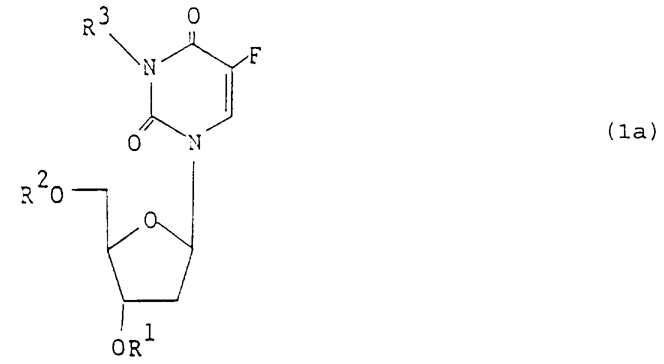 wherein one of R¹ and R is phenyl-lower alkyl group optionally having substituent selected from the group consisting of lower alkyl group, lower alkoxy group, halogen atom, carboxyl group, lower alkoxycarbonyl group and di(lower alkyl)amino group on the phenyl ring, phenyl-lower alkyl group having lower alkylenedioxy or phenyl group as the substituent on the phenyl ring, phenyl-lower alkenyl group or naphthyl-lower alkyl group, the other of R¹ and R is hydrogen atom or acyl group, and R³ is hydrogen atom, acyl group or tetrahydrofuranyl group, with the proviso that at least one of residues R¹, R and R³ is an acyl group, represented by the formula 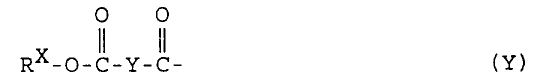 wherein R^{X} is pyridyl groups optionally substituted with 1 to 4 substituents selected from the group consisting of hydroxy group, oxo group, halogen atom, amino group, carboxyl group, cyano group, nitro group, carbymoyl group, lower alkylcarbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group and Y is arylene group.

Preferred compounds of the present invention are represented by the above formula (1a) wherein the acyl group represented by R¹, R and R³ is selected from the group consisting of:
(i) C₁-C₂₀ alkanoyl groups optionally substituted with the substituent selected from the group consisting of halogen atom, hydroxy group, lower alkoxy group, aryloxy group, substituted or unsubstituted aryl group, phenyl-lower alkoxycarbonyl group and lower alkylcarbamoyl group;
(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group;
(iii) 5- or 6- membered unsaturated hetero ring-carbonyl groups having nitrogen atom, sulfur atom or oxygen atom as the hetero atom;
(iv) carbonic acid ester residue such as aryloxycarbonyl groups, straight or branched-chain or cyclic alkoxycarbonyl groups;
(v) substituted or unsubstituted cycloalkylcarbonyl groups;
(vi) lower alkenyl (or lower alkynyl) carbonyl groups;
(vii) lower alkenyl (or lower alkynyl) oxycarbonyl groups; and
(viii) pyridyloxycarbonylarylenecarbonyl groups represented by the formula 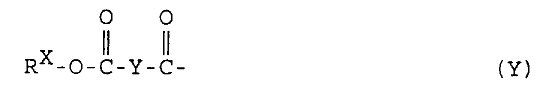 wherein R^{X} is pyridyl groups optionally substituted with 1 to 4 substituents selected from the group consisting of hydroxy group, oxo group, halogen atom, amino group, carboxy group, cyano group, nitro group, carbamoyl group, lower alkylcarbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group having acyl defined above under the items (i) to (vii) in the acyl moiety, and Y is arylene group.

Of interest is also a compound selected from the group consisting of 3'-O-(4-bromobenzyl)-2'-deoxy-5-fluorouridine, 3'-O-(4-chlorobenzyl)-2' -deoxy-5-fluorouridine and 3'-O-(4-chlorobenzyl)-5'-O-acetyl-2'-deoxy-5-fluorouridine.

Throughout the specification, the terms "lower alkyl" and "lower alkoxy" used as such or as contained in various functional groups are intended to exemplify C₁-C₆ straight or branched chain alkyl groups such as methyl, ethyl, propyl, isopropyl, butyl, tert-butyl, pentyl, hexyl, etc; and C₁-C₆ straight or branched chain alkoxy groups, such as methoxy, ethoxy, propoxy, isopropoxy, butoxy, tert-butoxy, pentyloxy, hexyloxy, etc., respectively. Further throughout the specification, the term "halogen" used as it is or as contained in various functional groups is intended to exemplify fluorine, chlorine, bromine, iodine, etc. Also throughout the specification, the term "lower alkylenedioxy" used as such or as contained in various functional groups is meant to exemplify C₁-C₄ alkylenedioxy groups such as methylenedioxy, ethylenedioxy, trimethylenedioxy, tetramethylenedioxy, etc.

With respect to the substituents for substituted phenyl-lower alkyl groups, examples of lower alkyl groups include C₁-C₆ straight or branched chain alkyl groups such as methyl, ethyl, propyl, isopropyl, butyl, tert-butyl, pentyl, hexyl, etc; examples of lower alkoxy groups are C₁-C₆ straight or branched chain alkoxy groups, such as methoxy, ethoxy, propoxy, isopropoxy, butoxy, tert-butoxy, pentyloxy, hexyloxy, etc; examples of halogen atoms are fluorine, chlorine, bromine and iodine; examples of lower alkoxycarbonyl groups are C₂-C₇ straight or branched chain alkoxycarbonyl groups such as methoxycarbonyl, ethoxycarbonyl, propoxycarbonyl, isopropoxycarbonyl, butoxycarbonyl, tert-butoxycarbonyl, pentyloxycarbonyl, hexyloxycarbonyl, etc. Examples of phenyl-lower alkyl groups optionally substituted with the above substituents or with carboxy or di(lower alkyl)amino group on the phenyl ring are phenylalkyl groups in which the phenyl group optionally substituted with one to three of the above substituents is linked with C₁-C₆ alkylene group such as methylene, ethylene, trimethylene, 1-methylethylene, tetramethylene, 2-methyltrimethylene, pentamethylene, hexamethylene, etc. Examples thereof are as follows. benzyl, 2-methylbenzyl, 3-methylbenzyl, 4-methylbenzyl, 2-ethylbenzyl, 3-ethylbenzyl, 4-ethylbenzyl, 2-propylbenzyl, 3-propylbenzyl, 4-propylbenzyl, 2-butylbenzyl, 3-butylbenzyl, 4-butylbenzyl, 2-tert-butylbenzyl, 3-tert-butylbenzyl, 4-tert-butylbenzyl, 2-pentylbenzyl, 3-pentylbenzyl, 4-pentylbenzyl, 2-hexylbenzyl, 3-hexylbenzyl, 4-hexylbenzyl, 2,3-dimethylbenzyl, 2,4-dimethylbenzyl, 2,5-dimethylbenzyl, 2,6-dimethylbenzyl, 3,4-dimethylbenzyl, 3,5-dimethylbenzyl, 2,3,4-trimethylbenzyl, 2,4,5-trimethylbenzyl, 2,3,5-trimethylbenzyl, 2,4,6-trimethylbenzyl, 3,4,5-trimethylbenzyl, 2,3-diethylbenzyl, 2,4-diethylbenzyl, 2,5-diethylbenzyl, 2,6-diethylbenzyl, 2,4,6-triethylbenzyl, 2,4-dipropylbenzyl, 3,4,5-triethylbenzyl, 3-methyl-4-ethylbenzyl, 1-phenylethyl, 2-phenylethyl, 2-phenyl-1-methyl-ethyl, 1-(2-methylphenyl)ethyl, 2-(2-methylphenyl)ethyl, 2-(3-methylphenyl)ethyl, 2-(4-methylphenyl)ethyl, 1-(2,4-dimethylphenyl)ethyl, 2-(2,4-dimethylphenyl)ethyl, 1-(2,4,6-trimethylphenyl)ethyl, 2-(2,4,6-trimethylphenyl)ethyl, 3-phenylpropyl, 3-(4-methylphenyl)propyl, 4-phenylbutyl, 4-(2-methylphenyl)butyl, 5-phenylpentyl, 5-(3-methylphenyl)pentyl, 6-phenylhexyl, 6-(4-methylphenyl)hexyl, 2-methoxybenzyl, 3-methoxybenzyl, 4-methoxybenzyl, 2,3-dimethoxybenzyl, 2,4-dimethoxybenzyl, 2,5-dimethoxybenzyl, 3-methoxy-4-ethoxybenzyl, 2,6-dimethoxybenzyl, 3,4-dimethoxybenzyl, 3,5-dimethoxybenzyl, 2,3,4-trimethoxybenzyl, 2,4,5-trimethoxybenzyl, 2,3,5-trimethoxybenzyl, 2,4,6-trimethoxybenzyl, 3,4,5-trimethoxybenzyl, 2-ethoxybenzyl, 4-propoxybenzyl, 3-butoxybenzyl, 2-tert-butoxybenzyl, 3-pentyloxybenzyl, 4-hexyloxybenzyl, 2,3-diethoxybenzyl, 1-(4-methoxyphenyl)ethyl, 2-(3,4-dimethoxyphenyl)ethyl, 3-(4-methoxyphenyl)propyl, 4-(2-methoxyphenyl)butyl, 5-(4-methoxyphenyl)pentyl, 6-(4-methoxyphenyl)hexyl, 6-(3,4,5-tripentyloxyphenyl)hexyl, 2-fluorobenzyl, 3-fluorobenzyl, 4-fluorobenzyl, 2,3-difluorobenzyl, 2,4-difluorobenzyl, 2,5-difluorobenzyl, 2-fluoro-3-chlorobenzyl, 2-fluoro-3-bromobenzyl, 2,6-difluorobenzyl, 2,3,4-trifluorobenzyl, 2,4,5-trifluorobenzyl, 2,3,5-trifluorobenzyl, 2,4,6-trifluorobenzyl, 3,4,5-trifluorobenzyl, 1-(2-fluorophenyl)ethyl, 2-(2-fluorophenyl)ethyl, 3-(3-fluorophenyl)propyl, 4-(2-fluorophenyl)butyl, 5-(2-fluorophenyl)pentyl, 6-(3-fluorophenyl)hexyl, 2-bromobenzyl, 3-bromobenzyl, 4-bromobenzyl, 2,3-dibromobenzyl, 2-bromo-3-fluorobenzyl, 2-fluoro-4-bromobenzyl, 2,4-dibromobenzyl, 2,5-dibromobenzyl, 2,6-dibromobenzyl, 2,3,4-tribromobenzyl, 2,4,5-tribromobenzyl, 2,3,5-tribromobenzyl, 2,4,6-tribromobenzyl, 3,4,5-tribromobenzyl, 1-(2-bromophenyl)ethyl, 2-(2-bromophenyl)ethyl, 3-(2-bromophenyl)propyl, 4-(3-bromophenyl)butyl, 5-(2-bromophenyl)pentyl, 6-(4-bromophenyl)hexyl, 2-chlorobenzyl, 3-chlorobenzyl, 4-chlorobenzyl, 2,3-dichlorobenzyl, 2,4-dichlorobenzyl, 2,5-dichlorobenzyl, 2,6-dichlorobenzyl, 2-bromo-4-chlorobenzyl, 2-fluoro-4-chlorobenzyl, 2,3,4-trichlorobenzyl, 2,4,5-trichlorobenzyl, 2,3,5-trichlorobenzyl, 2,4,6-trichlorobenzyl, 3,4,5-trichlorobenzyl, 1-(4-chlorophenyl)ethyl, 2-(4-chlorophenyl)ethyl, 3-(2-chlorophenyl)propyl, 4-(4-chlorophenyl)butyl, 5-(3-chlorophenyl)pentyl, 6-(4-chlorophenyl)hexyl, 2-(3,4-dichlorophenyl)ethyl, 2-iodobenzyl, 3-iodobenzyl, 4-iodobenzyl, 3,4-diiodobenzyl, 3,4,5-triiodobenzyl, 2-(3-iodophenyl)ethyl, 6-(2-iodophenyl)hexyl, 2-carboxybenzyl, 3-carboxybenzyl, 4-carboxybenzyl, 2,3-dicarboxybenzyl, 2,4-dicarboxybenzyl, 2,5-dicarboxybenzyl, 2,6-dicarboxybenzyl, 3,4-dicarboxybenzyl, 3,5-dicarboxybenzyl, 2,3,4-tricarboxybenzyl, 2,4,6-tricarboxybenzyl, 3,4,5-tricarboxybenzyl, 1-(4-carboxyphenyl)ethyl, 2-(4-carboxyphenyl)ethyl, 2-(2,6-dicarboxyphenyl)ethyl, 3-(3-carboxyphenyl)propyl, 5-(2,4,6-tricarboxyphenyl)pentyl, 6-(4-carboxyphenyl)hexyl, 6-(3,5-dicarboxyphenyl)hexyl, 4-methoxycarbonylbenzyl, 3-methoxycarbonylbenzyl, 2-methoxycarbonylbenzyl, 4-ethoxycarbonylbenzyl, 3-ethoxycarbonylbenzyl, 2-ethoxycarbonylbenzyl, 4-propoxycarbonylbenzyl, 4-butoxycarbonylbenzyl, 4-pentyloxycarbonylbenzyl, 4-hexyloxycarbonylbenzyl, 3,4-dimethoxycarbonylbenzyl, 2,4-dimethoxycarbonylbenzyl, 3,5-dimethoxycarbonylbenzyl, 2,3,4-trimethoxycarbonylbenzyl, 3,4,5-trimethoxycarbonylbenzyl, 2-(4-methoxycarbonylphenyl)ethyl, 6-(4-methoxycarbonylphenyl)hexyl, 2-(N,N-dimethylamino)benzyl, 3-(N,N-dimethylamino)benzyl, 4-(N,N-dimethylamino)benzyl, 4-(N-methyl- N-ethylamino)benzyl, 2,4-di(N,N-dimethylamino)benzyl, 2,4,6-tri(N,N-dimethylamino)benzyl, etc.

Examples of phenyl-lower alkyl groups having lower alkylenedioxy or phenyl group as the substituent and represented by R¹ and R in the formula (1a) are phenylalkyl groups in which phenyl group substituted with C₁-C₄ alkylenedioxy or phenyl group is linked with C₁-C₆ alkylene group, such as 2,3-methylenedioxybenzyl, 3,4-methylenedioxybenzyl, 2,3-ethylenedioxybenzyl, 3,4-ethylenedioxybenzyl, 3,4-trimethylenedioxybenzyl, 3,4-tetramethylenedioxybenzyl, 1-(3,4-methylenedioxyphenyl)ethyl, 2-(3,4-methylenedioxyphenyl)ethyl, 3-(3,4-methylenedioxyphenyl)propyl, 4-(3,4-methylenedioxyphenyl)butyl, 5-(3,4-methylenedioxyphenyl)pentyl, 6-(3,4-methylenedioxyphenyl)hexyl, 3-(3,4-trimethylenedioxyphenyl)propyl, 2-phenylbenzyl, 3-phenylbenzyl, 4-phenylbenzyl, 2-(3-phenylphenyl)ethyl, 1-(4-phenylphenyl)ethyl, 2-(4-phenylphenyl)ethyl, 3-(4-phenylphenyl)propyl, 4-(4-phenylphenyl)butyl, 5-(4-phenylphenyl)pentyl, 6-(4-phenylphenyl)hexyl, etc.

Examples of phenyl-lower alkenyl groups represented by R¹ and R are phenyl-C₂-C₆ alkenyl groups such as 2-phenylethylenyl, 3-phenyl-1-propenyl, 3-phenyl-2-propenyl, 4-phenyl-1-butenyl, 4-phenyl-2-butenyl, 4-phenyl-3-butenyl, 5-phenyl-4-pentenyl, 6-phenyl-5-hexenyl, etc.

Examples of naphthyl lower alkyl groups represented by R¹ and R are naphthyl-C₁-C₆ alkyl groups such as α-naphthylmethyl, β-naphthylmethyl, 2-(α-naphthyl)ethyl, 3-(β-naphthyl)propyl, 4-(α-naphthyl)butyl, 5-(β-naphthyl)pentyl, 6-(α-naphthyl)hexyl, 3-(α-naphthyl)-2-methylpropyl, 1-(α-naphthyl)ethyl, etc.

Examples of acyl groups disclosed in the specification and claims and especially represented by R¹, R and R³ are various and include the following:
(i) C₁-C₂₀ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.
(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group.
(iii) 5- or 6- membered unsaturated hetero ring-carbonyl groups having nitrogen atom, sulfur atom or oxygen atom as the hetero atom.
(iv) carbonic acid ester residue such as aryloxycarbonyl groups, straight or branched-chain or cyclic alkoxycarbonyl groups.
(v) substituted or unsubstituted cycloalkylcarbonyl groups.
(vi) lower alkenyl (or lower alkynyl)carbonyl groups.
(vii) lower alkenyl (or lower alkynyl)oxycarbonyl groups.
(viii) pyridyloxycarbonylarylenecarbonyl groups represented by the formula 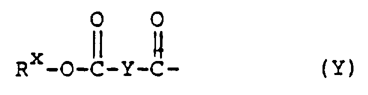 wherein R^{X} is pyridyl groups optionally substituted with 1 to 4 substituents selected from the group consisting of hydroxy group, oxo group, halogen atom, amino group, carboxy group, cyano group, nitro group, carbamoyl group, lower alkyl carbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group having acyl as represented by R¹, R and R³, preferably acyl exemplified in the foregoing items (i) to (vii) in the acyl moiety, and Y is arylene group.

Examples of the acyl groups included in the items (i) to (viii) are as follows.

(i) C₁-C₂₀ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.

C₁-C₂₀ unsubstituted alkanoyl groups such as formyl, acetyl, propionyl, butyryl, isobutyryl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl, eicosanoyl, etc; C₂-C₆ alkanoyl groups substituted with 1 to 3 halogen atoms such as monochloroacetyl, monobromoacetyl, dichloroacetyl, trichloroacetyl, 3-chloropropionyl, 4-chlorobutyryl, 5-chloropentanoyl, 6-chlorohexanoyl, etc; C₂-C₆ alkanoyl groups substituted with hydroxy group such as hydroxyacetyl, 3-hydroxypropionyl, 5-hydroxypentanoyl, 4-hydroxybutanoyl, 6-hydroxyhexanoyl, etc; C₂-C₆ alkanoyl groups substituted with C₁-C₆ alkoxy group such as methoxyacetyl, ethoxyacetyl, 3-propoxypropionyl, 6-hexyloxyhexanoyl, 3-methoxypropionyl, etc; C₂-C₆ alkanoyl groups substituted with phenoxy or naphthyloxy group such as phenoxyacetyl, 2-phenoxypropionyl, 3-phenoxypropionyl, 4-phenoxybutyryl, 5-phenoxypentanoyl, 6-phenoxyhexanoyl, α-naphthyloxyacetyl, etc; C₂-C₆ alkanoyl groups substituted with aryl group optionally substituted with 1 to 3 substituents selected from the group consisting of halogen atom, lower alkyl group, lower alkoxy group, carboxy group, lower alkoxycarbonyl group, nitro group and cyano group on the aryl ring (phenyl ring, naphthyl ring, etc.), such as α-phenylacetyl, 2-phenylpropionyl, 3-phenylpropionyl, 4-phenylbutyryl, 5-phenylpentanoyl, 6-phenylhexanoyl, α-(2-chlorophenyl)acetyl, α-(4-methylphenyl)acetyl, α-(3,4,5-trimethoxyphenyl)acetyl, α-(3,4-dimethoxyphenyl)acetyl, 6-(4-carboxyphenyl)hexanoyl, 4-(4-ethoxycarbonylphenyl)pentanoyl, α-(4-nitrophenyl)acetyl, α-(4-cyanophenyl)acetyl, α-naphthylacetyl, β-naphthylacetyl, etc; C₁-C₂₀ alkanoyl groups substituted with phenyl-C₂-C₇ lower alkoxycarbonyl group such as α-benzyloxycarbonylacetyl, 2-benzyloxycarbonylpropionyl, 3-benzyloxycarbonylpropionyl, 4-benzyloxycarbonylbutyryl, 5-benzyloxycarbonylpentanoyl, 6-benzyloxycarbonylhexanoyl, 3-(α-phenethyloxycarbonyl)propionyl, 3-(β-phenethyloxycarbonyl)propionyl, 5-(benzyloxycarbonyl)hexanoyl, 7-(benzyloxycarbonyl)heptanoyl, 8-(α-phenethyloxycarbonyl)octanoyl, 9-(β-phenethyloxycarbonyl)nonanoyl, 10-(benzyloxycarbonyl)decanoyl, 11-(β-phenethyloxycarbonyl)tridecanoyl, 15-(benzyloxycarbonyl)pentadecanoyl, 17-(benzyloxycarbonyl)heptadecanoyl, 20-(benzyloxycarbonyl)eicosanoyl, etc; C₁-C₂₀ alkanoyl groups substituted with C₂-C₇ lower alkylcarbamoyl group such as methylcarbamoylacetyl, ethylcarbamoylacetyl, propylcarbamoylacetyl, butylcarbamoylacetyl, tert-butylcarbamoylacetyl, pentylcarbamoylacetyl, hexylcarbamoylacetyl, methylcarbamoylpropionyl, ethylcarbamoylbutyryl, propylcarbamoylpentanoyl, ethylcarbamoylhexanoyl, ethylcarbamoylheptanoyl, methylcarbamoyloctanoyl, ethylcarbamoylnonanoyl, methylcarbamoyldecanoyl, methylcarbamoyltridecanoyl, ethylcarbamoylpentadecanoyl, methylcarbamoylheptadecanoyl, methylcarbamoyleicosaonyl, etc;

(ii) aryl-carbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarboxy group, hydroxy group, guanidyl group, phenyl-lower alkoxy group, amino group and amino group substituted with lower alkyl group on the aryl ring.

Aryl-carbonyl groups such as phenylcarbonyl, naphthylcarbonyl, etc. optionally substituted with lower alkylenedioxy group or with 1 to 3 substituents selected from the group consisting of halogen atom, lower alkyl group, lower alkoxy group, carboxy group, lower alkoxycarbonyl groups, nitro group, cyano group, phenyl-lower alkoxycarbonyl group, hydroxy group, guanidyl group, phenyl-lower alkoxy group, amino group and amino group substituted with lower alkyl group, such as benzoyl, α-naphthylcarbonyl, β-naphthylcarbonyl, 2-methylbenzoyl, 3-methylbenzoyl, 4-methylbenzoyl, 2,4-dimethylbenzoyl, 3,4,5-trimethylbenzoyl, 4-ethylbenzoyl, 2-methoxybenzoyl, 3-methoxybenzoyl, 4-methoxybenzoyl, 2,4-dimethoxybenzoyl, 3,4,5-trimethoxybenzoyl, 4-ethoxybenzoyl, 2-methoxy-4-ethoxybenzoyl, 2-propoxybenzoyl, 3-propoxybenzoyl, 4-propoxybenzoyl, 2,4-dipropoxybenzoyl, 3,4,5-tripropoxybenzoyl, 2-carboxybenzoyl, 3-carboxybenzoyl, 4-carboxybenzoyl, 2-chlorobenzoyl, 3-chlorobenzoyl, 4-chlorobenzoyl, 2,3-dichlorobenzoyl, 2,4,6-trichlorobenzoyl, 2-bromobenzoyl, 4-fluorobenzoyl, 2-methoxycarbonylbenzoyl, 3-methoxycarbonylbenzoyl, 4-methoxycarbonylbenzoyl, 2-ethoxycarbonylbenzoyl, 3-ethoxycarbonylbenzoyl, 4-ethoxycarbonylbenzoyl, 2-propoxycarbonylbenzoyl, 3-propoxycarbonylbenzoyl, 4-propoxycarbonylbenzoyl, 2-isopropoxycarbonylbenzoyl, 3-isopropoxycarbonylbenzoyl, 4-isopropoxycarbonylbenzoyl, 2-butoxycarbonylbenzoyl, 3-butoxycarbonylbenzoyl, 4-butoxycarbonylbenzoyl, 2-tert-butoxycarbonylbenzoyl, 3-tert-butoxycarbonylbenzoyl, 4-tert-butoxycarbonylbenzoyl, 2-pentyloxycarbonylbenzoyl, 3-pentyloxycarbonylbenzoyl, 4-pentyloxycarbonylbenzoyl, 2-hexyloxycarbonylbenzoyl, 3-hexyloxycarbonylbenzoyl, 4-hexyloxycarbonylbenzoyl, 3,5-dimethoxycarbonylbenzoyl, 3,4,5-trimethoxycarbonylbenzoyl, β-methyl-α-naphthylcarbonyl, α-methoxy-β-naphthylcarbonyl, β-chloro-α-naphthylcarbonyl, 2-cyanobenzoyl, 4-cyanobenzoyl, 2-nitrobenzoyl, 4-nitrobenzoyl, 2-benzyloxycarbonylbenzoyl, 3-benzyloxycarbonylbenzoyl, 4-benzyloxycarbonylbenzoyl, 3-(α-phenethyloxycarbonyl)benzoyl, 4-(β-phenethyloxycarbonyl)benzoyl, 4-(3-phenylpropoxycarbonyl)benzoyl, 4-(6-phenylhexyloxycarbonyl)benzoyl, 2-hydroxybenzoyl, 3-hydroxybenzoyl, 2,3-dihydroxybenzoyl, 3,4-dihydroxybenzoyl, 3,4,5-trihydroxybenzoyl, 4-hydroxybenzoyl, 2-guanidylbenzoyl, 3-guanidylbenzoyl, 4-guanidylbenzoyl, 2-(benzyloxy)benzoyl, 3-(benzyloxy)benzoyl, 4-(benzyloxy)benzoyl, 2-(α-phenethyloxy)benzoyl, 3-(α-phenethyloxy)benzoyl, 4-(α-phenethyloxy)benzoyl, 2-(β-phenethyloxy)benzoyl, 3-(β-phenethyloxy)benzoyl, 4-(β-phenethyloxy)benzoyl, 4-(3-phenylpropoxy)benzoyl, 4-(4-phenylbutoxy)benzoyl, 4-(5-phenylpentyloxy)benzoyl, 4-(6-phenylhexyloxy)benzoyl, 2-aminobenzoyl, 3-aminobenzoyl, 4-aminobenzoyl, 2-methylaminobenzoyl, 3-methylaminobenzoyl, 4-methylaminobenzoyl, 2-ethylaminobenzoyl, 3-propylaminobenzoyl, 4-butylaminobenzoyl, 3-pentylaminobenzoyl, 4-hexylaminobenzoyl, 2-(N,N-dimethylamino)benzoyl, 3-(N,N-dimethylamino)benzoyl, 4-(N,N-dimethylamino)benzoyl, 3-(N-methyl-N-ethylamino)benzoyl, 4-(N,N-diethylamino)benzoyl, 3-(N,N-dipropylamino)benzoyl, 4-(N,N-dibutylamino)benzoyl, 4-(N,N-dipentylamino)benzoyl, 4-(N,N-dihexylamino)benzoyl, 3-benzyloxycarbonyl-1-naphthylcarbonyl, 6-(β-phenethyloxycarbonyl)-1-naphthylcarbonyl, 4-benzyloxycarbonyl-2-naphthylcarbonyl, 5-(α-phenethyloxycarbonyl)-2-naphthylcarbonyl, 2-hydroxy-1-naphthylcarbonyl, 3-hydroxy-1-naphthylcarbonyl, 4-hydroxy-1-naphthylcarbonyl, 5-hydroxy-l-naphthylcarbonyl, 6-hydroxy-1-naphthylcarbonyl, 7-hydroxy-1-naphtylcarbonyl, 8-hydroxy-1-naphthylcarbonyl, 1-hydroxy-2-naphthylcarbonyl, 4-hydroxy-2-naphthylcarbonyl, 5-hydroxy-2-naphthylcarbonyl, 7-hydroxy-2-naphthylcarbonyl, 2-guanidyl-1-naphtylcarbonyl, 3-guanidyl-1-naphthylcarbonyl, 5-guanidyl-1-naphthylcarbonyl, 6-guanidyl-1-naphthylcarbonyl, 8-guanidyl-1-naphthylcarbonyl, 1-guanidyl-2-naphthylcarbonyl, 4-guanidyl-2-naphthylcarbonyl, 6-guanidyl-2-naphthylcarbonyl, 8-guanidyl-2-naphthylcarbonyl, 2-amino-1-naphthylcarbonyl, 3-amino-1-naphthylcarbonyl, 4-amino-1-naphthylcarbonyl, 6-amino-1-naphthylcarbonyl, 4-amino-2-naphthylcarbonyl, 5-amino-1-naphthylcarbonyl, 7-amino-2-naphthylcarbonyl, 8-amino-2-naphthylcarbonyl, 3-(N,N-dimethylamino)-2-naphthylcarbonyl, 4-(N-methyl-N-ethylamino)-1-naphthylcarbonyl, 6-(N,N-dimethylamino)-1-naphthylcarbonyl, 7- (N-methyl-N-ethylamino)-2-naphthylcarbonyl, 8-(N-methyl-N-ethylamino)-1-naphthylcarbonyl, 3,4-methylenedioxybenzoyl, 3,4-ethylenedioxybenzoyl, 2,3-methylenedioxybenzoyl, etc.

(iii) 5- or 6- membered unsaturated hetero ring-carbonyl groups having nitrogen atom, sulfur atom or oxygen atom as the hetero atom.

thienylcarbonyl, furanylcarbonyl, thiazolylcarbonyl, quinolylcarbonyl, pyrazinylcarbonyl, pyridylcarbonyl, etc., such as 2-thienylcarbonyl, 3-thienylcarbonyl, 2-furanylcarbonyl, 3-furanylcarbonyl, 4-thiazolylcarbonyl, 2-quinolylcarbonyl, 2-pyrazinylcarbonyl, 2-pyridylcarbonyl, 3-pyridylcarbonyl, 4-pyridylcarbonyl, etc.

(iv) carbonic acid ester residues such as aryloxycarbonyl groups, straight or branched-chain or cyclic alkoxycarbonyl groups.

aryloxycarbonyl groups optionally substituted with 1 to 3 substituents selected from the group consisting of halogen atom, lower alkyl group and lower alkoxy group on the aryl ring (phenyl ring, naphthyl ring, etc.), such as phenoxycarbonyl, α-naphthyloxycarbonyl, β-naphthyloxycarbonyl, 2-methylphenoxycarbonyl, 3-methylphenoxycarbonyl, 4-methylphenoxycarbonyl, 2,4-dimethylphenoxycarbonyl, 3,4,5-trimethylphenoxycarbonyl, 4-ethylphenoxycarbonyl, 2-methoxyphenoxycarbonyl, 3-methoxyphenoxycarbonyl, 4-methoxyphenoxycarbonyl, 2,4-dimethoxyphenoxycarbonyl, 3,4,5-trimethoxyphenoxycarbonyl, 4-ethoxyphenoxycarbonyl, 2-propoxyphenoxycarbonyl, 3-propoxyphenoxycarbonyl, 4-propoxyphenoxycarbonyl, 2,4-dipropoxyphenoxycarbonyl, 3,4,5-tripropoxyphenoxycarbonyl, 2-chlorophenoxycarbonyl, 3-chlorophenoxycarbonyl, 4-chlorophenoxycarbonyl, 2,3-dichlorophenoxycarbonyl, 2,4,6-trichlorophenoxycarbonyl, 2-bromophenoxycarbonyl, 4-fluorophenoxycarbonyl, β-methyl-α-naphthyloxycarbonyl, α-methoxy-β-naphthyloxycarbonyl, β-chloro-α-naphthyloxycarbonyl etc; straight or branched-chain or cyclic alkoxycarbonyl groups having 1 to 8 carbon atoms in the alkoxy moiety, such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl, cyclopropyloxycarbonyl, cyclobutyloxycarbonyl, cyclopentyloxycarbonyl, cyclohexyloxycarbonyl, cycloheptyloxycarbonyl, cyclooctyloxycarbonyl, etc.

(v) substituted or unsubstituted cycloalkyl carbonyl groups.

cycloalkylcarbonyl groups optionally substituted with halogen atom, hydroxy group, lower alkoxy group or lower alkyl group and having 3 to 8 carbon atoms in the cycloalkyl ring, such as cyclopropylcarbonyl, cyclobutylcarbonyl, cyclopentylcarbonyl, cyclohexylcarbonyl, cycloheptylcarbonyl, cyclooctylcarbonyl, 2-chlorocyclohexylcarbonyl, 3-hydroxycyclopentylcarbonyl, 3-methylcyclohexylcarbonyl, 4-methoxycyclohexylcarbonyl, etc.

(vi) lower alkenyl (or lower alkynyl)carbonyl groups.

carbonyl groups having C₂-C₆ alkenyl or alkynyl group, such as vinylcarbonyl, allylcarbonyl, 2-butenylcarbonyl, 3-butenylcarbonyl, 1-methylallylcarbonyl, 2-pentenylcarbonyl, 3-hexenylcarbonyl, ethynylcarbonyl, propynylcarbonyl, 2-butynylcarbonyl, 1-methyl-3-pentynylcarbonyl, 4-hexynylcarbonyl, etc.

(vii) lower alkenyl (or lower alkynyl)oxycarbonyl groups.

carbonyl groups having C₂-C₆ alkenyloxy or alkynyloxy group, such as vinyloxycarbonyl, allyloxycarbonyl, 2-butenyloxycarbonyl, 3-butenyloxycarbonyl, 2-hexenyloxycarbonyl, 1-methylallyloxycarbonyl, 2-pentenyloxycarbonyl, 3-hexenyloxycarbonyl, ethynyloxycarbonyl, propynyloxycarbonyl, 2-butynyloxycarbonyl, 1-methyl-3-pentynyloxycarbonyl, 4-hexynyloxycarbonyl, etc.

(viii) pyridyloxycarbonylarylenecarbonyl group represented by the formula (Y)

The substituents on the substituted pyridyl group represented by R^{x} are exemplified below.

Examples of lower alkylcarbamoyl grcups are alkylcarbamoyl groups having one or two C₁-C₆ alkyl groups such as N-methylcarbamoyl, N-ethylcarbamoyl, N-propylcarbamoyl, N-isopropylcarbamoyl, N-butylcarbamoyl, N-tert-butylcarbamoyl, N-pentylcarbamoyl, N-hexylcarbamoyl, N,N-dimethylcarbamoyl, N,N-diethylcarbamoyl, N,N-dipropylcarbamoyl, N-isopropyl-N-methylcarbamoyl, N-ethyl-N-methylcarbamoyl, N-methyl-N-pentyl-carbamoyl, N-propyl-N-pentylcarbamoyl, N,N-dipentylcarbamoyl, N-ethyl-N-hexylcarbamoyl, N-hexyl-N-pentylcarbamoyl, N,N-dihexylcarbamoyl and the like.

Examples of phenylcarbamoyl groups optionally having 1 to 3 substituents selected from the group consisting of halogen, lower alkyl and lower alkoxy on the phenyl ring are carbamoyl groups having one or two phenyl groups which may optionally have 1 to 3 substituents selected from the group consisting of halogen, C₁-C₆ alkoxy and C₁-C₆ alkyl on the phenyl ring such as N-(2-chlorophenyl)carbamoyl, N-(3,5-dichloro)phenylcarbamoyl, N-(3-methoxyphenyl)carbamoyl, N-(4-propoxyphenyl)carbamoyl, N-(2-methylphenyl)carbamoyl, N-(4-ethylphenyl)carbamoyl, N-(3-isopropylphenyl)carbamoyl, N-(4-hexylphenyl)carbamoyl, N-phenylcarbamoyl, N,N-diphenylcarbamoyl and the like.

Examples of lower alkenyl groups are C₂-C₆ alkenyl groups such as vinyl, allyl, 2-butenyl, 3-butenyl, 1-methylallyl, 2-pentenyl, 2-hexenyl and the like.

Examples of lower alkoxycarbonyl groups are carbonyl groups having C₁-C₆ alkoxy group such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl and the like.

Examples of tetrahydrofuranyl groups are 2-tetrahydrofuranyl, 3-tetrahydrofuranyl and the like.

Examples of lower alkoxy-lower alkyl groups are alkoxyalkyl groups in which the alkoxy moiety and alkyl moiety each have 1 to 6 carbon atoms, such as methoxymethyl, 3-methoxypropyl, 4-ethoxybutyl, 6-propoxyhexyl, 5-isopropoxypentyl, 1,1-dimethyl-2-butoxyethyl, 2-methyl-3-tert-butoxypropyl, 2-pentyloxyethyl, 2-hexyloxyethyl and the like.

Examples of phenyl-lower alkoxy-lower alkyl groups are alkoxyalkyl groups which are substituted with phenyl group and in which the alkoxy moiety and alkyl moiety each have 1 to 6 carbon atoms, such as benzyloxymethyl, 2-benzyloxyethyl, 5-(2-phenylpentyloxy)pentyl, 6-benzyloxyhexyl, 4-hexyloxyhexyl, phenylmethoxymethyl, 2-phenylethoxymethyl, 2-(phenylmethoxy)ethyl, 2-(phenylmethoxy) propyl, 4-(phenylmethoxy)butyl, 5-(phenylmethoxy)pentyl, 6-(phenylmethoxy)hexyl, 2-(2-phenylethoxy)ethyl, 2-(4-phenylbutoxy)ethyl, 4-(4-phenylbutoxy)butyl, 6-phenylmethoxyhexyl and the like.

Examples of lower alkoxycarbonyl-lower alkylcarbamoyl groups are carbamoyl groups substituted with one alkoxycarbonylalkyl group in which the alkoxy moiety and the alkyl moiety each have 1 to 6 carbon atoms, such as methoxycarbonylmethylcarbamoyl, 3-methoxycarbonylpropylcarbamoyl, ethoxycarbonylmethylcarbamoyl, propoxycarbonylmethylcarbamoyl, isopropoxycarbonylmethylcarbamoyl, butoxycarbonylmethylcarbamoyl, tert-butoxycarbonylmethylcarbamoyl, pentyloxycarbonylmethylcarbamoyl, hexyloxycarbonylmethylcarbamoyl, 2-methoxycarbonylethylcarbamoyl, 2-methoxycarbonyl-1-methyl-ethylcarbamoyl, 4-methoxycarbonylbutylcarbamoyl, 2-methoxycarbonyl-1,1-dimethylethylcarbamoyl, 5-methoxycarbonylpentylcarbamoyl, 6-methoxycarbonylhexylcarbamoyl, 2-ethoxycarbonylethylcarbamoyl, 4-ethoxycarbonylbutylcarbamoyl, 6-propoxycarbonylhexylcarbamoyl, 5-isopropoxycarbonylpentylcarbamoyl, 1,1-dimethyl-2-butoxycarbonylethylcarbamoyl, 2-methyl-3-tert-butoxycarbonylpropylcarbamoyl, 2-pentyloxycarbonylethylcarbamoyl, hexyloxycarbonylethylcarbamoyl and the like.

Examples of carboxy-lower alkylcarbamoyl groups are carboxy-C₁-C₆ alkylcarbamoyl groups, such as N-(carboxymethyl)carbamoyl, N-(2-carboxyethyl)carbamoyl, N-(3-carboxypropyl)carbamoyl, N-(2-methyl-2-carboxyethyl)carbamoyl, N-(4-carboxybutyl)carbamoyl, N-(2-methyl-3-carboxypropyl)carbamoyl, N-(2,2-dimethyl-2-carboxyethyl)carbamoyl, N-(5-carboxypentyl)carbamoyl, N-(6-carboxyhexyl)carbamoyl and the like.

Examples of tetrahydropyranyl groups are 2-tetrahydropyranyl, 3-tetrahydropyranyl, 4-tetrahydropyranyl and the like.

Examples of lower alkylthio-lower alkyl groups are C₁-C₆ alkylthio-C₁-C₆ alkyl groups, such as methylthiomethyl, ethylthiomethyl, propylthiomethyl, butylthiomethyl, tert-butylthiomethyl, pentylthiomethyl, hexylthiomethyl, methylthioethyl, methylthiopropyl, methylthiobutyl, methylthiopentyl, methylthiohexyl, ethylthioethyl, ethylthiobutyl, propylthiohexyl and the like.

The pyridine ring is preferably substituted at any of the 2- to 6-positions with one to four of the substituents selected from the group consisting of the hydroxy, halogen, amino, carboxyl, cyano, nitro, oxo, lower alkyl, lower alkenyl, lower alkoxycarbonyl, carbamoyl and acyloxy among the substituents exemplified above, or preferably substituted at the 1-position with the carbamoyl, lower alkylcarbamoyl, phenylcarbamoyl optionally having 1 to 3 substituents selected from the group consisting of halogen, lower alkoxy and lower alkyl on the phenyl ring, tetrahydrofuranyl, phthalidyl, lower alkoxy-lower alkyl, phenyl-lower alkoxy-lower alkyl or lower alkoxycarbonyl-lower alkylcarbamoyl group among the substituents exemplified above.

Preferable examples of the group of the formula (Y) are those represented by the formula 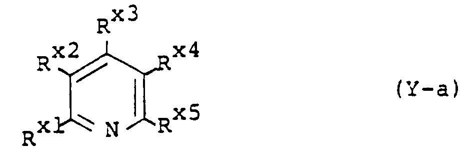 wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy; when at least one of R^{x1}, R^{x3} and R^{x5} is free hydroxy, the structure of 1-position on the pyridine ring can be 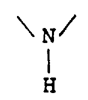 due to the keto-enol tautomerism, said hydrogen attached to nitrogen being optionally substituted with a substituent selected from the group consisting of lower alkyl, tetrahydrofuranyl, tetrahydropyranyl, lower alkoxy-lower alkyl, phthalidyl, carbamoyl, lower alkoxycarbonyl-lower alkylcarbamoyl, phenyl-lower alkoxy-lower alkyl, phenylcarbamoyl which may have 1 to 3 substituents selected from the group consisting of halogen, lower alkoxy and lower alkyl on the phenyl ring, lower alkylcarbamoyl, carboxy-lower alkylcarbamoyl, lower alkylthio-lower alkyl and lower alkenyl, provided that at least one of R^{x1}, R^{x3} and R^{x5} represents hydroxy group and that the hydrogen of one hydroxyl group represented by R^{x1}, R^{x3} or R^{x5} is substituted with a group 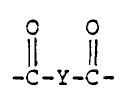 wherein Y is as defined above.

More preferable groups of the formula (Y) are those represented by the formula (Y-a) wherein R^{x4} is halogen, amino, carboxyl, carbamoyl, cyano, nitro, lower alkyl, lower alkenyl or lower alkoxycarbonyl.

The most preferable groups of the formula (Y) are those represented by the formula (Y-a) wherein R^{x4} is halogen or cyano.

Examples of the pyridyl groups represented by R^{x} and optionally having the above various substituents are as follows.

2-pyridyl, 3-pyridyl, 4-pyridyl, 2-hydroxy-3-pyridyl, 2-hydroxy-4-pyridyl, 2-hydroxy-5-pyridyl, 2-hydroxy-6-pyridyl, 2-hydroxy-5-chloro-3-pyridyl, 2-hydroxy-5-chloro-4-pyridyl, 4-hydroxy-5-chloro-2-pyridyl, 2-hydroxy-5-chloro-6-pyridyl, 2-hydroxy-5-fluoro-4-pyridyl, 4-hydroxy-5-fluoro-2-pyridyl, 2-hydroxy-5-fluoro-6-pyridyl, 2-hydroxy-5-bromo-4-pyridyl, 4-hydroxy-5-bromo-2-pyridyl, 2-hydroxy-5-bromo-6-pyridyl, 2-hydroxy-5-iodo-4-pyridyl, 4-hydroxy-5-iodo-2-pyridyl, 2-hydroxy-5-iodo-6-pyridyl, 2-hydroxy-3-bromo-4-pyridyl, 4-hydroxy-3-bromo-2-pyridyl, 2-hydroxy-3-chloro-4-pyridyl, 4-hydroxy-3-chloro-2-pyridyl, 2-hydroxy-3-chloro-6-pyridyl, 2-hydroxy-4-chloro-6-pyridyl, 1-(2-tetrahydrofuranyl)-1,2-dihydro-2-oxo-3-pyridyl, 1-(2-tetrahydrofuranyl)-1,2-dihydro-2-oxo-4-pyridyl, 1-(2-tetrahydrofuranyl)-1,2-dihydro-2-oxo-5-pyridyl, 1-(2-tetrahydrofuranyl)-1,2-dihydro-2-oxo-6-pyridyl, 1-(3-tetrahydrofuranyl)-1,2-dihydro-2-oxo-4-pyridyl, 1-(2-tetrahydrofuranyl)-1,2-dihydro-2-oxo-5-chloro-4-pyridyl, 1-(2-tetrahydrofuranyl)-1,2-dihydro-2-oxo-5-chloro-6-pyridyl, 1-(2-tetrahydrofuranyl)-1,2-dihydro-2-oxo-5-fluoro-4-pyridyl, 1-(2-tetrahydrofuranyl)-1,2-dihydro-2-oxo-5-bromo-4-pyridyl, 1-(2-tetrahydrofuranyl)-1,2-dihydro-2-oxo-5-iodo-4-pyridyl, 1-(3-tetrahydrofuranyl)-1,2-dihydro-2-oxo-5-chloro-4-pyridyl, 1-(2-tetrahydrofuranyl)-1,2-dihydro-2-oxo-3-chloro-4-pyridyl, 1-ethoxymethyl-1,2-dihydro-2-oxo-3-pyridyl, 1-ethoxymethyl-1,2-dihydro-2-oxo-4-pyridyl, 1-ethoxymethyl-1,2-dihydro-2-oxo-5-pyridyl, 1-ethoxymethyl-1,2-dihydro-2-oxo-6-pyridyl, 1-methoxymethyl-1,2-dihydro-2-oxo-4-pyridyl, 1-(2-methoxyethyl)-1,2-dihydro-2-oxo-4-pyridyl, 1-ethoxymethyl-1,2-dihydro-2-oxo-5-chloro-3-pyridyl, 1-ethoxymethyl-1,2-dihydro-2-oxo-5-chloro-4-pyridyl, 1-ethoxymethyl-1,2-dihydro-2-oxo-5-chloro-6-pyridyl, 1-ethoxymethyl-1,2-dihydro-2-oxo-5-fluoro-4-pyridyl, 1-ethoxymethyl-1,2-dihydro-2-oxo-5-bromo-4-pyridyl, 1-ethoxymethyl-1,2-dihydro-2-oxo-5-iodo-4-pyridyl, 1-methoxymethyl-1,2-dihydro-2-oxo-5-chloro-4-pyridyl, 1-(2-methoxyethyl)-1,2-dihydro-2-oxo-5-chloro-4-pyridyl, 1-(2-ethoxymethyl)-1,2-dihydro-2-oxo-3-chloro-4-pyridyl, 2-acetyloxy-5-chloro-4-pyridyl, 2-acetyloxy-5-fluoro-4-pyridyl, 2-acetyloxy-5-bromo-4-pyridyl, 2-acetyloxy-5-iodo-4-pyridyl, 2-acetyloxy-5-methyl-4-pyridyl, 2-acetyloxy-5-cyano-4-pyridyl, 2-acetyloxy-5-nitro-4-pyridyl, 2-acetyloxy-5-carboxy-4-pyridyl, 2-propanoyloxy-5-chloro-4-pyridyl, 2-butanoyloxy-5-chloro-4-pyridyl, 2-isobutanoyloxy-5-chloro-4-pyridyl, 2-pentanoyloxy-5-chloro-4-pyridyl, 2-hexanoyloxy-5-chloro-4-pyridyl, 3-acetyloxy-5-chloro-4-pyridyl, 2-acetyloxy-3-chloro-4-pyridyl, 4-acetyloxy-5-chloro-2-pyridyl, 4-acetyloxy-5-fluoro-2-pyridyl, 4-acetyloxy-5-bromo-2-pyridyl, 4-acetyloxy-5-iodo-2-pyridyl, 4-acetyloxy-5-methyl-2-pyridyl, 4-acetyloxy-5-cyano-2-pyridyl, 4-acetyloxy-5-nitro-2-pyridyl, 4-acetyloxy-5-carboxy-2-pyridyl, 6-acetyloxy-5-chloro-2-pyridyl, 4-propanoyloxy-5-chloro-2-pyridyl, 4-butanoyloxy-5-chloro-2-pyridyl, 4-isobutanoyloxy-5-chloro-2-pyridyl, 4-pentanoyloxy-5-chloro-2-pyridyl, 4-hexanoyloxy-5-chloro-2-pyridyl, 4-acetyloxy-3-chloro-2-pyridyl, 3-acetyloxy-5-chloro-2-pyridyl, 2-acetyloxy-4-pyridyl, 2-propanoyloxy-4-pyridyl, 2-hexanoyloxy-4-pyridyl, 4-acetyloxy-2-pyridyl, 4-propanoyloxy-2-pyridyl, 4-hexanoyloxy-2-pyridyl, 2-methoxycarbonyloxy-5-chloro-4-pyridyl, 2-methoxycarbonyloxy-6-chloro-4-pyridyl, 2-ethoxycarbonyloxy-3-chloro-4-pyridyl, 2-ethoxycarbonyloxy-5-chloro-4-pyridyl, 2-ethoxycarbonyloxy-5-fluoro-4-pyridyl, 2-ethoxycarbonyloxy-5-bromo-4-pyridyl, 2-ethoxycarbonyloxy-6-chloro-4-pyridyl, 2-ethoxycarbonyloxy-5-chloro-3-pyridyl, 2-ethoxycarbonyloxy-5-chloro-6-pyridyl, 2-propoxycarbonyloxy-5-chloro-4-pyridyl, 2-hexyloxycarbonyloxy-5-chloro-4-pyridyl, 2-benzoyloxy-4-pyridyl, 3-benzoyloxy-4-pyridyl, 4-benzoyloxy-2-pyridyl, 3-benzoyloxy-2-pyridyl, 2-(2-methylbenzoyl)oxy-4-pyridyl, 2-(3-methylbenzoyl)oxy-4-pyridyl, 2-(4-methylbenzoyl)oxy-4-pyridyl, 2-(4-ethylbenzoyl)oxy-4-pyridyl, 2-(2-chlorobenzoyl)oxy-4-pyridyl, 2-(3-chlorobenzoyl)oxy-4-pyridyl, 2-(4-chlorobenzoyl)oxy-4-pyridyl, 2-(4-fluorobenzoyl)oxy-4-pyridyl, 2-(4-tert-butylbenzoyl)oxy-4-pyridyl, 2-(4-hexylbenzoyl)oxy-4-pyridyl, 4-(2-methylbenzoyl)oxy-2-pyridyl, 4-(4-ethylbenzoyl)oxy-2-pyridyl, 4-(3-chlorobenzoyl)oxy-2-pyridyl, 4-(4-fluorobenzoyl)oxy-2-pyridyl, 4-(4-tert-butylbenzoyl)oxy-2-pyridyl, 2-benzoyloxy-5-chloro-4-pyridyl, 2-benzoyloxy-5-fluoro-4-pyridyl, 2-benzoyloxy-5-bromo-4-pyridyl, 2-benzoyloxy-5-iodo-4-pyridyl, 2-benzoyloxy-5-methyl-4-pyridyl, 2-benzoyloxy-5-cyano-4-pyridyl, 2-benzoyloxy-5-nitro-4-pyridyl, 2-benzoyloxy-5-carboxy-4-pyridyl, 3-benzoyloxy-5-chloro-4-pyridyl, 2-benzoyloxy-3-chloro-4-pyridyl, 2-(2-methylbenzoyl)oxy-5-chloro-4-pyridyl, 2-(3-methylbenzoyl)oxy-5-chloro-4-pyridyl, 2-(4-methylbenzoyl)oxy-5-chloro-4-pyridyl, 2-(3,4,5-trimethylbenzoyl)oxy-5-chloro-4-pyridyl, 2-(4-ethylbenzoyl)oxy-5-chloro-4-pyridyl, 2-(4-tert-butylbenzoyl)oxy-5-fluoro-4-pyridyl, 2-(4-hexylbenzoyl)oxy-5-bromo-4-pyridyl, 2-(2-chlorobenzoyl)oxy-5-chloro-4-pyridyl, 2-(3-chlorobenzoyl)oxy-5-chloro-4-pyridyl, 2-(4-chlorobenzoyl)oxy-5-chloro-4-pyridyl, 2-(4-fluorobenzoyl)oxy-5-chloro-4-pyridyl, 2-(2,4-dichlorobenzoyl)oxy-5-chloro-4-pyridyl, 2-(3,4,5-trichlorobenzoyl)oxy-5-chloro-4-pyridyl, 2-(2-methoxybenzoyl)oxy-5-chloro-4-pyridyl, 2-(3-methoxybenzoyl)oxy-5-chloro-4-pyridyl, 2-(4-methoxybenzoyl)oxy-5-chloro-4-pyridyl, 2-(3,4,5-trimethoxybenzoyl)oxy-5-chloro-4-pyridyl, 4-benzoyloxy-5-chloro-2-pyridyl, 4-benzoyloxy-5-fluoro-2-pyridyl, 4-benzoyloxy-5-bromo-2-pyridyl, 4-benzoyloxy-5-iodo-2-pyridyl, 4-benzoyloxy-5-methyl-2-pyridyl, 4-benzoyloxy-5-cyano-2-pyridyl, 4-benzoyloxy-5-nitro-2-pyridyl, 4-benzoyloxy-5-carboxy-2-pyridyl, 3-benzoyloxy-5-chloro-2-pyridyl, 6-benzoyloxy-5-chloro-2-pyridyl, 6-(2,4-dichlorobenzoyloxy)-3-cyano-2-pyridyl, 6-(3,4,5-trimethoxybenzoyloxy)-3-cyano-2-pyridyl, 6-(4-fluorobenzoyloxy)-3-chloro-2-pyridyl, 6-benzoyloxy-3-cyano-2-pyridyl, 4-(4-methylbenzoyl)oxy-5-chloro-2-pyridyl, 4-(2,4-dimethylbenzoyl)oxy-5-chloro-2-pyridyl, 4-(3,4,5-trimethylbenzoyl)oxy-5-chloro-2-pyridyl, 4-(2-chlorobenzoyl)oxy-5-chloro-2-pyridyl, 4-(2,4-dichlorobenzoyl)oxy-5-chloro-2-pyridyl, 4-(4-methoxybenzoyl)oxy-5-chloro-2-pyridyl, 1-phthalidyl-1,2-dihydro-2-oxo-3-pyridyl, 1-phthalidyl-1,2-dihydro-2-oxo-4-pyridyl, 1-phthalidyl-1,2-dihydro-2-oxo-5-pyridyl, 1-phthalidyl-1,2-dihydro-2-oxo-6-pyridyl, 1-carbomethoxymethylcarbamoyl-1,2-dihydro-2-oxo-3-pyridyl, 1-carbomethoxymethylcarbamoyl-1,2-dihydro-2-oxo-4-pyridyl, 1-carbomethoxymethylcarbamoyl-1,2-dihydro-2-oxo-5-pyridyl, 1-carbomethoxymethylcarbamoyl-1,2-dihydro-2-oxo-6-pyridyl, 1-carboethoxymethylcarbamoyl-1,2-dihydro-2-oxo-4-pyridyl, 2-hydroxy-5-methyl-3-pyridyl, 2-hydroxy-5-methyl-4-pyridyl, 2-hydroxy-5-methyl-6-pyridyl, 2-hydroxy-5-ethyl-4-pyridyl, 2-hydroxy-3-methyl-4-pyridyl, 2-hydroxy-3-cyano-4-pyridyl, 2-hydroxy-3-cyano-5-pyridyl, 2-hydroxy-3-cyano-6-pyridyl, 2-hydroxy-5-cyano-6-pyridyl, 2-hydroxy-3-nitro-4-pyridyl, 2-hydroxy-3-nitro-5-pyridyl, 2-hydroxy-5-nitro-6-pyridyl, 2-hydroxy-6-nitro-4-pyridyl, 2-hydroxy-5-carboxy-3-pyridyl, 2-hydroxy-5-carboxy-4-pyridyl, 2-hydroxy-5-carboxy-6-pyridyl, 2-hydroxy-3-carboxy-4-pyridyl, 2-hydroxy-5-ethoxycarbonyl-3-pyridyl, 2-hydroxy-5-ethoxycarbonyl-4-pyridyl, 2-hydroxy-5-ethoxycarbonyl-6-pyridyl, 2-hydroxy-3-ethoxycarbonyl-4-pyridyl, 2-hydroxy-3,5-dichloro-4-pyridyl, 2-hydroxy-3,5-dichloro-6-pyridyl, 2-hydroxy-3,5-dibromo-4-pyridyl, 2-hydroxy-3,5-dibromo-6-pyridyl, 2-hydroxy-3-amino-4-pyridyl, 2-hydroxy-3-amino-5-pyridyl, 2-hydroxy-3-amino-6-pyridyl, 2-hydroxy-3-carbamoyl-4-pyridyl, 2-hydroxy-3-carbamoyl-5-pyridyl, 2-hydroxy-3-carbamoyl-6-pyridyl, 2,4-dihydroxy-6-pyridyl, 2,6-dihydroxy-4-pyridyl, 1,2-dihydro-2-oxo-4-pyridyl, 1,6-dihydro-6-oxo-2-pyridyl, 1-(benzyloxymethyl)-5-chloro-1,2-dihydro-2-oxo-4-pyridyl, 1-(benzyloxymethyl) -5-fluoro-1,2-dihydro-2-oxo-6-pyridyl, 1-(3-phenethyloxymethyl)-5-brono-1,2-dihydro-2-oxo-4-pyridyl, 1-(2-benzyloxyethyl)-5-chloro-1,2-dihydro-2-oxo-4-pyridyl, 5-chloro-4-[p-(N,N-dimethylamino)benzoyloxy]-2-pyridyl, 5-bromo-4-[p-(N,N-dimethylamino)benzoyloxy]-2-pyridyl, 5-chloro-4-[p-(N,N-dimethylamino)benzoyloxy]-3-pyridyl, 5-chloro-4-[p-(N-methyl-N-ethylamino)benzoyloxy]-2-pyridyl, 5-fluoro-4-[o-(N,N-dimethylamino)benzoyloxy]-2-pyridyl, 5-fluoro-4-hexanoyloxy-2-pyridyl, 5-bromo-4-hexanoyloxy-2-pyridyl, 5-iodo-4-hexanoyloxy-2-pyridyl, 5-fluoro-4-octadecanoyloxy-2-pyridyl, 5-chloro-4-octadecanoyloxy-2-pyridyl, 5-bromo-4-octadecanoyloxy-2-pyridyl, 5-iodo-4-octadecanoyloxy-2-pyridyl, 5-fluoro-4-(2-furoyloxy)-2-pyridyl, 5-chloro-4-(2-furoyloxy)-2-pyridyl, 5-bromo-4-(2-furoyloxy)-2-pyridyl, 5-iodo-4-(2-furoyloxy)-2-pyridyl, 5-chloro-4-(3-furoyloxy)-2-pyridyl, 5-bromo-4-(3-furoyloxy)-2-pyridyl, 5-fluoro-4-(2-thenoyloxy)-2-pyridyl, 5-chloro-4-(2-thenoyloxy)-2-pyridyl, 5-bromo-4-(2-thenoyloxy)-2-pyridyl, 5-iodo-4-(2-thenoyloxy)-2-pyridyl, 5-chloro-4-(3-thenoyloxy)-2-pyridyl, 5-iodo-4-(3-thenoyloxy)-2-pyridyl, 4-(1-naphthoyloxy)-2-pyridyl, 4-(2-naphthoyloxy)-2-pyridyl, 5-(1-naphthoyloxy)-2-pyridyl, 5-(2-naphthoyloxy)-2-pyridyl, 6-(2-naphthoyloxy)-2-pyridyl, 3-cyano-6-(2-thenoyloxy)-2-pyridyl, 3-cyano-6-(3-thenoyloxy)-2-pyridyl, 4-cyano-6-(2-thenoyloxy)-2-pyridyl, 3-cyano-6-(2-furoyloxy)-2-pyridyl and the like.

Arylene groups represented by Y in the pyridyloxycarbonyl-arylene carbonyl group of the formula (Y) include those formed from aromatic hydrocarbons or 5-or 6- membered aromatic heterocycles containing one or two heteroatoms which are the same or different and which is or are selected from the group consisting of nitrogen and oxygen by removing two hydrogen atoms each bonded to the two different carbon atoms. Examples of such arylene groups are phenylene groups such as 1,2-phenylene, 1,3-phenylene, 1,4-phenylene and the like; naphthylene groups such as 1,2-naphthylene, 1,3-naphthylene, 1,4-naphthylene, 1,5-naphthylene, 1,6-naphthylene, 1,7-naphthylene, 1,8-naphthylene and the like; pyridinediyl groups such as 2,4-pyridinediyl, 2,5-pyridinediyl, 2,6-pyridinediyl, 3,4-pyridinediyl, 3,5-pyridinediyl and the like; pyrazinediyl groups such as 2,3-pyrazinediyl, 2,6-pyrazinediyl, 2,5-pyrazinediyl, and the like; furandiyl groups such as 2,3-furandiyl, 3,4-furandiyl, 2,5-furandiyl, and the like; 4-pyridon-1-lower alkyl-diyl groups such as 4-pyridon-1-methyl-2,3-diyl, 4-pyridon-1-methyl-2,5-diyl, 4-pyridon-1-methyl-2,6-diyl and the like.

The compounds of the invention having the substituent of the formula (Y) include keto-enol tautomers. This invention includes these tautomers. The following processes represented by the reaction schemes (a) to (d) may be involved in the preparation of the compounds of the present invention.

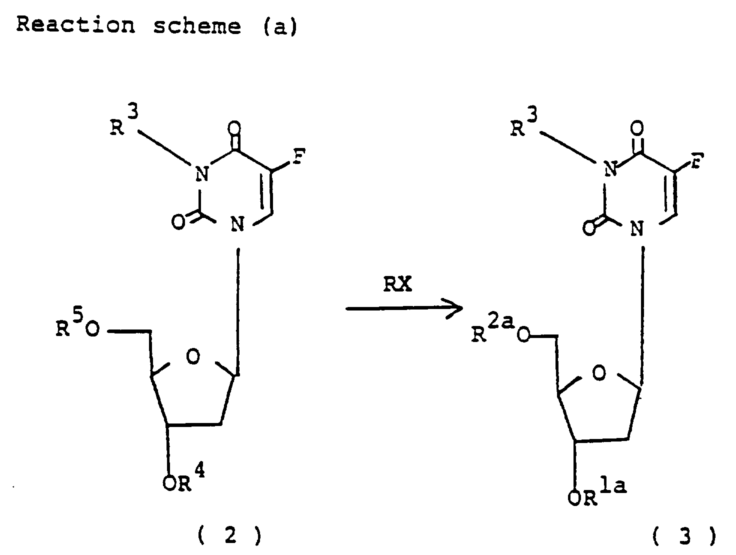

wherein R³ is as defined above, one of R⁴ and R⁵ is a hydrogen atom and the other is a hydrogen atom, acyl or protective group, R is phenyl-lower alkyl which may have substituent selected from the group consisting of lower alkyl, lower alkoxy, halogen atom, carboxyl, lower alkoxycarbonyl and di(lower alkyl)amino group on the phenyl ring, phenyl-lower alkyl having lower alkylenedioxy or phenyl as the substituent, phenyl-lower alkenyl or naphthyl-lower alkyl, one of R^{1a} and R^{2a} is a hydrogen atom or acyl and the other is the same group as R, and X is a halogen atom.

The protective groups represented by R⁴ or R⁵ include the following groups.
(A) Triaryl-substituted methyl groups represented by the formula 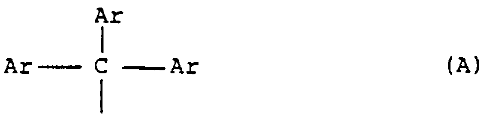 wherein Ar is aryl. Exemplary of such a group is a methyl group substituted with three aryl groups such as phenyls which may have halogen atom, nitro, lower alkyl or lower alkoxy as the substituent.
(B) Cyclic ether residue groups represented by the formula 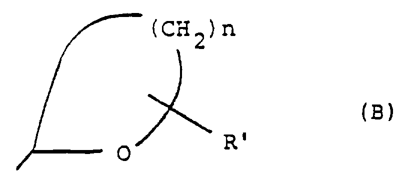 wherein R′ is lower alkyl, and n is 2 or 3. Examples of such groups are 2-tetrahydrofuranyl and 2-tetrahydropyranyl.
(C) Lower alkoxymethyl groups such as methoxymethyl, ethoxymethyl and hexyloxymethyl.
(D) Tri(lower alkyl)silyl groups such as trimethylsilyl and t-butyldimethylsilyl.

The present reaction is conducted by reacting a compound of the formula (2) (hereinafter referred to as "compound (2)") with a phenyl-lower alkyl halide, phenyl-lower alkenyl halide or naphthyl-lower alkyl halide (RX) to substitute the desired group R for the hydrogen atom of the hydroxyl group at the 3′- or 5′-position of the compound (2), followed by a reaction for removing the protective group or acyl when required, to obtain a compound (3).

The reaction for introducing the group R is conducted under the usual reaction conditions for removing a hydrogen halide. The hydrogen halide removing agent to be used can be any of various basic compounds which are generally used for such reactions. Examples of useful compounds are sodium hydroxide, potassium hydroxide, sodium carbonate, potassium carbonate, sodium hydrogencarbonate, potassium hydrogencarbonate, alkali metals such as sodium and potassium, alkali metal hydrides such as sodium hydride and potassium hydride, etc.

The reaction can be conducted in the presence of a solvent or in the absence thereof. Examples of useful solvents are usual inert solvents such as water, ethers such as tetrahydrofuran (THF) and dioxane, aromatic hydrocarbons such as benzene, toluene, xylene and chlorobenzene, ketones such as acetone and methyl ethyl ketone, nitriles such as acetonitrile and propionitrile, etc.

While the ratio of the compound (2) to the phenyl-lower alkyl halide, phenyl-lower alkenyl halide or naphthyl-lower alkyl halide is not limited specifically but is widely variable, usually at least about 1 mole, preferably 1 to 5 moles, of the latter is used per mole of the former. The reaction temperature is not limited specifically either but is widely variable. It is, however, usually 0 to 100°C, preferably room temperature to 80°C. The reaction is carried usually for about 30 minutes to about 64 hours, preferably about 1 to about 5 hours.

When the compound obtained by the above reaction has a protective group at the 3′- or 5′-position, the desired compound (3) can be obtained by subsequently subjecting the product to a reaction for removing the protective group. This reaction is carried out usually in a solvent, using a suitable amount of a catalyst which is commonly used for acid hydrolysis reactions. Examples of suitable catalysts are inorganic acids such as hydrochloric acid, sulfuric acid and perchloric acid, and organic acids including lower alkanoic acids such as formic acid, acetic acid and propionic acid, benzoic acid, organosulfonic acids such as methanesulfonic acid, ethanesulfonic acid, benzenesulfonic acid and 4-methylbenzenesulfonic acid. Examples of useful solvents are usual inert solvents including water, lower alcohols such as methanol, ethanol and isopropanol, ketones such as acetone and methyl ethyl ketone, ethers such as diethyl ether, THF and dioxane, aromatic hydrocarbons such as benzene, toluene, xylene and chlorobenzene, lower alkanoic acids such as acetic acid and propionic acid, and mixtures of such solvents. The reaction temperature is not limited specifically but is suitably determined from a wide range. Usually it is 0 to 100°C, preferably room temperature to about 80°C. The reaction takes about 3 minutes to about 20 hours. The acid is usually used in a catalytic amount to an excessive amount, preferably in an excessive amount.

When the compound (3) prepared by the process of the scheme (a) has acyl at least at one of the 3-, 3'- and 5'-positions, the compound may be subjected to a hydrolysis reaction, whereby one or all of the acyl groups can be converted to hydrogen. The hydrolysis reaction is carried out under the usual conditions for acid or alkali hydrolysis. The catalyst to be used for this reaction can be any one of those which are commonly used for acid or alkali hydrolysis. Typical of these catalysts are basic compounds such as sodium hydroxide, potassium hydroxide and barium hydroxide, and inorganic acids such as hydrochloric acid, sulfuric acid and nitric acid. The amount of catalyst to be used is not limited specifically but is suitably variable over a wide range. Generally, the reaction proceeds advantageously in a solvent. A wide variety of usual inert solvents are usable for this purpose. Examples of useful solvents are water, lower alcohols such as ethanol, methanol and isopropanol, ketones such as acetone and methyl ethyl ketone, and mixtures of such solvents. The reaction temperature is not limited specifically but is suitably determined from a wide range. It is usually 0 to 100°C, preferably room temperature to about 80°C. The reaction takes about 30 minutes to about 10 hours.

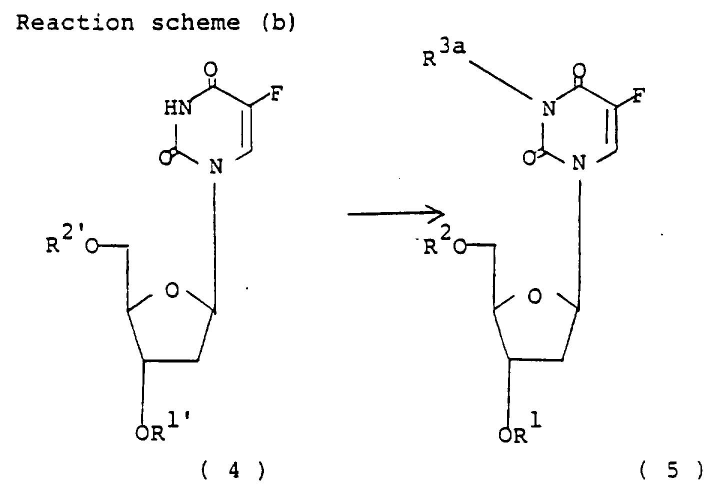

wherein one of R^{1'} and R^{2'} is the same as the group R defined above, the other is a hydrogen atom, acyl or a protective group, R^{3a} is acyl, and R¹ and R are as defined above.

The present reaction, wherein acyl is introduced into the 3-position of the pyrimidine moiety (acylation), can be conducted by a usual process, e.g., the acid halide process. With the acid halide process, an acyl halide (R^{3a}X) is caused to act on the compound (4) in a suitable solvent in the presence of an acid scavenger to give the desired compound (5). Examples of useful acid scavengers are sodium hydrogen carbonate, sodium carbonate, potassium carbonate, pyridine, triethylamine, etc. Examples of useful solvents are benzene, chloroform, methylene chloride, carbon tetrachloride, dioxane, tetrahydrofuran, etc. The acyl halide is used in an amount of at least about one mole, preferably about 1 to about 3 moles, per mole of the compound (4). The reaction temperature is usually -30 to 100°C, preferably room temperature to about 80°C. The reaction takes about 20 minutes to about 20 hours.

When the compound (4) to be reacted has a free hydroxyl group at its 3′- or 5′-position, acylation takes place also at such position simultaneously with acylation at the 3-position. Accordingly, it is desirable to protect the hydroxyl group at the 3′- or 5′-position before acylation and to remove the protective group after the acylation. The reaction for introducing the protective group will be described later. The reaction to remove the protective group can be carried out by the same method as already described for the reaction scheme (a).

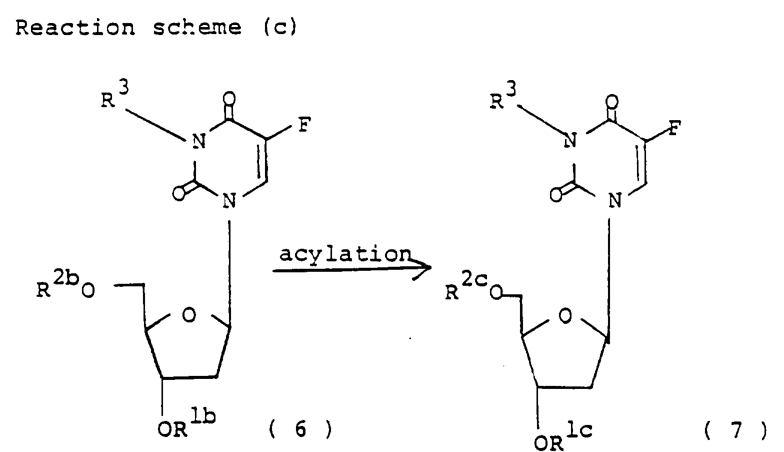

wherein one of R^{1b} and R^{2b} is a hydrogen atom, the other is the same as the group R defined above, one of R^{1c} and R^{2c} is acyl, the other is the same as the group R defined above, and R³ is as defined above.

This reaction acylates the free hydroxyl group at the 3'- or 5'-position of the compound (6) to afford a compound (7). For the acylation reaction, any of usual acylation processes are usable, such as the acid halide process, acid anhydride process, mixed acid anhydride process, N,N-dicyclohexylcarbodiimide process (DCC process), etc., among which the acid anhydride process and acid halide process are advantageous.

The acid anhydride process is conducted by heating the compound (6) with an acid anhydride in a suitable solvent. The acid anhydride to be used is the anhydride of an acid corresponding to the acyl group to be introduced into the 3′- or 5′-position. Examples of such anhydrides are acetic anhydride, propionic anhydride, butyric anhydride, benzoic anhydride, etc. These acid anhydrides are used in an amount of at least one mole, preferably about 1 to about 3 moles, per mole of the compound (6). Examples of useful solvents are various inert solvents including pyridine, hydrocarbon halides such as chloroform and dichloromethane, ethers such as dioxane and THF, aromatic hydrocarbons such as benzene and toluene, dimethylformamide (DMF), dimethyl sulfoxide (DMSO), acetonitrile, etc. The reaction temperature is usually about -30°C to about 100°C, preferably room temperature to about 80°C. The reaction takes about 20 minutes to about 20 hours. The reaction can be carried out advantageously in the presence of a basic compound. Examples of useful basic compounds are organic bases such as pyridine, triethylamine, N,N-dimethylaniline and like tertiary amines, and inorganic basic compounds such as sodium hydrogencarbonate, potassium carbonate and sodium acetate.

The acid halide process is practiced by causing an acyl halide (R^{3a}X) to act on the compound (6) in a suitable solvent in the presence of an acid scavenger, in the same manner as described for the reaction scheme (b).

When the reagent, i.e., the acid halide is used in an amount of at least two moles per mole of the starting compound for the processes of the reaction schemes (b) and (c), the reaction may give an O- and N-acylated compound wherein the 3'- or 5'-position and the 3-position are acylated at the same time, in addition to an O-acylated compound wherein the 3'- or 5'-position is acylated and an N-acylated compound wherein the 3-position is acylated. The O- and N-acylated compound can be easily separated from the O-acylated compound or N-acylated compound.

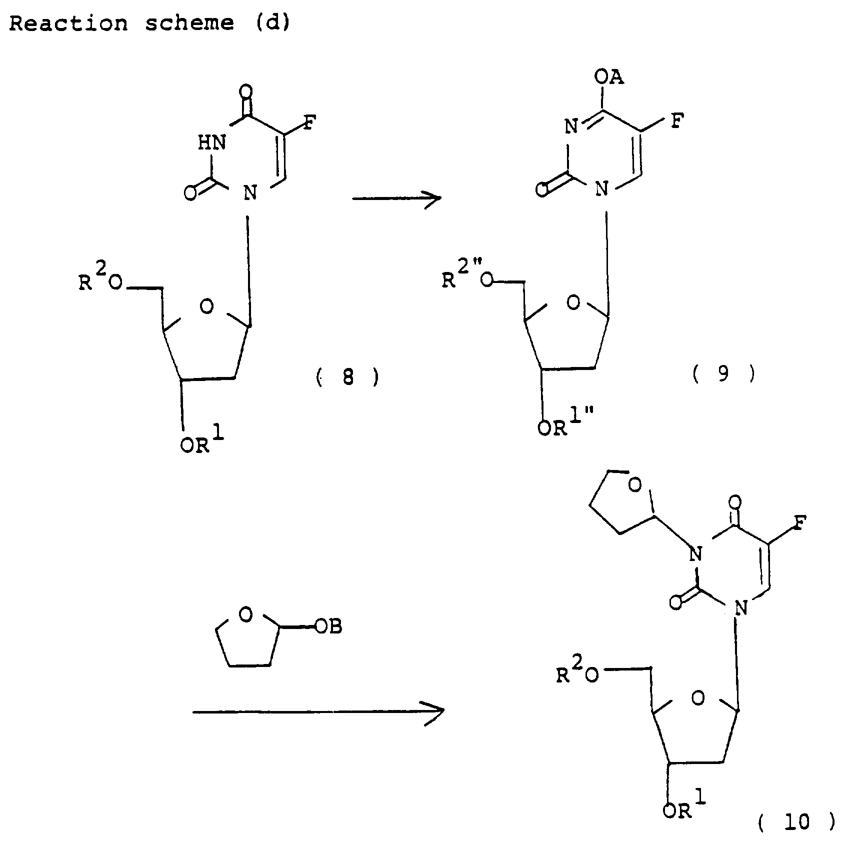

wherein R¹ and R are as defined above, A is tri(lower alkyl)silyl, B is lower alkanoyl such as C₁-C₆ alkanoyl, one of R^{1"} and R^{2"} is the same as the group R defined above and the other is the same as the group A or acyl.

According to the above scheme, bis-N,O-tri(lower alkyl)silylacetamide is reacted with a compound (8) (trialkylsilylation) to obtain a compound (9), which is then reacted with 2-lower alkanoyloxytetrahydrofuran (tetrahydrofuranylation) to give a compound (10).

The trialkylsilylation reaction is conducted in a suitable inert solvent at about 0 to about 100°C, preferably at room temperature to 50°C for 30 minutes to 6 hours. Examples of suitable solvents are ethers such as dioxane and THF, aromatic hydrocarbons such as benzene and toluene, DMF, DMSO, acetonitrile, etc. The bis-N,O-tri(lower alkyl)silylacetamide is used in an amount of at least one equivalent, preferably 1 to 2 equivalents, per functional group to be reacted therewith.

The subsequent tetrahydrofuranylation reaction is carried out in a solvent as exemplified above at about 0 to about 100°C, preferably at room temperature to 50°C for 30 minutes to 6 hours. 2-Lower alkanoyloxytetrahydrofuran is used in an amount of at least one mole, preferably 1 to 2 moles, per mole of the compound (9). This reaction proceeds advantageously when the reaction system contains a Lewis acid, such as stannic chloride (SnCl₄), aluminum chloride or zinc chloride, usually in an amount of at least 0.1 mole based on the compound (9). When the compound (9) used for the tetrahydrofuranylation reaction contains tri(lower alkyl)silyl as R^{1˝} or R^{2˝}, the product is subsequently subjected to a reaction to remove this group, whereby the desired compound (10) is obtained. This reaction is carried out in the same manner as the reaction already stated for the reaction scheme (a) for the removal of the protective group.

In this way, the compound of the present invention is obtained. The compound is isolated and purified easily by a usual method, such as reprecipitation, recrystallization, silica gel chromatography, ion exchange chromatography, gel chromatography, affinity chromatography or the like.

The starting materials to be used for the processes of the reaction schemes (a) to (d) can be prepared, for example, by the processes represented by the following reaction schemes (e) to (g).

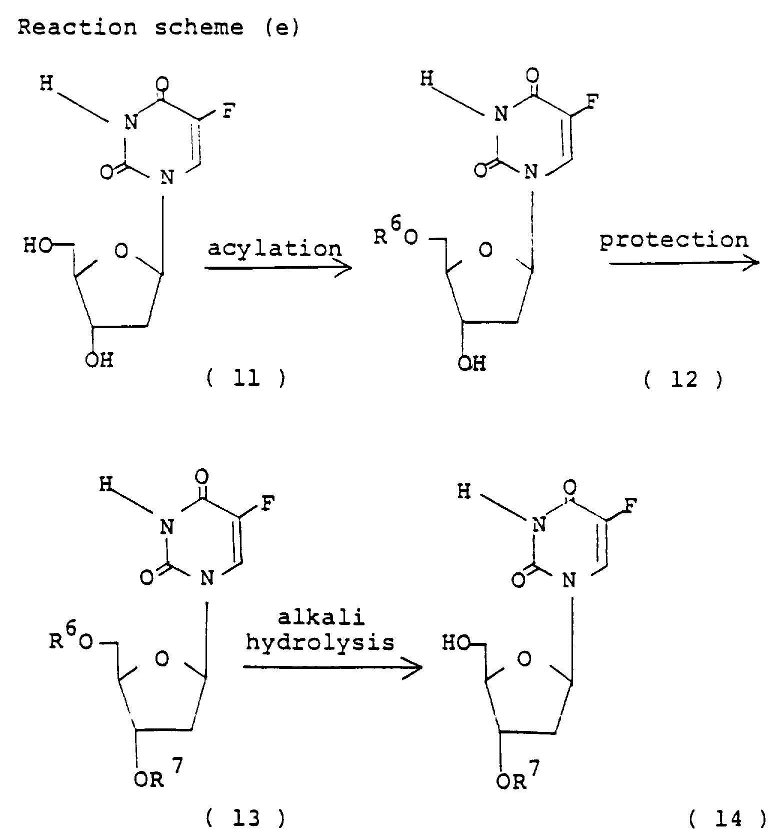

wherein R⁶ is acyl, and R⁷ is a protective group.

The acylation of the compound (11) is conducted in the same manner as the acylation of the compound (6) shown in the reaction scheme (c). More preferably, the acylation is conducted by reacting the ccmpound (11) with an acid anhydride, corresponding to the acyl to be introduced into the 5′-position, in an amount of about 1 to about 1.5 moles per mole of the compound (11) at a temperature of about -30°C to about 80°C for about 1 to about 6 hours in the same inert solvent as used for the acid anhydride process shown by the scheme (c).

The above reaction gives as the main product a compound (12) wherein the 5′-position is acylated and also as a secondary product a compound wherein the 3′-position is acylated.

The compound (12) resulting from the reaction is then subjected to a reaction to protect the hydroxyl group at the 3′-position. By this reaction, the protective group mentioned with respect to the reaction scheme (a) is introduced into the 3′-position of the compound (12). Useful reagents for introducing the protective group are triaryl-substituted methyl halides for giving a protective group represented by the formula (A), unsaturated cyclic ethers represented by the formula 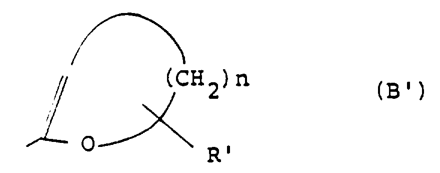 wherein R′ and n are the same as those in the formula (B) for giving a protective group represented by the formula (B), lower alkoxymethyl halides and tri(lower alkyl)silyl halides.

The protective group-introducing reaction wherein such a halide is used is conducted in the same manner as the hydrogen halide removing reaction shown in the reaction scheme (a). However, it is desirable that the reagent is used in an amount of 1 to 2 moles, preferably 1 to 1.5 moles, per mole of the compound (12), and that the reaction temperature is -30°C to 80°C.

The protective group-introducing reaction wherein an unsaturated cyclic ether of the formula (B′) is used is conducted in the presence of an acid catalyst in an aprotic inert solvent such as THF, dioxane or acetonitrile. Examples of useful acid catalysts are hydrohalogenic acids such as hydrogen bromide and hydrogen chloride, and Lewis acids such as aluminum chloride, boron fluoride and zinc chloride. The reaction is conducted using 1 to 1.5 moles of the reagent per mole of the compound (12) at -30°C to 60°C for about 2 to about 5 hours.

The reaction to remove the acyl from the 5′-position of the resulting product (13) is conducted under the conditions of alkali hydrolysis, i.e. under the same conditions as the hydrolysis reaction for the reaction scheme (a) wherein a basic compound is used.

### Reaction scheme (f)

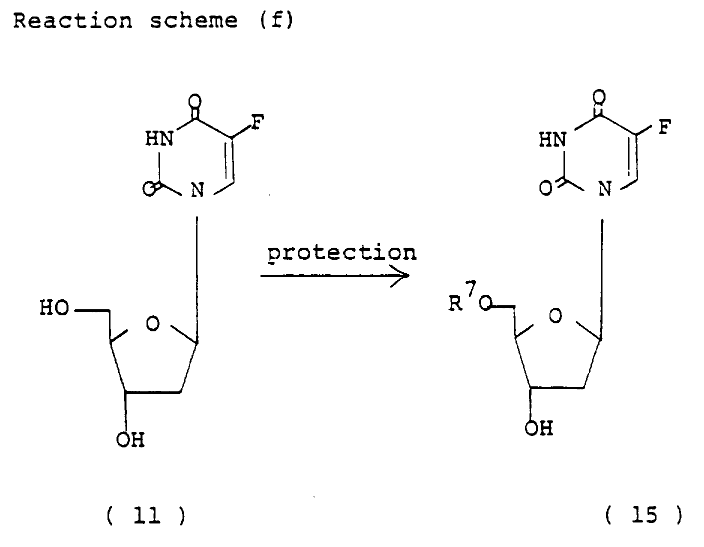

wherein R⁷ is as defined above.

This reaction introduces a protective group directly into the compound (11), giving a compound (15) having the protective group at the 5'-position. The reaction is conducted under the same conditions as in the reaction scheme (e).

The processes of the schemes (e) and (f) afford starting compounds having an acyl group or protective group at the 3'- or 5'-position.

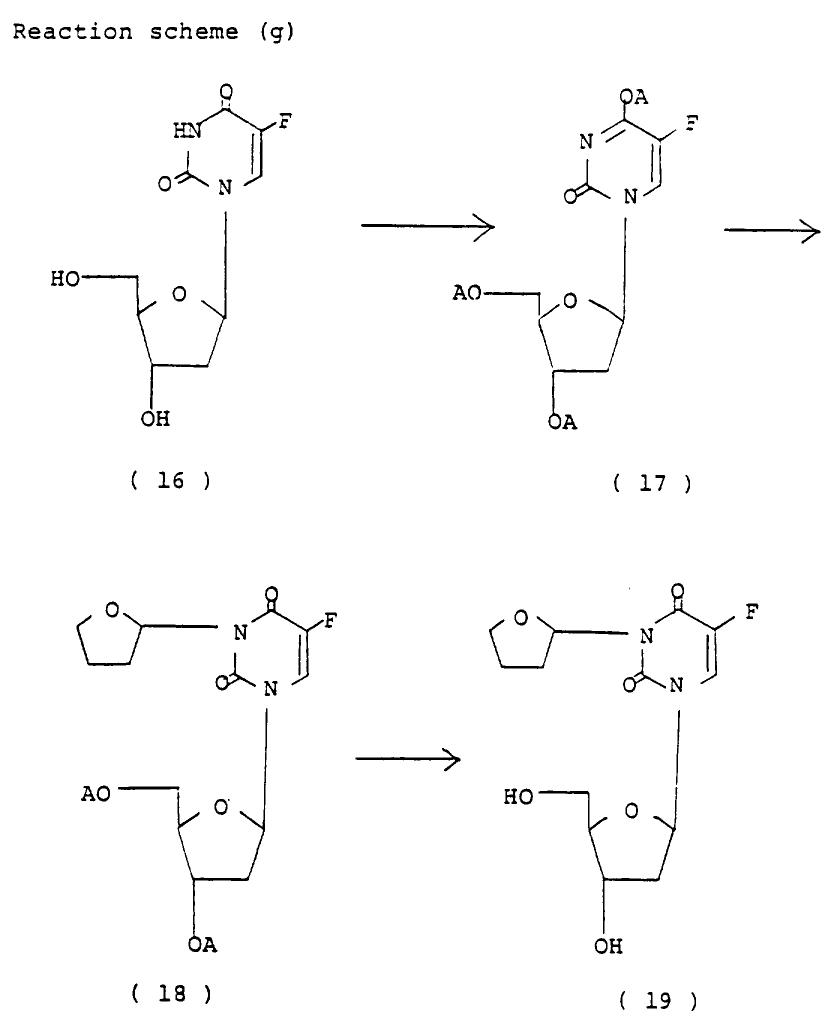

wherein A is as defined above.

The trialkylsilylation of the compound (16) is conducted in the same manner as that of the compound (8) shown in the reaction scheme (d) except that at least 3 moles of bis-O,N-tri(lower alkyl)silylacetamide is used per mole of the compound (16).

The tetrahydrofuranylation of the 3-position of the resulting compound (17) and the subsequent reaction for removing the lower alkylsilyl group from the 3′- and 5′-positions of the compound (18) are conducted in the same manner as the corresponding reactions shown in the reaction scheme (d). Consequently a compound (19) is obtained which has tetrahydrofuranyl at the 3-position. When subjected to the reaction of the scheme (e) or (f), the compound (19) provides a starting compound having tetrahydrofuranyl at the 3-position and further having an acyl grcup or protective group at the 3′- or 5′-position.

The starting compounds obtained by the processes of the reaction schemes are usable directly as such or after isolation from the reaction system by a usual method.

When the acyl of the formula (Y) is to be introduced by the acylation reaction described, the acyl halide material can be prepared by the process of the following reaction scheme (h).

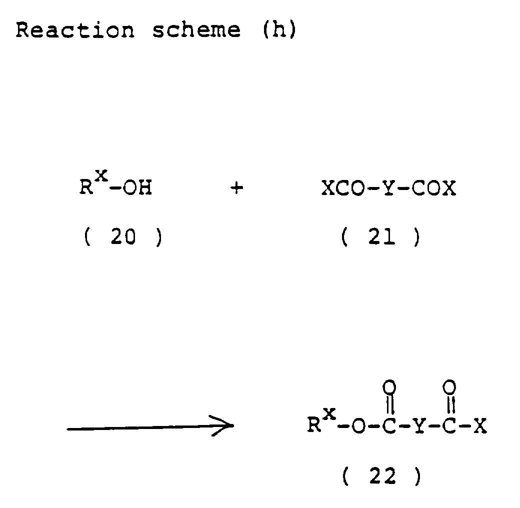

wherein R^{x}, Y and X are as defined above.

The above reaction can be conducted in the same manner as the acid halide process described for the reaction scheme (c).

The compounds of the present invention can be prepared also by the processes of the following reaction schemes (i) to (o).

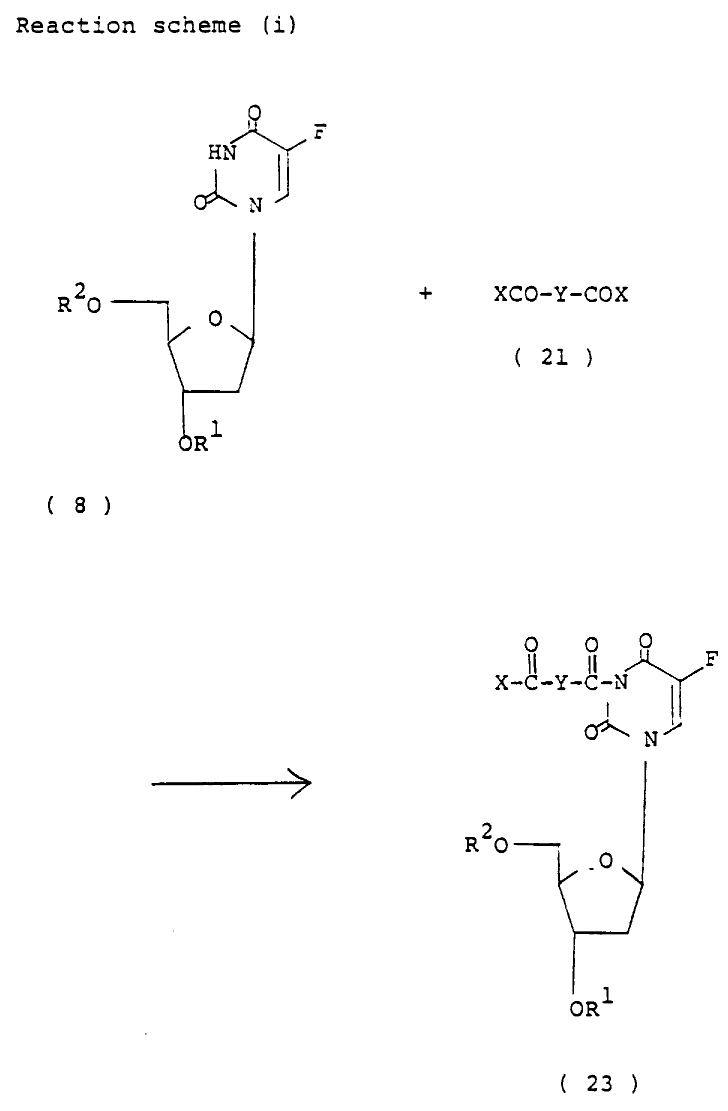

wherein R¹, R, X and Y are as defined above.

The above reaction can be conducted in the same manner as the acid halide process described for the reaction scheme (b).

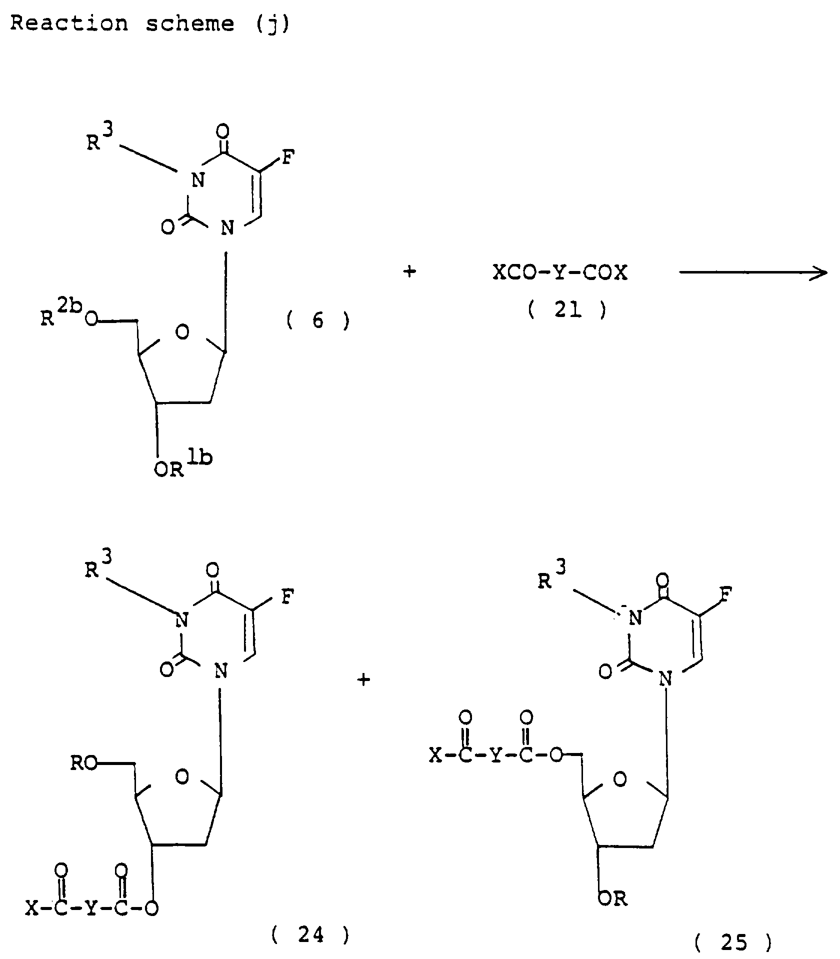

wherein R, R^{1b}, R^{2b}, R³, X and Y are as defined above.

The above reaction can be conducted in the same manner as is the case with the scheme (i).

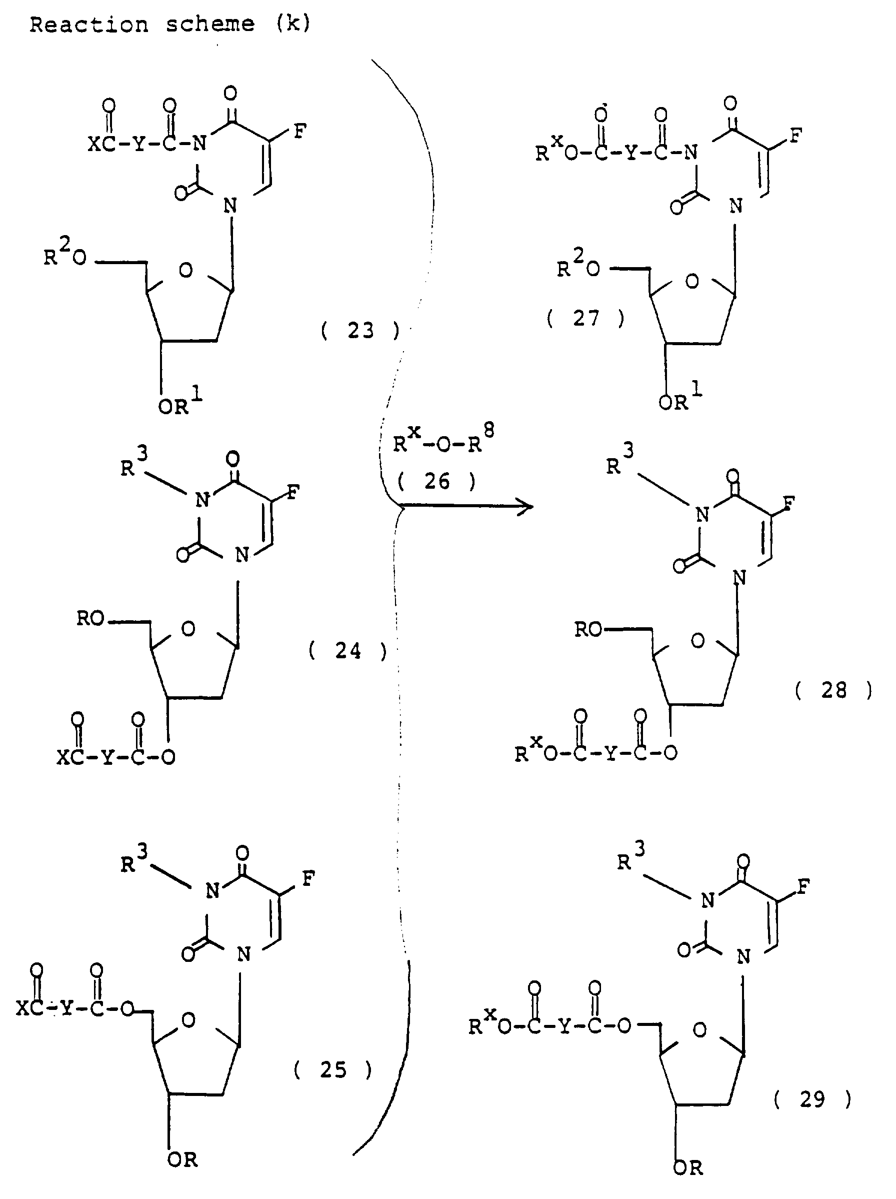

wherein R¹, R, R³, X, Y, R and R^{x} are as defined above, and R⁸ is a hydrogen atom or tri(lower alkyl)silyl group.

According to the above scheme, one of the acid halides (23), (24) and (25) obtained by the processes of the schemes (i) and (j) is reacted with a compound (26) for acylation. When the compound (26) used is one wherein R⁸ is hydrogen, the reaction can be carried out in the same manner as in the scheme (c).

Further when the compound (26) used is one wherein R⁸ is tri(lower alkyl)silyl group, the reaction is conducted using a solvent, which will not adversely affect the reaction, in a dry state, preferably in an anhydrous state. Examples of preferred solvents are halogenated hydrocarbons such as dichloromethane and chloroform, aromatic hydrocarbons such as benzene and toluene, and aprotic solvents such as dioxane, acetonitrile, acetone and tetrahydrofuran. Usually, at least about one mole, preferably 1 to 3 moles, of the compound (26) is used per mole of the acid halide (23), (24) or (25). The reaction is conducted at about 0 to about 100°C, preferably room temperature to about 80°C, for 1 to 6 hours, preferably about 2 hours. When R⁸ is hydrogen, it is desirable to use a base, such as triethylamine, trimethylamine, dimethylaniline or pyridine, for the reaction. Further if R⁸ is tri(lower alkyl)silyl, it is desirable to use a Lewis acid such as stannic chloride, zinc chloride or aluminum chloride.

The compounds (26) to be used for the present reaction are generally those known but include some novel compounds. Such compounds can be easily prepared by the following process. The compound substituted with 2-tetrahydrofuranyl at the 1-position of the pyridine ring can be prepared from a corresponding known pyridine derivative having a hydrogen atom at the 1-position, by adding hexamethyldisilazane to the derivative, refluxing the mixture for about 2 to about 20 hours, thereafter removing the excess of the silazane from the reaction mixture, dissolving the residue in halogenated hydrocarbon such as dichloromethane and chloroform or in an aprotic solvent such as dioxane and acetonitrile, adding to the solution 2-acetoxytetrahydrofuran in an amount of at least about one mole to 2 moles per mole of the pyridine derivative and a Lewis acid such as stannic chloride, zinc chloride or aluminum chloride, and reacting the mixture at about 0 to about 100°C, preferably around room temperature for about 1 to about 10 hours. When 2-acetoxytetrahydrofuran used for the reaction is replaced by an aroyl halide, lower alkanoyl halide, aroyloxycarbonyl halide, lower alkoxycarbonyl halide, 2-halogenophthalide allyl halide, phenyl-lower alkoxy-lower alkyl halide or lower alkoxy-lower alkyl halide, the compound obtained has the substituent of aroyloxy group, lower alkanoyloxy group, aroyloxycarbonyloxy group or lower alkoxycarbonyloxy group at the 2 and/or 4 position(s) of the pyridine ring, or of phthalidyl group, allyl group, phenyl-lower alkoxy-lower alkyl group or lower alkoxy-lower alkyl group at the 1-position of the pyridine ring. Further the compound substituted with N-(lower alkoxycarbonyl lower alkyl)aminocarbonyl at the 1-position of the pyridine ring can be prepared from a corresponding pyridine derivative having a hydrogen atom at the 1-position, by adding to the derivative with at least about one mole of a lower alkoxycarbonyl-lower alkyl-isocyanate per mole of the derivative in a solvent such as dioxane, dichloromethane, chloroform, acetonitrile, acetone or pyridine, and subjecting the mixture to reaction at about 0 to about 100°C for about 10 minutes to about 1 hour, preferably about 30 minutes.

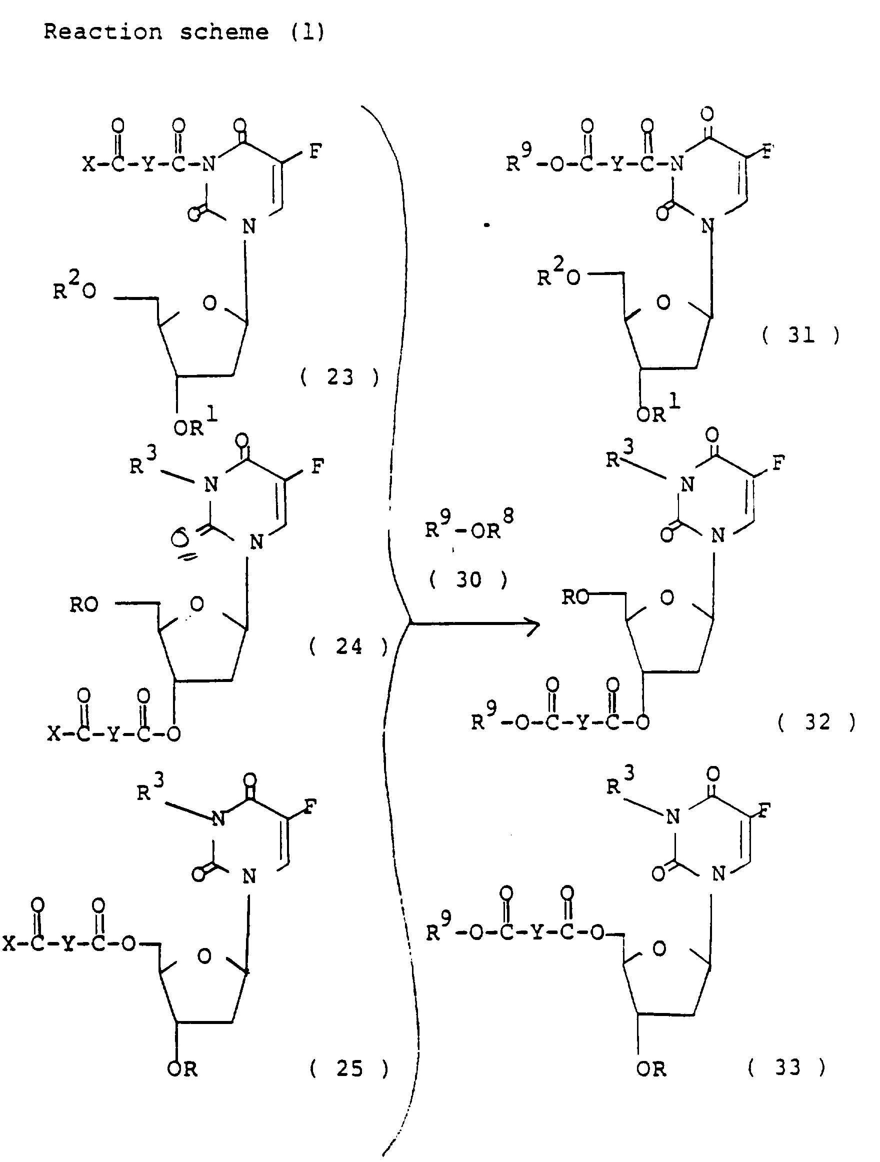

wherein R¹, R, R³, R⁸, X, Y and R are as defined above, and R⁹ is lower alkyl.

According to the above scheme, a compound (30) is reacted with one of the acid halides (23), (24) and (25) obtained from the processes of the schemes (i) and (j). The reaction is conducted under the same conditions as in the reaction scheme (k).

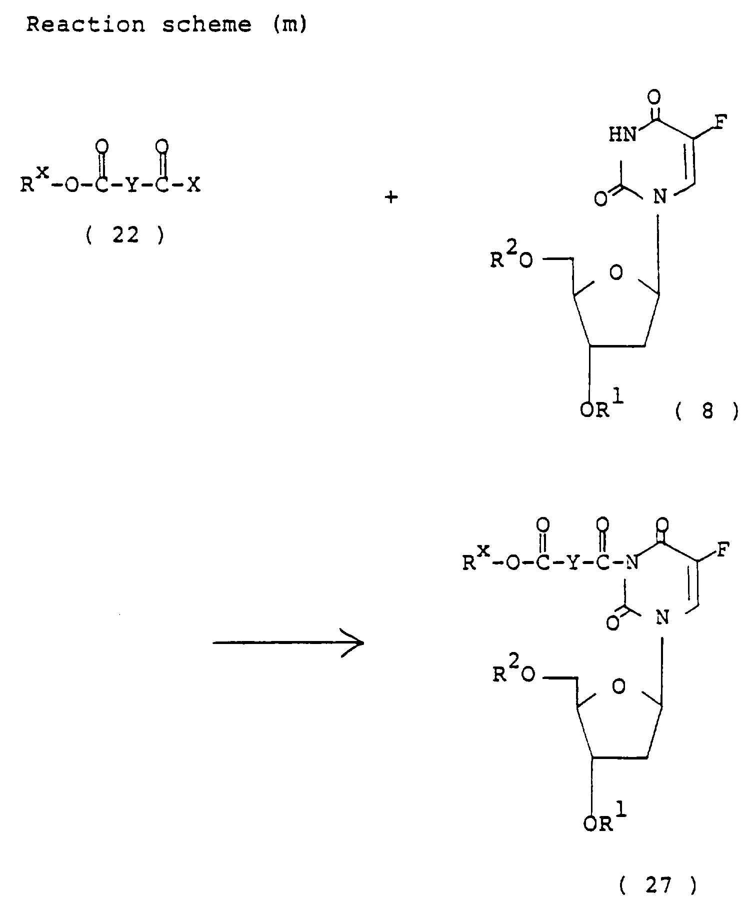

wherein R¹, R, R^{x}, X and Y are as defined above.

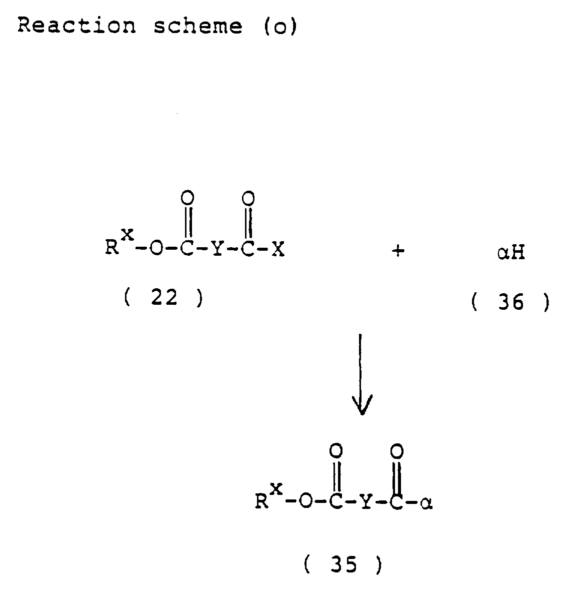

wherein R^{x}, α, X and Y are as defined above.

This reaction can be carried out in the same manner as the reaction of the scheme (i).

The compounds (1a) and (1b) of the present invention have an outstanding anticancer effect and are low in toxicity. For example, they are reduced in side effects such as loss of body weight. The present compounds are therefore very useful as antitumor agents for curing cancers in man and animals.

Our research has revealed that the anticancer effect of the present compounds (1) can be further enhanced when they are used in combination with a pyridine derivative represented by the formula (X) below. 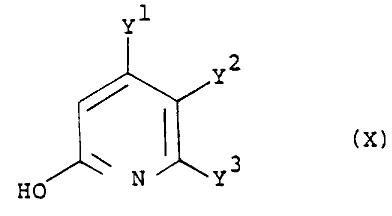 wherein one of Y¹ and Y³ is a hydrogen atom, the other is hydroxyl, Y is a hydrogen atom, halogen atom, lower alkyl, cyano or nitro, and the hydroxyl group(s) at the 2-, 4- and/or 6-position(s) may be an acylated one.

Some of the compounds of the formula (X) are known. The others can be easily prepared by a known process (Triv. Chem. Rec. 73, 704 (1954)). The acylation of the hydroxyl group(s) at the 2-, 4- and/or 6-position(s) can be conducted in the same manner as in reaction scheme (b) or (c).

The desired products of the present invention are used in the form of generally acceptable pharmaceutical compositions which are prepared by using diluents and excipients such as fillers, bulking agents, binders, wetting agents, disintegrating agents, surfactants, lubricants and the like. Administration unit forms of these pharmaceutical compositions of the present invention can be varied and selected so as to meet various therapeutical purposes. Typical forms of the pharmaceutical compositions can be exemplified such as tablets, pills, powders, liquids, suspensions, emulsions, granules, capsules, suppositories, injections (liquids, suspensions and others), ointments and the like.

In shaping into the form of tablets, those known as the carriers in this field can widely be applied for example, excipients such as lactose, purified sugar, sodium chloride, glucose, urea, starch, calcium carbonate, kaolin, crystalline cellulose, silicic acid and others; binders such as water, ethanol, propanol, simple syrup, a glucose solution, starch solution, gelatin solution, carboxymethyl cellulose, shellac, methyl cellulose, potassium phosphate, polyvinylpyrrolidone and others; disintegrating agents such as dried starch, sodium alginate, agar-agar powder, laminaran powder, sodium hydrogen carbonate, calcium carbonate, a fatty acid ester of polyoxyethylene sorbitan, sodium laurylsulfate, monoglyceride of stearic acid, starch, lactose and others; disintegration inhibitors such as purified sugar, stearin cacao butter, hydrogenated oils and others; absorption accelerators such as quaternary ammonium base, sodium laurylsulfate and others; wetting agents such as glycerin, starch and others; adsorption accelerators such as starch, lactose, kaolin, bentonite, colloidal silicic acid and others; and lubricants such as purified talc powder, stearic acid salts, boric acid powder, polyethylene glycol and others can be exemplified. If necessary, the tablets can further be coated with usual coating film to make them into coated tablets, for example sugar-coated tablets, gelatin film-coated tablets, enteric film-coated tablets, film-coated tablets, or double-layered tablets, multiple-layered tablets and others. In shaping into the form of pills, those known as the carriers in this field can widely be applied for example, excipients such as glucose, lactose, starch, cacao butter, hydrogenated vegetable oils, kaolin, talc and others; binders such as powdered gum arabi, powdered tragacanth gum, gelatin, ethanol and others; disintegrating agents such as laminaran, agar-agar powder and others. In shaping into the form of suppositories, those known in this field can widely be applied for example, polyethylene glycol, cacao butter, a higher alcohol, an ester of a higher alcohol, gelatin, semi-synthesized glyceride and others. Capsules are prepared in a conventional manner by admixing the compound of the invention with the foregoing various carriers and encapsulating the mixture into hard-gelatin capsules, soft-gelatin capsules, etc. In case of preparing injections, solutions, emulsions and suspensions being prepared are sterilized, and they are preferably isotonic to the blood. In preparing into the form of solutions, emulsions and suspensions, those known as the diluents in this field can widely be applied, for example water, ethanol, propylene glycol, ethoxylated isostearyl alcohol, polyoxylated isostearyl alcohol, a polyoxyethylene sorbitan fatty acid ester and others. In case of preparing isotonic solutions, sufficient amount of sodium chloride, glucose or glycerin may be added to make the solution to be isotonic to the blood. The pharmaceutical compositions for injection preparation may further be contain usual dissolving agents, buffer solutions, analgesic agents or the like if necessary. The pharmaceutical composition of the present invention may also contain coloring agents, preservatives, perfumes, seasoning agents, sweetening agents and others, as well as contain other medicines, if necessary. In shaping into the form of pastes, creams and gels, diluents such as white vaseline, paraffins, glycerine, cellulose derivatives, polyethylene glycols, silicones, bentonite and the like can be used.

The amount of the desired product according to the present invention to be contained as the active ingredient in the pharmaceutical composition is not specifically restricted and can be selected from a wide range, generally 1 to 70 % by weight may be used.

Administration method of the above-mentioned pharmaceutical composition is not specifically restricted and can be administered through a suitable method for the respective types of administration forms, depending upon age of the patient, distinction of the sex and other conditions, conditions of the patient and others. For example, tablets, pills, liquids, suspensions, emulsions, granules and capsules are administered orally; injections are administered intraveneously singly or as a mixture with usual injectable transfusions such as a glucose solution, amino acids solutions, and others; and if necessary the injections are administered singly intramuscularly, intracutaneously, subcutaneously or intraperitoneally; and the suppositories are administered into rectum.

The dosage of the desired products of the present invention may suitably be selected depending upon the method for administration, age of the patient, distinction of sex and other conditions, and conditions of the symptoms, and generally the pharmaceutical compositions of the invention can be administered in an amount of about 0.5 to about 20 mg/kg of the body weight/day, calculated as the compound of the invention (active ingredient), in 1 to 4 divided doses.

The present invention will be described in greater detail with reference to the following reference examples, examples, pharmaceutical tests and preparation examples.

In connection with the NMR data in the reference examples and examples, the numerals used as a subscript at the right of the symbol "C", "H" or "N" are used to refer to the position in the compound. Thus the term "C₆-H", for example, refers to the hydrogen bonded to the carbon atom at the 6-position. Similarly the term "C_{3'·4'·5'}-H", for example, denotes the hydrogens bonded to the carbon atoms at the 3′-, 4′- and 5′-positions. Also the term "H₁", for example, refers to the hydrogen bonded to the carbon atom at the 1-position. The term "N₃-H", for example, refers to the hydrogen bonded to the nitrogen atom at the 3-position of the 5-fluorouracil ring.

### Reference Example 1

### Preparation of 2′-deoxy-5′-O-trityl-5-fluorouridine.

A 9.00 g quantity of sodium chloride was added to a solution of 5.00 g of 2′-deoxy-5-fluorouridine in pyridine at room temperature with stirring, and the solution was stirred for 15 hours. The solvent was distilled off and the residue was dissolved in ethyl acetate. The solution was washed three times with a saturated aqueous solution of sodium chloride. The ethyl acetate layer was dried on sodium sulfate and concentrated. The residue was applied to a silica gel column to conduct gradient elution using chloroform and methanol(up to 2 %)-chloroform mixtures, giving 6.80 g of 2′-deoxy-5′-O-trityl-5-fluorouridine in a yield of 69 %.

### Reference Example 2

### Preparation of 2′-deoxy-5-fluoro-3-(2-tetrahydrofuranyl) uridine

A 16.5 g quantity of N,O-bis(trimethylsilyl) acetamide was added to a suspension of 5.00 g of 2′-deoxy-5-fluorouridine in 50 ml of dry dichloromethane at room temperature with stirring, and the mixture was stirred for four hours. Then a solution of 4.00 g of 2-acetoxytetrahydrofuran and 1.17 ml of stannic chloride in 10 ml of dry dichloromethane was added and the mixture was stirred for 1.5 hours. Then 8.0 ml of triethylamine was added to neutralize the mixture and the mixture was washed with water. The dichloromethane layer was concentrated and the residue was dissolved in 100 ml of methanol. A 3 ml quantity of acetic acid was added thereto and the mixture was left to stand at 40°C for 3 hours. The solvent was distilled off and the residue was applied to a silica gel column to conduct gradient elution using chloroform and methanol (up to 4 %)-chloroform mixtures, giving 5.25 g of 2′-deoxy-5-fluoro-3-(2-tetrahydrofuranyl)uridine as an oil in a yield of 81.7 %.
¹H-NMR(CDCl₃)δ:
8.06 (1H, d, J = 6Hz, C₆-H)
6.53 (1H, bt, J = 6Hz, 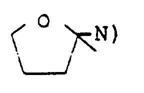 6.23 (1H, bt, J = 6Hz, C_{1′}-H)
4.45-3.75 (8H, m, C_{3'}·_{4'}·_{5'}-H, C_{3'}·_{5'}-OH, 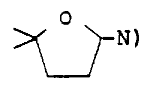 2.29-2.16 (6H, m, C_{2'}-H,

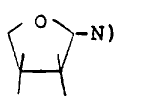

### Reference Example 3

### Preparation of 5-chloro-4-hydroxy-1-(2-tetrahydrofuranyl)-2(1H)-pyridone

A 10 ml quantity of hexamethyldisilazane was added to 1.00 g of 5-chloro-4-hydroxy-2(1H)-pyridone, and the mixture was refluxed for 6 hours. Then excess hexamethyldisilazane was distilled off and the oily residue was dissolved in 50 ml of dichloromethane. To the solution was added 1.00 g of 2-acetoxytetrahydrofuran and 0.1 ml of stannic chloride and the mixture was stirred overnight at room temperature. The reaction mixture was neutralized with triethylamine, and the solvent was distilled off. Methanol was added to the residue and the mixture was stirred at room temperature for 2 hours. The solvent was distilled off and the residue was placed on a silica gel column and eluted with 2 % methanol-chloroform, giving 1.07 g of the title compound in a yield of 73.5 %.
M.p. 170-173°C
¹H-NMR(DMSO-d₆)δ:
11.6 (1H, bs, OH)
7.59 (1H, s, C₆-H)
6.09-5.99 (1H, q, 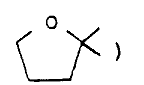 5.76 (1H, s, C₃-H)
4.39-3.73 (2H, m, 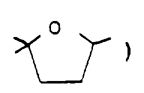 2.42-1.82 (4H, m,

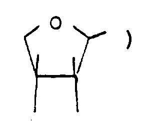

### Reference Example 4

### Preparation of 5-chloro-1-ethoxymethyl-4-hydroxy-2(1H)-pyridone

Following the general procedure of Reference Example 3 and using chloromethyl ethyl ether in place of the 2-acetoxytetrahydrofuran, 5-chloro-1-ethoxymethyl-4-hydroxy-2(1H)-pyridone was prepared in a yield of 39 %.
M.p. 217-219°C
¹H-NMR (DMSO-d₆)δ:
11.63 (1H, bs, OH)
7.87 (1H, s, C₆-H)
5.75 (1H, s, C₃-H)
5.16 (2H, s, N-CH₂-O-)
3.49 (2H, q, J = 7Hz, -OCH₂ CH₃)
1.09 (3H, t, J = 7Hz, -OCH₂ CH₃)

### Reference Example 5

### Preparation of 2-benzoyloxy-5-chloro-4-hydroxypyridine

The title compound was prepared in a yield of 51 % by following the general procedure of Reference Example 3 and using benzoyl chloride in place of the 2-acetoxytetrahydrofuran. The title compound softens at 184°C.
¹H-NMR (DMSO-d₆)δ:
8.27 (1H, s, C₆-H)
8.16-8.07 (2H, m, 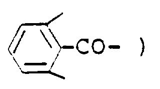 7.78-7.51 (3H, m, 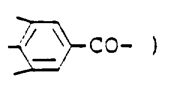 6.91 (1H, s, C₃-H)

### Reference Example 6

### Preparation of 4-hydroxy-1-(3-phthalidyl)-2(1H)-pyridone

A 10 ml quantity of hexamethyldisilazane was added to 1.00 g of 4-hydroxy-2(1H)-pyridone and the mixture was refluxed for 6 hours. Then the reaction mixture was concentrated under reduced pressure and the concentrate was dissolved in 20 ml of acetonitrile. To this solution was added 2.29 g of 3-bromophthalide and the mixture was stirred overnight at room temperature. Methanol was added to the reaction mixture and the resulting mixture was stirred at room temperature for 15 minutes. Then the reaction mixture was concentrated under reduced pressure, and the concentrate was applied to a silica gel column and eluted with 1 % methanol-chloroform, giving 0.62 g of the title compound in a yield of 30 %.
M.p. 239-241°C
¹H-NMR(DMSO-d₆)δ:
11.05 (1H, bs, OH)
8.29-7.52 (5H, m, 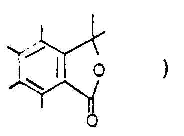 6.99 (1H, d, J = 8Hz, C₆-H)
5.88 (1H, dd, J₃·₅ = 2Hz, J₅·₆ = 8Hz, C₅-H)
5.65 (1H, d, J = 2Hz, C₃-H)

### Reference Example 7

### Preparation of 1-carbomethoxymethylcarbamoyl-4-hydroxy-2(1H)-pyridone

A 2.49 g quantity of carbomethoxymethylisocyanate was added to a suspension of 2.00 g of 4-hydroxy-2(1H)-pyridone in 60 ml of dioxane, and the mixture was stirred at 90°C for 30 minutes. The solvent was distilled off, and ether was added to the residue. The precipitate thus formed was filtered, giving 2.20 g of the title compound in a yield of 54 %.
M.p. 124 - 126°C ¹H-NMR (DMSO-d₆) δ:
11.52 (1H, bs, OH)
10.82 (1H, t, J = 6Hz, -CONHCH₂-)
8.20 (1H, d, J = 8Hz, C₆-H)
6.17 (1H, dd, J₅·₆ = 8Hz, J₃·₅ = 2Hz, C₅-H)
5.74 (1H, d, J = 2Hz, C₃-H)
4.15 (2H, d, J = 6Hz, -CONHCH₂-)
3.68 (3H, s, -COOCH₃)

### Reference Example 8

### Preparation of 1-benzyloxymethyl-5-chloro-4-hydroxy-2(1H)-pyridone

Following the general procedure of Reference Example 3 and using benzyloxymethyl chloride in place of 2-acetoxytetrahydrofuran and also using acetonitrile as the solvent in place of dichloromethane, the title compound was prepared in a yield of 57 %.
M.p. 165-167°C
¹H-NMR(DMSO-d₆)δ:
11.65 (1H, bs, OH)
7.92 (1H, s, C₆-H)
7.31 (5H, s, phenyl-H)
5.77 (1H, s, C₃-H)
5.27 (2H, s, -NCH₂O-)
4.55 (2H, s,

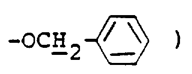

### Reference Example 9

### Preparation of 2,4-bis(trimethylsilyloxy)-5-chloropyridine

A 50 ml quantity of hexamethyldisilazane was added to 9.6 g of 5-chloro-4-hydroxy-2(1H)-pyridone, and the mixture was stirred overnight on an oil bath at 140°C. The insolubles were removed by filtration, and the filtrate was distilled under reduced pressure, giving 14.4 g of the title compound boiling at 120°C/7 mmHg.
Yield 75 %.

### Reference Example 10

### Preparation of 2-acetoxy-5-chloro-4-hydroxy-pyridine

A 2.09 ml quantity of acetyl bromide and 0.10 ml of stannic chloride were added to a solution of 5.00 g of 2,4-bis(trimethylsilyloxy)-5-chloropyridine in 250 ml of dry dichloromethane, and the mixture was stirred at room temperature for 3.5 hours. The reaction mixture was neutralized with triethylamine, and the solvent was distilled off. The residue was applied to a silica gel column and eluted with 40 % ethyl acetate-benzene as an eluent, giving 2.07 g of the title compound in a yield of 64 %.
M.p. 270-272°C
¹H-NMR(DMSO-d₆)δ:
11.90 (1H, bs, OH)
8.20 (1H, s, C₆-H)
6.69 (1H, s, C₃-H)
2.27 (3H, s, COCH₃)

### Example 1 (for comparison)

### Preparation of 5'-O-benzyl-2'-deoxy-5-fluorouridine (R¹ = R³ = H, R = C₆H₅CH₂)

To a solution of 1.00 g of 5'-O-acetyl-2'-deoxy-5-fluoro-3'-O-trityluridine in 30 ml of methanol was added 1N aqueous solution of sodium hydroxide, and the mixture was stirred at room temperature for 4 hours. The reaction mixture was neutralized with acetic acid and concentrated. The concentrate was washed with petroleum ether, dried, and dissolved in a mixture of 20 ml of benzene and 20 ml of dioxane. To the solution were added 0.27 ml of benzyl bromide and 0.15 g of potassium hydroxide powder, and the mixture was refluxed overnight. The solvent was distilled off and the residue was dissolved in 50 ml of 80 % acetic acid, and the solution was left to stand at 80°C for 4 hours. The solvent was distilled off, and the residue was placed on a silica gel column to conduct a gradient elution using chloroform and mixtures of methanol (up to 2 %) and chloroform. The fractions corresponding to 5'-O-benzyl-2'-deoxy-5-fluorouridine were collected and concentrated. The concentrate was recrystallized from ethanol, giving 0.33 g of the title compound in a yield of 52 %.
M.p. 129-130°C
Elementary Analysis: for C₁₆H₁₇FN₂O₅
Calcd. (%) : C 57.14; H 5.09; N 8.33
Found (%) : C 57.14; H 5.35; N 8.34
¹H-NMR (DMSO-d₆) δ:
11.76 (1H, bs, -NH-, disappeared by addition of D₂O)
7.95 (1H, d, J = 7Hz, C₆-H)
7.34 (5H, s, phenyl-H)
6.15 (1H, t, J = 7Hz, C₁,-H)
5.33 (1H, bs, 3'-OH, disappeared by addition of D₂O)
4.55 (2H, s, 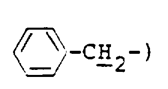 4.34-4.16 (1H, m, C₃,-H)
4.00-3.89 (1H, m, C₄,-H)
3.69-3.63 (2H, m, C₅,-H)
2.14 (2H, t, J = 6Hz, C₂,-H).

### Example 2 (for comparison)

### Preparation of 3'-O-benzyl-2'-deoxy-5-fluorouridine (R = R³ = H, R¹ = C₆H₅CH₂)

To a mixture of 50 ml of benzene and 50 ml of dioxane were added 1.00 g of 2'-deoxy-5-fluoro-5'-O-trityluridine, 0.30 ml of benzyl bromide and 0.23 g of potassium hydroxide powder, and the mixture was refluxed for 25 hours. The insolubles were removed by filtration and the filtrate was concentrated and the concentrate was dissolved in 5 ml of 80 % acetic acid. The solution was left to stand at 80°C for 2 hours. The solvent was distilled off and the residue was placed on a silica gel column to conduct a gradient elution using chloroform and mixtures of methanol (up to 2 %) and chloroform. The fractions corresponding to 3'-O-benzyl-2'-deoxy-5-fluorouridine were collected and concentrated. The concentrate was recrystallized from ethanol, giving 0.14 g of the title compound in a yield of 20 %.
M.p. 138-139°C
Elementary Analysis: for C₁₆H₁₇FN₂O₅
Calcd. (%): C 57.14; H 5.09; N 8.33
Found (%) : C 57.16; H 5.30; N 8.13
¹H-NMR (DMSO-d₆) δ:
11.82 (1H, bs, -NH-, disappeared by addition of D₂O)
8.21 (1H, d, J = 7Hz, C₆-H)
7.35 (5H, s, phenyl-H)
6.16 (1H, t, J = 6Hz, C₁,-H)
5.22 (1H, bs, 5'-OH, disappeared by addition of D₂O)
4.54 (2H, s, 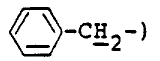 4.24-4.19 (1H, m, C₃,-H)
4.09-4.06 (1H, m, C₄,-H)
3.65-3.53 (2H, m, C₅,-H)
2.51-2.16 (2H, m, C₂,-H)

### Example 3 (for comparison)

### Preparation of 2'-deoxy-5-fluoro-3'-O-(3,4-methylenedioxybenzyl)uridine (R = R³ = H, R¹ = 3,4-methylenedioxybenzyl)

The title compound was prepared in a yield of 23 % in the same manner as in Example 2 except that acetonitrile was used as the reaction solvent in place of the dioxane.
M.p. 186-188°C
Elementary Analysis: for C₁₇H₁₇FN₂O₇·0.5 H₂O
Calcd. (%): C 52.44; H 4.66; N 7.19
Found (%) : C 52.60; H 4.62; N 7.03
¹H-NMR (CDCl₃) δ:
11.80 (1H, d, J = 5Hz, -NH-, disappeared by addition of D₂O)
8.18 (1H, d, J = 7Hz, C₆-H)
6.90-6.85 (3H, m, phenyl-H)
6.11 (1H, t, J = 6Hz, C₁,-H)
5.99 (2H, s, -O-CH₂-O-)
5.17 (1H, t, J = 5Hz, 5'-OH, disappeared by addition of D₂O)
4.42 (2H, s, 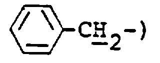 4.18-4.00 (2H, m, C_{3'}·C_{4'}-H)
3.64-3.61 (2H, m, C_{5'}-H)
2.30-2.20 (2H, m, C_{2'}-H)

### Examples 4 and 5 (for comparison)

### Preparation of 3'-O-benzyl-2'-deoxy-5-fluorouridine (R = R³ = H, R¹ = C₆H₅CH₂, Example 4) and 5'-O-benzyl-2'-deoxy-5-fluorouridine (R¹ = R³ = H, R = C₆H₅CH₂, Example 5)

A 11.4 g quantity of potassium hydroxide was dissolved in a mixture of 350 ml of water and 100 ml of dioxane. To the mixture were added 10.0 g of 2'-deoxy-5-fluorouridine and 3.0 ml of benzyl bromide at room temperature with stirring. Then, every 24 hours, 100 ml of 5 % aqueous solution of potassium hydroxide and 3.0 ml of benzyl bromide were added three times to the mixture. Then, the resulting mixture was stirred overnight. The reaction mixture was washed twice with 200 ml of ether, and the aqueous layer was neutralized with 6N-HCl and concentrated to about 200 ml. The concentrate was adjusted to a pH of about 3 to 4 with 6N-HCl and extracted twice with 100 ml of ethyl acetate. The ethyl acetate layer was separated, dried on anhydrous sodium sulfate and concentrated. The oily residue was placed on a silica gel column to conduct a gradient elution using chloroform and mixtures of methanol (up to 2 %) and chloroform. The fractions corresponding to 3'-O-benzyl-2'-deoxy-5-fluorouridine were collected and concentrated. The concentrate was recrystallized from ethanol, giving 3.57 g of the desired compound in a yield of 26.1 %.
M.p. 138-139°C
Elementary Analysis: for C₁₆H₁₇FN₂O₅
Calcd. (%): C 57.14; H 5.09; N 8.33
Found (%) : C 57.12; H 5.28; N 8.24

Then the fractions corresponding to 5'-O-benzyl-2'-deoxy-5-fluorouridine were collected and concentrated. The concentrate was recrystallized from ethanol, giving 0.40 g of the desired compound in a yield of 2.9 %.
M.p. 129-130°C
Elementary Analysis: for C₁₆H₁₇FN₂O₅
Calcd. (%): C 57.14; H 5.09; N 8.33
Found (%) : C 57.29; H 5.30; N 8.26

The general procedures of Examples 4 and 5 were followed, thereby giving the following compounds.

### Example 6 (for comparison)

### 3'-O-(2-fluorobenzyl)-2'-deoxy-5-fluorouridine (R = R³ = H, R¹ = 2-fluorobenzyl)

Yield 34 %
M.p. 121-123°C
¹H-NMR (DMSO-d₆) δ:
11.82 (1H, bs, -NH-, disappeared by addition of D₂O)
8.21 (1H, d, J = 7Hz, C₆-H)
7.67-7.19 (4H, m, phenyl-H)
6.15 (1H, t, J = 6Hz, C_{1'}-H)
5.26 (1H, bs, 5'-OH, disappeared by addition of D₂O)
4.60 (2H, s, 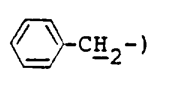 4.28-4.05 (2H, m, C_{3'}·C_{4'}-H)
3.77-3.67 (2H, m, C_{5'}-H)
2.41-2.18 (2H, m, C_{2'}-H)

### Example 7 (for comparison)

### 5'-O-(2-fluorobenzyl)-2'-deoxy-5-fluorouridine (R¹ = R³ = H, R = 2-fluorobenzyl)

Yield 5.2 %
M.p.-(oily)
¹H-NMR (DMSO-d₆) δ:
11.7 (1H, bs, -NH-, disappeared by addition of D₂O)
7.92 (1H, d, J = 7Hz, C₆-H)
7.52-7.07 (4H, m, phenyl-H)
6.20 (1H, t, J = 6Hz, C_{1'}-H)
4.62 (2H, s, 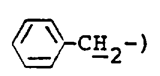 4.44-3.97 (2H, m, C_{3'}·C_{4'}-H)
3.84-3.57 (2H, m, C_{5'}-H)
2.21-2.08 (2H, m, C_{2'}-H)

### Example 8 (for comparison)

### 3'-O-(3-fluorobenzyl)-2'-deoxy-5-fluorouridine (R = R³ = H, R¹ = 3-fluorobenzyl)

Yield 27%
M.p. 113-115°C
¹H-NMR(DMSO-d₆) δ:
11.81 (1H, bs, -NH-, disappeared by addition of D₂O)
8.21 (1H, d, J = 7Hz, C₆-H)
7.46-7.01 (4H, m, phenyl-H)
6.17 (1H, t, J = 6Hz, C_{1'}-H)
5.22 (1H, bt, J = 5Hz, 5'-OH, disappeared by addition of D₂O)
4.58 (2H, s, 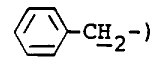 4.31-4.06 (2H, m, C_{3'}·_{4'}-H)
3.74-3.59 (2H, m, C_{5'}-H)
2.38-2.03 (2H, m, C_{2'}-H)

### Example 9 (for comparison)

### 5'-O-(3-fluorobenzyl)-2'-deoxy-5-fluorouridine (R¹ = R³ = H, R = 3-fluorobenzyl)

Yield 5.9%
M.p.-(oily)
¹H-NMR(CDCl₃) δ:
10.4 (1H, bs, -NH-, disappeared by addition of D₂O)
7.92 (1H, d, J = 7Hz, C₆-H)
7.41-6.77 (4H, m, phenyl-H)
6.28 (1H, bs, C_{1'}-H)
4.63-3.51 (6H, m, C_{3'}·_{4'}·_{5'}-H, 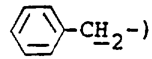 2.49-1.93 (2H, m, C_{2'}-H)

### Example 10 (for comparison)

### 3'-O-(2-bromobenzyl)-2'-deoxy-5-fluorouridine (R = R³ = H, R¹ = 2-bromobenzyl)

Yield 33 %
M.p. 122-124°C
¹H-NMR(DMSO-d₆) δ:
11.81 (1H, bs, -NH-, disappeared by addition of D₂O)
8.20 (1H, d, J = 7Hz, C₆-H)
7.67-7.32 (4H, m, phenyl-H)
6.15 (1H, t, J = 6Hz, C_{1'}-H)
5.21 (1H, t, J = 5Hz, 5'-OH, disappeared by addition of D₂O)
4.57 (2H, s, 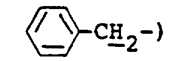 4.25-4.05 (2H, m, C_{3'}·_{4'}-H)
3.70-3.52 (2H, m, C_{5'}-H)
2.40-1.94 (2H, m, C_{2'}-H)

### Example 11 (for comparison)

### 5'-O-(2-bromobenzyl)-2'-deoxy-5-fluorouridine (R¹ = R³ = H, R = 2-bromobenzyl)

Yield 5%
M.p.-(oily)
¹H-NMR(DMSO-d₆) δ:
11.78 (1H, bs, -NH-, disappeared by addition of D₂O)
7.91 (1H, d, J = 7Hz, C₆-H)
7.69-7.32 (4H, m, phenyl-H)
6.15 (1H, t, J = 6Hz, C_{1'}-H)
5.35 (1H, t, J = 7Hz, 5'-OH, disappeared by addition of D₂O)
4.60 (2H, s, 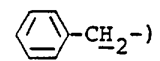 4.31-3.42 (4H, m, C_{3'}·_{4'}·_{5'}-H)
2.20-2.09 (2H, m, C_{2'}-H)

### Example 12 (for comparison)

### 3'-O-(3-bromobenzyl)-2'-deoxy-5-fluorouridine (R = R³ = H, R¹ = 3-bromobenzyl)

Yield 19 %
M.p. 166-168°C
¹H-NMR(DMSO-d₆) δ:
11.80 (1H, bs, -NH-, disappeared by addition of D₂O)
8.18 (1H, d, J = 7Hz, C₆-H)
7.69-7.31 (4H, m, phenyl-H)
6.15 (1H, t, J = 6Hz, C_{1'}-H)
5.19 (1H, t, J = 5Hz, 5'-OH, disappeared by addition of D₂O)
4.54 (2H, s, 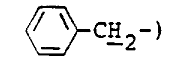 4.23-4.03 (2H, m, C_{3'}·_{4'}-H)
3.71-3.57 (2H, m, C_{5'}-H)
2.34-2.21 (2H, m, C_{2'}-H)

### Example 13 (for comparison)

### 5'-O-(3-bromobenzyl)-2'-deoxy-5-fluorouridine (R¹ = R³ = H, R = 3-bromobenzyl)

Yield 3 %
M.p.-(oily)
¹H-NMR(DMSO-d₆) δ:
11.90 (1H, bs, -NH-, disappeared by addition of D₂O)
8.00 (1H, d, J = 7Hz, C₆-H)
7.67-7.34 (4H, m, phenyl-H)
6.12 (1H, t, J = 6Hz, C_{1'}-H)
5.46 (1H, bs, 3'-OH, disappeared by addition of D₂O)
4.54 (2H, s, 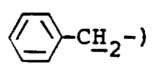 4.30-3.90 (2H, m, C_{3'}·_{4'}-H)
3.74-3.68 (2H, m, C_{5'}-H)
2.13 (2H, t, J = 6Hz, C_{2'}-H)

### Example 14

### 3'-O-(4-bromobenzyl)-2'-deoxy-5-fluorouridine (R = R³ = H, R¹ = 4-bromobenzyl)

Yield 12 %
M.p. 214-217°C
¹H-NMR(DMSO-d₆) δ:
11.80 (1H, bs, -NH-, disappeared by addition of D₂O)
8.18 (1H, d, J = 7Hz, C₆-H)
7.55 and 7.30 (each 2H, d, J = 8Hz, phenyl-H)
6.11 (1H, t, J = 6Hz, C_{1'}-H)
5.19 (1H, t, J = 5Hz, 5'-OH, disappeared by addition of D₂O)
4.51 (2H, s, 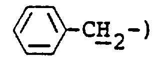 4.23-4.02 (2H, m, C_{3'}·_{4'}-H)
3.73-3.60 (2H, m, C_{5'}-H)
2.36-2.07 (2H, m, C_{2'}-H)

### Example 15 (for comparison)

### 3'-O-(4-t-butylbenzyl)-2'-deoxy-5-fluorouridine (R = R³ = H, R¹ = 4-t-butylbenzyl)

Yield 17%
M.p.-(powder)
¹H-NMR(DMSO-d₆) δ:
11.80 (1H, bs, -NH-, disappeared by addition of D₂O)
8.18 (1H, d, J = 7Hz, C₆-H)
7.48 and 7.30 (each 2H, d, J = 8Hz, phenyl-H)
6.12 (1H, t, J = 6Hz, C_{1'}-H)
5.18 (1H, t, J = 5Hz, 5'-OH, disappeared by addition of D₂O)
4.48 (2H, s, 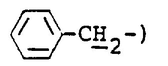 4.30-4.04 (2H, m, C_{3'}·_{4'}-H)
3.76-3.60 (2H, m, C_{5'}-H)
2.24-2.08 (2H, m, C_{2'}-H)
1.27 (9H, s, CH₃ x 3)

### Example 16 (for comparison)

### 5'-O-(4-t-butylbenzyl)-2'-deoxy-5-fluorouridine (R¹ = R³ = H, R = 4-t-butylbenzyl)

Yield 2%
M.p.-(oily)
¹H-NMR(DMSO-d₆) δ:
11.80 (1H, bs, -NH-, disappeared by addition of D₂O)
7.94 (1H, d, J = 7Hz, C₆-H)
7.34 and 7.16 (each 2H, d, J = 8Hz, phenyl-H)
6.14 (1H, t, J = 6Hz, C_{1'}-H)
5.31 (1H, bs, 3'-OH, disappeared by addition of D₂O)
4.51 (2H, s, 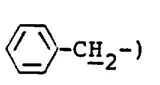 4.28-3.94 (2H, m, C_{3'}·_{4'}-H)
3.74-3.64 (2H, m, C_{5'}-H)
2.13 (2H, t, J = 6Hz, C_{2'}-H)
1.27 (9H, s, CH₃ x 3)

### Example 17 (for comparison)

### 3'-O-(4-phenylbenzyl)-2'-deoxy-5-fluorouridine (R = R³ = H, R¹ = 4-phenylbenzyl)

Yield 12 %
M.p. 207-209°C
¹H-NMR (DMSO-d₆) δ:
11.90 (1H, bs, -NH-, disappeared by addition of D₂O)
8.19 (1H, d, J = 7Hz, C₆-H)
7.69-7.39 (9H, m, phenyl-H)
6.15 (1H, t, J = 6Hz, C_{1'}-H)
5.25 (1H, t, J = 5Hz, 5'-OH, disappeared by addition of D₂O)
4.58 (2H, s, 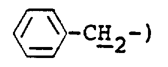 4.29-4.07 (2H, m, C_{3'}·_{4'}-H)
3.83-3.63 (2H, m, C_{5'}-H)
2.26-2.06 (2H, m, C_{2'}-H)

### Examples 18 and 19

### Preparation of 3'-O-(4-chlorobenzyl)-2'-deoxy-5-fluorouridine (R = R³ = H, R¹ = 4-chlorobenzyl, Example 18) and 5'-O-(4-chlorobenzyl)-2'-deoxy-5-fluorouridine (R¹ = R³ = H, R = 4-chlorobenzyl, Example 19 (for comparison))

A 4.0 g quantity of potassium hydxoride was dissolved in a mixture of 150 ml of water and 40 ml of dioxane. To the mixture were added 2.00 g of 2'-deoxy-5-fluorouridine and 5.5 g of 4-chlorobenzyl chloride at room temperature with stirring. Two days later, the same subsequent procedures as in Examples 4 and 5 were conducted and the resulting residue was placed on a silica gel column to conduct a gradient elution using chloroform and mixtures of methanol (up to 2 %) and chloroform. The fractions corresponding to 3'-O-(4-chlorobenzyl)-2'-deoxy-5-fluorcuridine were collected and concentrated, giving 0.50 g of the desired compound in a yield of 17 %.
M.p. 196-198°C
¹H-NMR(DMSO-d₆) δ:
11.81 (1H, bs, -NH-, disappeared by addition of D₂O)
8.20 (1H, d, J = 7Hz, C₆-H)
7.38 (4H, s, phenyl-H)
6.14 (1H, t, J = 7Hz, C_{1'}-H)
5.21 (1H, bt, J = 5Hz, 5'-OH, disappeared by addition of D₂O)
4.53 (2H, s, 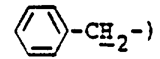 4.23-4.14 (1H, m, C_{3'}-H)
4.10-4.03 (1H, m, C_{4'}-H)
3.71-3.58 (2H, m, C_{5'}-H)
2.41-2.02 (2H, m, C_{2'}-H)
Elementary Analysis: for C₁₆H₁₆ClFN₂O₅
Calcd. (%): C 51.83; H 4.35; N 7.56
Found (%) : C 51.82; H 4.60; N 7.41

Then the fractions corresponding to 5'-O-(4-chlorobenzyl)-2'-deoxy-5-fluorouridine were collected and concentrated, giving 0.12 g of the desired compound in a yield of 4.0 % as a powder.
¹H-NMR(DMSO-d₆) δ:
11.79 (1H, bs, -NH-, disappeared by addition of D₂O)
7.91 (1H, d, J = 7Hz, C₆-H)
7.38 (4H, s, phenyl-H)
6.13 (1H, t, J = 6Hz, C_{1'}-H)
5.33 (1H, bs, 3'-OH, disappeared by addition of D₂O)
4.53 (2H, s, 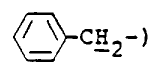 4.38-4.21 (1H, m, C_{3'}-H)
4.04-3.82 (1H, m, C_{4'}-H)
3.78-3.74 (2H, m, C_{5'}-H)
2.25-1.98 (2H, m, C_{2'}-H)
Elementary Analysis: for C₁₆R₁₆ClFN₂O₅
Calcd. (%): C 51.83; H 4.35; N 7.56
Found (%) : C 51.73; H 4.80; N 7.97

### Examples 20-22 (for comparison)

The general procedures of Examples 18 and 19 were followed, thereby giving the following compounds.

### Example 20 (for comparison)

### 3'-O-(2,4-dichlorobenzyl)-2'-deoxy-5-fluorouridine (R = R³ = H, R¹ = 2,4-dichlorobenzyl)

Yield 14 %
M.p. 88-90°C
¹H-NMR(DMSO-d₆) δ:
11.82 (1H, bs, -NH-, disappeared by addition of D₂O)
8.20 (1H, d, J = 7Hz, C₆-H)
7.60-7.37 (3H, m, phenyl-H)
6.14 (1H, t, J = 6Hz, C_{1'}-H)
5.21 (1H, t, J = 5Hz, 5'-OH, disappeared by addition of D₂O)
4.59 (2H, s, 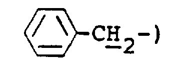 4.28-4.03 (2H, m, C_{3'}·_{4'}-H)
3.69-3.60 (2H, m, C_{5'}-H)
2.37-2.19 (2H, m, C_{2'}-H)

### Example 21 (for comparison)

### 5'-O-(2,4-dichlorobenzyl)-2'-deoxy-5-fluorouridine (R¹ = R³ = H, R = 2,4-dichlorobenzyl)

Yield 3.3 %
M.p. 109-111°C
¹H-NMR(DMSO-d₆) δ:
11.77 (1H, bs, -NH-, disappeared by addition of D₂O)
7.89 (1H, d, J = 7Hz, C₆-H)
7.60-7.36 (3H, m, phenyl-H)
6.14 (1H, t, J = 6Hz, C_{1'}-H)
5.33 (1H, bs, 3'-OH, disappeared by addition of D₂O)
4.61 (2H, s, 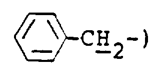 4.36-3.83 (2H, m, C_{3'}·_{4'}-H)
3.74-3.60 (2H, m, C_{5'}-H)
2.14 (2H, t, J = 6Hz, C_{2'}-H)

### Example 22 (for comparison)

### 3'-O-(4-methoxybenzyl)-2'-deoxy-5-fluorouridine (R = R³ = H, R¹ = 4-methoxybenzyl)

Yield 8.1 %
M.p. 164-166°C
¹H-NMR(DMSO-d₆) δ:
11.81 (1H, bs, -NH-, disappeared by addition of D₂O)
8.19 (1H, d, J = 7Hz, C₆-H)
7.27 and 6.91 (each 2H, d, J = 8Hz, phenyl-H)
6.12 (1H, t, J = 6Hz, C_{1'}-H)
5.19 (1H, bt, J = 5Hz, 5'-OH, disappeared by addition of D₂O)
4.45 (2H, s, 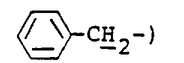 4.19-4.02 (2H, m, C_{3'}·_{4'}-H)
3.70-3.50 (2H, m, C_{5'}-H)
2.31-2.13 (2H, m, C_{2'}-H)

### Example 23 (for comparison)

### Preparation of 2'-deoxy-5-fluoro-3'-(2-methylbenzyl) uridine (R = R³ = H, R¹ = 2-methylbenzyl)

A 1.14 g quantity of potassium hydroxide was dissolved in a mixture of 33 ml of water and 16 ml of acetonitrile. To the solution were added 1.00 g of 2'-deoxy-5-fluorouridine and 1.50 g of 2-methylbenzyl bromide at room temperature with stirring. Then the same subsequent procedures as in Examples 4 and 5 were conducted, giving 0.29 g of the title compound in a yield of 20 %.
M.p. 114-116°C
¹H-NMR(DMSO-d₆) δ:
11.79 (1H, bs, -NH-, disappeared by addition of D₂O)
8.19 (1H, d, J = 7Hz, C₆-H)
7.30-7.17 (4H, m, phenyl-H)
6.11 (1H, t, J = 6Hz, C_{1'}-H)
5.19 (1H, t, J = 5Hz, 5'-OH, disappeared by addition of D₂O)
4.45 (2H, s, 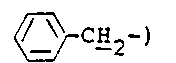 4.22-4.02 (2H, m, C_{3'}·C_{4'}-H)
3.66-3.62 (2H, m, C_{5'}-H)
2.29-2.21 (5H, m, C_{2'}-H and CH₃)
Elementary Analysis: for C₁₇H₁₉FN₂O₅
Calcd. (%): C 58.28; H 5.46; N 7.99 Found (%) : C 58.12; H 5.64; N 8.01

### Examples 24 and 25 (for comparison)

The general procedure of Example 23 was followed, thereby giving the following compounds.

### Example 24 (for comparison)

### 3'-O-(3-methylbenzyl)-2'-deoxy-5-fluorouridine (R = R³ = H, R¹ = 3-methylbenzyl)

Yield 23 %
M.p. 129-131°C
¹H-NMR(DMSO-d₆) δ:
11.80 (1H, bs, -NH-, disappeared by addition of D₂O)
8.19 (1H, d, J = 7Hz, C₆-H)
7.15 (4H, s, phenyl-H)
6.12 (1H, t, J = 6Hz, C_{1'}-H)
5.18 (1H, t, J = 5Hz, 5'-OH, disappeared by addition of D₂O)
4.49 (2H, s, 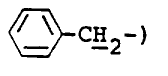 4.21-4.02 (2H, m, C_{3'}·_{4'}-H)
3.66-3.61 (2H, m, C_{5'}-H)
2.31-2.22 (5H, m, C_{2'}-H and CH₃)

### Example 25 (for comparison)

### 3'-O-(4-methylbenzyl)-2'-deoxy-5-fluorouridine (R = R³ = H, R¹ = 4-methylbenzyl)

Yield 21 %
M.p. 178-180°C
¹H-NMR(DMSO-d₆) δ:
11.81 (1H, bs, -NH-, disappeared by addition of D₂O)
8.18 (1H, d, J = 7Hz, C₆-H)
7.30-7.13 (4H, m, phenyl-H)
6.12 (1H, t, J = 6Hz, C_{1'}-H)
5.17 (1H, t, J = 5Hz, 5'-OH, disappeared by addition of D₂O)
4.20-4.01 (2H, m, C_{3'}·C_{4'}-H)
3.65-3.60 (2H, m, C_{5'}-H)
2.29-2.12 (5H, m, C_{2'}-H and CH₃)

### Example 26 (for comparison)

### Preparation of 3'-O-benzyl-2'-deoxy-5-fluoro-5'-O-nicotinoyluridine (R¹ = C₆H₅CR₂, R³ = H, R = 3-pyridinecarbonyl)

A 0.21 g quantity of nicotinoyl chloride (hydrochloride) was added to a solution of 0.20 g of 3'-O-benzyl-2'-deoxy-5-fluorouridine in 10 ml of pyridine, and the mixture was left to stand at 80°C for 3 hours. The solvent was distilled off and the residue was dissolved in 30 ml of ethyl acetate. The solution was washed twice with 20 ml of water. The ethyl acetate layer was dried on anhydrous sodium sulfate and concentrated. The concentrate was placed on a silica gel column and eluted with chloroform, giving 0.18 g of the title compound in a yield of 69 %.
M.p. 130-132°C
¹H-NMR(CDCl₃) δ:
11.14 (1H, bs, -NH-, disappeared by addition of D₂O)
9.22 (1H, s, 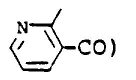 8.81 (1H, d, J = 4Hz, 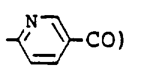 8.24 (1H, d, J = 8Hz, 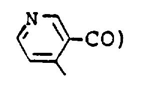 7.53-7.28 (7H, m, 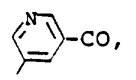 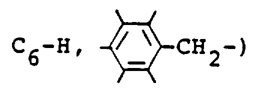 6.22 (1H, t, J = 6Hz, C_{1'}-R)
4.71-4.21 (6H, m, 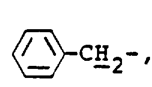 C_{3'}·_{4'}·_{5'}-H)
2.75-2.04 (2H, m, C₂,-H)
Elementary Analysis: for C₂₂H₂₀FN₃O₆
Calcd. (%): C 59.86; H 4.57; N 9.52
Found (%) : C 60.01; H 4.56; N 9.58

### Examples 27-38 (for comparison)

The general procedure of Example 26 was followed, thereby giving the following compounds.

### Example 27 (for comparison)

### 3'-O-benzyl-5'-O-benzoyl-2'-deoxy-5-fluorouridine (R¹ = C₆H₅CH₂, R = C₆H₅CO, R³ = H)

Yield 75 %
M.p. 125-127°C
¹H-NMR(DMSO-d₆) δ:
11.94 (1H, bs, -NH-, disappeared by addition of D₂O)
8.00-7.41 (6H, m, 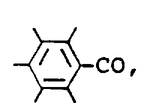 C₆-H)
7.33 (5H, s, 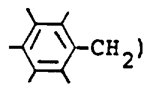 6.17 (1H, t, J = 6Hz, C_{1'}-H)
4.59 (6H, m, 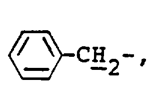 C_{3'}·_{4'}·_{5'}-H)
2.40-2.27 (2H, m, C_{2'}-H)

### Example 28 (for comparison)

### 3'-O-benzyl-5'-O-phenoxycarbonyl-2'-deoxy-5-fluorouridine (R¹ = C₆H₅CH₂, R = C₆H₅OCO-, R³ = H)

Yield 38%
M.p.-(oily)
¹H-NMR(DMSO-d₆) δ:
12.00 (1H, bs, -NH-, disappeared by addition of D₂O)
7.95 (1H, d, J = 7Hz, C₆-H)
7.55-7.16 (10H, m, phenyl-H)
6.17 (1H, t, J = 6Hz, C_{1'}-H)
4.56-4.21 (6H, m, 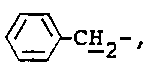 C_{3'}·_{4'}·_{5'}-H)
2.39-2.28 (2H, m, C_{2'}-H)

### Example 29 (for comparison)

### 3'-O-benzyl-5'-O-n-hexanoyl-2'-deoxy-5-fluorouridine (R¹ = C₆H₅CH₂, R = CH₃(CH₂)₄CO, R³ = H)

Yield 51%
M.p.-(oily)
¹H-NMR(DMSO-d₆) δ:
11.94 (1H, bs, -NH-, disappeared by addition of D₂O)
7.90 (1H, d, J = 7Hz, C₆-H)
7.33 (5H, s, phenyl-H)
6.19 (1H, t, J = 6Hz, C_{1'}-H)
4.54 (2H, s, 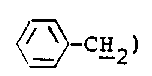 4.29-4.12 (4H, m, C_{3'}·_{4'}·₅,-H)
2.38-2.23 (4H, m, C_{2'}-H, -CH₂CO-)
1.61-1,15 (6H, m, CR₃(CH₂)₃CH₂CO-)
0.84 (3H, t, J = 6Hz, CH₃CH₂-)

### Example 30 (for comparison)

### 3'-O-benzyl-5'-O-benzyl-carbonyl-2'-deoxy-5-fluorouridine (R¹ = C₆H₅CH₂, R = C₆H₅CH₂CO, R³ = H)

Yield 42 %
M.p.-(oily)
¹H-NMR(CDCl₃) δ:
10.06 (1H, bs, -NH-, disappeared by addition of D₂O)
7.42-7.23 (11H, m, phenyl-H, C₆-H)
6.15 (1H, t, J = 6Hz, C_{1'}-H)
4.43 (2H, s, 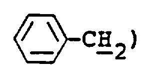 4.33-4.11 (3H, m, C_{3'}·_{5'}-H)
3.96-3.83 (1H, m, C_{4'}-H)
3.64 (2H, s, 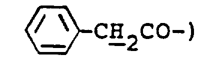 2.47-2.27, 1.65-1.49 (2H, m, C_{2'}-H)

### Example 31 (for comparison)

### 3'-O-benzyl-5'-O-ethoxycarbonyl-2'-deoxy-5-fluorouridine (R¹ = C₆H₅CH₂, R = CH₃CH₂OCO, R³ = H)

Yield 37 %
M.p.-(oily)
¹H-NMR(CDCl₃) δ:
10.18 (1H, bs, -NH-, disappeared by addition of D₂O)
7.68 (1H, d, J = 6Hz, C₆-H)
7.32 (5H, s, phenyl-H)
6.31 (1H, t, J = 6Hz, C_{1'}-H)
4.40 (2H, s, 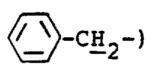 4.37-4,10 (6H, m, C_{3'}·_{4'}·_{5'}-H, OCH₂CH₃)
2.67-2.42, 2.23-1.92 (2H, m, C_{2'}-H)
1.30 (3H, t, J = 7Hz, -OCR₂CH₃)

### Example 32 (for comparison)

### 3'-O-benzyl-5'-O-(3-methylbenzoyl) -2'-deoxy-5-fluorouridine (R¹ = C₆H₅CH₂, R = 3-methylbenzoyl, R³ = H)

Yield 67 %
M.p. 131-133°C
¹H-NMR(CDCl₃) δ:
9.61 (1H, bs, -NH-, disappeared by addition of D₂O)
7.78-7.73 (2H, m, 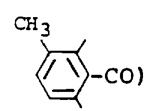 7.60 (1H, d, J = 6Hz, C₆-H)
7.38-7.22 (7H, m, 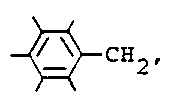 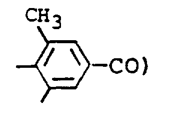 6.25 (1H, t, J = 6Hz, C_{1'}-H)
4.55-4.35 (5H, m, C_{3'}·_{5'}-H, 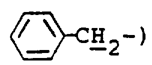 4.28-4.18 (1H, m, C_{4'}-H)
2.73-2.47, 2.16-1.87 (2H, m, C_{2'}-H)
2.38 (3H, s,

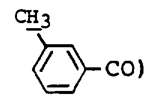

### Example 33 (for comparison)

### 3'-O-benzyl-5'-O-(4-n-propoxybenzoyl)-2'-deoxy-5-fluorouridine (R¹ = C₆H₅CH_{2'} R = 4-n-propoxybenzoyl, R³ = H)

Yield 78 %
M.p.-(oily)
¹H-NMR(CDCl₃) δ:
9.27 (1H, bs, -NH-, disappeared by addition of D₂O)
7.91 (2H, d, J = 9Hz, CO 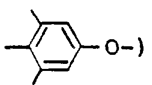 7.62 (1H, d, J = 6Hz, C₆-H)
7.32 (5H, s, 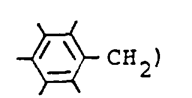 6.91 (2H, d, J = 9Hz, CO 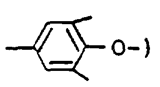 6.25 (1H, t, J = 6Hz, C_{1'}-H)
4.55-4.52 (4H, m, 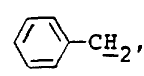 C_{5'}-H)
4.45-4.39 (1H, m, C_{3'}-H)
4.29-4.15 (1H, m, C_{4'}-H)
3.97 (2H, t, J = 7Hz, -CH₂O-)
2.74-2.52, 2.16-1.64 (4H, m, CH₃CH₂CH₂-O-, C₂,-H)
1.04 (3H, t, J = 7Hz, CH₃CH₂-)

### Example 34 (for comparison)

### 3'-O-benzyl-5'-O-phenoxymethylcarbonyl-2'-deoxy-5-fluorouridine (R¹ = C₆H₅CH₂, R = phenoxymethylcarbonyl, R³ = H)

Yield 90 %
M.p.-(oily)
¹H-NMR (CDCl₃) δ:
10.03 (1H, bs, -NH-, disappeared by addition of D₂O)
7.58 (1H, d, J = 6Hz, C₆-H)
7.35-6.77 (10H, m, phenyl-H)
6.22 (1H, t, J = 6Hz, C_{1'}-H)
4.65 (2H, s, 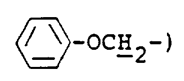 4.42-4.23 (5H, m, C_{3'}·_{5'}-H, 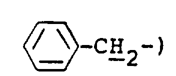 3.97-3.84 (1H, m, C_{4'}-H)
2.49-2.23, 1.96-1.65 (1H, m, C_{2'}-H)

### Example 35 (for comparison)

### 3'-O-benzyl-5'-O-α-naphthylcarbonyl-2'-deoxy-5-fluorouridine (R¹ = C₆H₅CH₂, R = α-naphthylcarbonyl, R³ = H)

Yield 48 %
M.p. 158-160°C
¹H-NMR (CDCl₃) δ:
9.20 (1H, bs, -NH-, disappeared by addition of D₂O)
8.87-8.73 (1H, m, 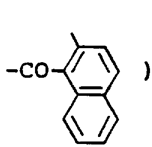 8.10-7.38 (12H, m, C₆-H, 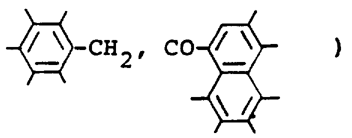 6.25 (1H, t, J = 6Hz, C_{1'}-H)
4.68-4.56 (4H, m, C_{5'}-H, 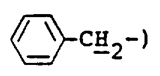 4.45-4.38 (1H, m, C_{3'}-H)
4.32-4.18 (1H, m, C_{4'}-H)
2.70-2.43, 2.17-1.86 (2H, m, C_{2'}-H)

### Example 36 (for comparison)

### 3'-O-(4-chlorobenzoyl)-5'-O-benzyl-2'-deoxy-5-fluorouridine (R¹ = 4-chlorobenzoyl, R = C₆H₅CH₂, R₃ = H)

Yield 57 %
M.p. 215-217°C
¹H-NMR(DMSO-d₆) δ:
11.83 (1H, bs, -NH-, disappeared by addition of D₂O)
8.06-7.97 (3H, m, C₆-H, CO 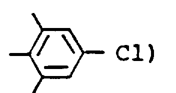 7.61 (2H, d, J = 8Hz, 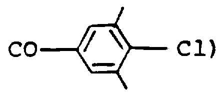 7.35 (5H, s, 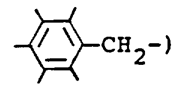 6.27 (1H, t, J = 6Hz, C_{1'}-H)
5.54-5.47 (1H, m, C_{3'}-H)
4.60 (2H, s, 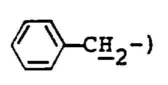 4.42-4.32 (1H, m, C_{4'}-H)
3.80-3.71 (2H, m, C_{5'}-H)
2.52-2.38 (2H, m, C_{2'}-H)

### Example 37 (for comparison)

### 3'-O-benzyl-5'-O-(3,4,5-trimethoxybenzoyl)-2'-deoxy-5-fluorouridine (R¹ = C₆H₅CH₂, R = 3,4,5-trimethoxybenzoyl, R³ = H)

Yield 63 %
¹H-NMR (DMSO-d₆) δ:
11.87 (1H, s, -NH-, disappeared by addition of D₂O)
7.93 (1H, d, J = 7Hz, C₆-H)
7.33 (5H, s, 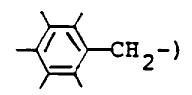 7.25 (2H, 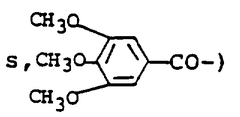 6.17 (1H, t, J = 7Hz, C_{1'}-H)
4.61-4.35 (6H, m, C_{3'}·_{4'}·_{5'}-H, 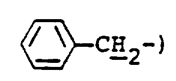 3.82, 3.77 (total 9H, each 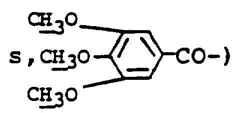 2.54-2.39 (2H, m, C_{2'}-H)

### Example 38

### 3'-O-(4-chlorobenzyl)-5'-O-acetyl-2'-deoxy-5-fluorouridine (R¹ = 4-chlorobenzyl, R = CH₃CO, R³ = H)

Yield 90 %
¹H-NMR(CDCl₃) δ:
7.67 (1H, d, J = 6Hz, C₆-H)
7.28 (5H, s, phenyl-H)
6.22 (1H, t, J = 6Hz, C_{1'}-H)
4.50 (2H, s, 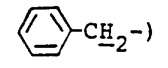 4.40-4.11 (4H, m, C_{3'}·_{4'}·_{5'}-H)
2.71-2.46, 2.28-1.93 (5H, m, C_{2'}-H, CH₃CO-)

### Example 39 (for comparison)

### Preparation of 3'-O-benzyl-2'-deoxy-5-fluoro-3-phenoxycarbonyluridine (R¹ = C₆H₅CH₂, R = H, R³ = phenoxycarbonyl)

To a solution of 0.50 g of 3'-O-benzyl-2'-deoxy-5-fluorouridine in 20 ml of dioxane were added 0.38 ml of trimethylchlorosilane and 1.04 ml of triethylamine, and the mixture was stirred at room temperature for 2 hours. Then, the resulting mixture was left to stand at 60°C for 30 minutes. To the reaction mixture were added 0.40 g of phenoxycarbonylchloride and 1.00 ml of triethylamine, and the mixture was left to stand at 60°C for 3 hours. The solvent was distilled off and the residue was dissolved in 50 ml of ethyl acetate. Then, the solution was washed with saturated aqueous solution of sodium chloride. The ethyl acetate layer was separated and concentrated. The residue was dissolved in 30 ml of methanol and 0.5 ml of acetic acid was added thereto. The mixture was left to stand overnight and the resulting mixture was concentrated. The residue was applied to a silica gel column to conduct gradient elution using chloroform and mixtures of methanol (up to 2 %) and chloroform, giving 0.58 g of the title compound in a yield of 86 %.
M.p. 110-112°C
¹H-NMR (CDCl₃) δ:
8.16 (1H, d, J = 7Hz, C₆-H)
7.34-7.22 (10H, m, phenyl-H)
6.27 (1H, t, J = 6Hz, C_{1'}-H)
4.49 (2H, s, 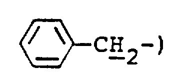 4.26-4.17 (2H, m, C_{3'}·_{4'}-H)
3.95-3.60 (2H, m, C_{5'}-H)
2.63-1.98 (2H, m, C_{2'}-H)
Elementary Analysis: for C₂₃H₂₁FN₂O₇
Calcd. (%) C 60.53; H 4.64; N 6.14
Found (%) C 60.60; H 4.72; N 6.08

### Example 40 (for comparison)

The general procedure of Example 39 was followed, thereby giving the following compound.

### 3'-O-benzyl-2'-deoxy-3-(4-propoxybenzoyl)-5-fluorouridine (R¹ = C₆H₅CH₂, R = H, R³ = 4-propoxybenzoyl)

Yield 65 %
M.p.-(glassy powder)
¹H-NMR(CDCl₃) δ:
8.19 (1H, d, J = 7Hz, C₆-H)
7.85 (2H, d, J = 9Hz, 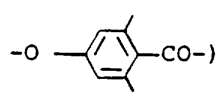 7.27 (5H, s, 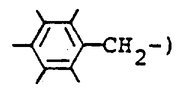 6.90 (2H, d, J = 9Hz, 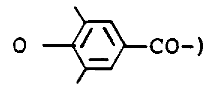 6.25 (1H, t, J = 6Hz, C_{1'}-H)
4.44 (2H, s, 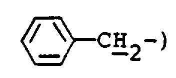 4.20-3.55 (6H, m, C_{3'}·_{4'}·_{5'}-H, -CH₂CH₂O-)
2.57-1.58 (4H, m, C_{2'}-H, CH₃CH₂CH₂O-)
0.99 (3H, t, J = 7Hz, CH₃CH₂-)

### Example 41 (for comparison)

### Preparation of 3'-O-benzyl-2'-deoxy-3-benzoyl-5-fluorouridine (R¹ = C₆H₅CH₂, R = H, R³ = C₆H₅CO)

To a solution of 0.50 g of 3′-O-benzyl-2′-deoxy-5-fluorouridine in 20 ml of dioxane were added 0.75 ml of trimethylchlorosilane and 2.00 ml of triethylamine, and the mixture was stirred at room temperature for 2 hours. Then, the resulting mixture was left to stand at 60°C for 30 minutes. To the reaction mixture were added 0.42 g of benzoyl bromide and 1.00 ml of triethylamine, and the mixture was left to stand at 60°C for 1 hour. The solvent was distilled off and the residue was dissolved in 50 ml of ethyl acetate. Then, the solution was washed with saturated aqueous solution of sodium chloride. The ethyl acetate layer was separated and concentrated. The residue was dissolved in 30 ml of methanol and 0.5 ml of acetic acid was added thereto. The mixture was left to stand overnight. The solvent was distilled off and the residue was applied to a silica gel column to conduct gradient elution using chloroform and mixtures of methanol (up to 2 %) and chloroform, giving 0.35 g of the title compound as a powder in a yield of 54 %.
M.p.-(glassy powder)
¹H-NMR (CDCl₃)δ:
8.19 (1H, d, J = 7Hz, C₆-H)
7.94-7.85 (2H, m, 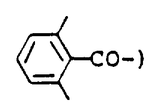 7.64-7.21 (8H, m, 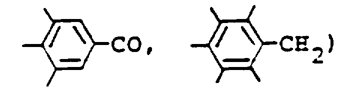 6.24 (1H, t, J = 6Hz, C_{1'}-H)
4.46 (2H, s, 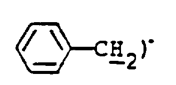 4.24-4.12 (2H, m, C_{3'}·_{4'}-H)
3.92-3.56 (2H, m, C_{5'}-H)
2.60-1.96 (2H, m, C_{2'}-H)

### Example 42 (for comparison)

### Preparation of 5'-O-acetyl-3'-O-benzyl-3-benzoyl-2'-deoxy-5-fluorouridine (R¹ = C₆H₅CH₂, R = CH₃CO, R³ = C₆H₅CO)

To a solution of 0.20 g of 5'-O-acetyl-3'-O-benzyl-2'-deoxy-5-fluorouridine in 10 ml of dioxane were added 0.29 g of benzoyl chloride and 0.73 ml of triethylamine, and the mixture was left to stand at 80°C for 2 hours. The solvent was distilled off and the residue was dissolved in 50 ml of ethyl acetate. The solution was washed with water. The ethyl acetate layer was dried on anhydrous sodium sulfate and concentrated. The concentrate was placed on a silica gel column and eluted with chloroform, giving 0.2 g of the title compound as an oil in a yield of 78 %.
¹H-NMR(CDCl₃) δ:
7.95-7.27 (11H, m, phenyl-H, C₆-H)
6.20 (1H, t, J = 6Hz, C_{1'}-H)
4.45 (2H, s, 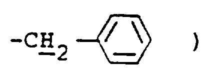 4.23-4.08 (4H, m, C_{3'}·_{4'}·_{5'}-H)
2.60-2.05 (5H, m, C_{2'}-H, COCH₃)
Elementary Analysis: for C₂₅H₂₃FN₂O₇
Calcd. (%) C 62.24; H 4.80; N 5.81
Found (%) C 62.34; H 5.06; N 5.77

### Examples 43-53 (for comparison)

The general procedure of Example 42 was followed, thereby giving the following compounds.

### Example 43 (for comparison)

### 3'-O-benzyl-5'-O-acetyl-2'-deoxy-3-(4-propokybenzoyl)-5-fluorouridine (R¹ = C₆H₅CH₂, R = CH₃CO, R³ = 4-propoxybenzoyl)

Yield 38 %
M.p.-(oily)
¹H-NMR (CDCl₃) δ:
7.85 (2H, d, J = 9Hz, 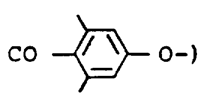 7.75 (1H, d, J = 7Hz, C₆-H)
7.30 (5H, s, 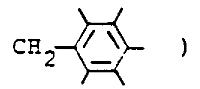 6.92 (2H, d, J = 9Hz, 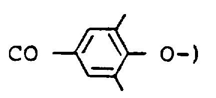 6.20 (1H, t, J = 6Hz, C_{1'}-H)
4.45 (2H, s, 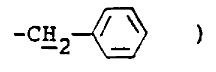 4.23-4.08 (4H, m, C_{3'}·_{4'}·_{5'}-H)
3.96 (2H, t, J = 6Hz, -OCH₂CH₂CH₃)
2.70-1.68 (7H, m, C_{2'}-H, COCH₃, -OCH₂CH₂CH₃)
1.01 (3H, t, J = 7Hz, -O(CH₂)₂CH₃)

### Example 44 (for comparison)

### 3'-O-benzyl-5'-O-acetyl-2'-deoxy-3-(4-chlorobenzoyl)-5-fluorouridine (R¹ = C₆H₅CH₂, R = CH₃CO, R³ = 4-chlorobenzoyl)

Yield 73 %
M.p.-(oily)
¹H-NMR (CDCl₃) δ:
7.84 (2H, d, J = 9Hz, 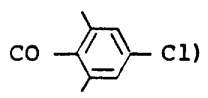 7.78 (1H, d, J = 6Hz, C₆-H)
7.44 (2H, d, J = 9Hz, 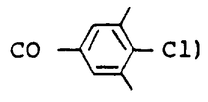 7.30 (5H, s, 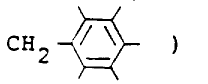 6.20 (1H, t, J = 6Hz, C_{1'}-H)
4.51 (2H, d, J = 1Hz, 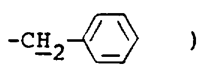 4.28-4.08 (4H, m, C_{3'}·_{4'}·_{5'}-H)
2.70-2.43, 2.25-2.03 (5H, m, C_{2'}-H, COCH₃)

### Example 45 (for comparison)

### 3'-O-benzyl-5'-O-acetyl-2'-deoxy-3-(2-methylbenzoyl)-5-fluorouridine (R¹ = C₆H₅CH₂, R = CH₃CO, R³ = 2-methylbenzoyl)

Yield 44 %
M.p.-(oily)
¹H-NMR(CDCl₃) δ:
7.62-7.32 (10H, m, C₆-H, phenyl-H)
6.19 (1H, t, J = 7Hz, C_{1'}-H)
4.47 (2H, d, J = 1Hz, 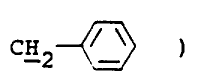 4.25-4.07 (4H, m, C_{3'}·_{4'}·_{5'}-H)
2.67-2.00 (8H, m, C_{2'}-H, COCH₃,

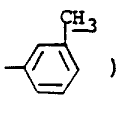

### Example 46 (for comparison)

### 3'-O-benzyl-5'-O-acetyl-2'-deoxy-3-(3-methylbenzoyl)-5-fluorouridine (R¹ = C₆H₅CH₂, R = CH₃CO, R³ = 3-methylbenzoyl)

Yield 68 %
M.p.-(oily)
¹H-NMR(CDCl₃) δ:
7.81-7.65 (3H, m, C₆-H, 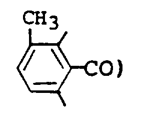 7.47-7.16 (7H, m, 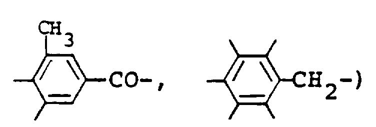 6.20 (1H, t, J = 6Hz, C_{1'}-H)
4.46 (2H, s, 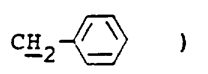 4.25-4.08 (4H, m, C_{3'}·_{4'}·₅'-H)
2.67-2.00 (8H, m, C_{2'}-H, COCH₃,

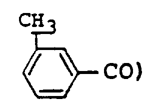

### Example 47 (for comparison)

### 3'-O-benzyl-5'-acetyl-2'-deoxy-3-(4-methylbenzoyl)-5-fluorouridine (R¹ = C₆H₅CH₂, R = CH₃CO, R³ = 4-methylbenzoyl)

Yield 84 %
M.p.-(oily)
¹H-NMR(CDCl₃) δ:
7.79 (2H, d, J = 8Hz, 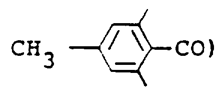 7.78 (1H, d, J = 7Hz, C₆-H)
7.27 (5H, s, 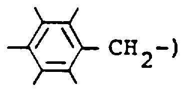 7.22 (2H, d, J = 8Hz, 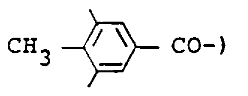 6.20 (1H, t, J = 6Hz, C_{1'}-H)
4.45 (2H, s, 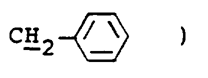 4.24-4.08 (4H, m, C_{3'}·_{4'}·_{5'}-H)
2.60-2.05 (8H, m, C_{2'}-H, COCH₃,

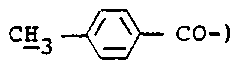

### Example 48 (for comparison)

### 3'-O-benzyl-5'-O-acetyl-2'-deoxy-3-(4-methoxybenzoyl)-5-fluorouridine (R¹ = C₆H₅CH₂, R = CH₃CO, R³ = 4-methoxybenzoyl)

Yield 70 %
M.p.-(powder)
¹H-NMR(CDCl₃) δ:
7.85 (2H, d, J = 8Hz, 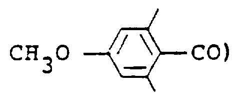 7.77 (1H, d, J = 7Hz, C₆-H)
7.28 (5H, s, 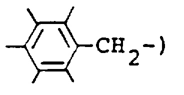 6.91 (2H, d, J = 8Hz, 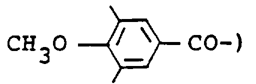 6.21 (1H, t, J = 6Hz, C_{1'}-H)
4.47 (2H, s, 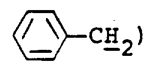 4.25-4.09 (4H, m, C_{3'}·_{4'}·_{5'}-H)
3.80 (3H, s, CH₃O-)
2.49-2.07 (5H, m, C_{2'}-H, COCH₃)

### Example 49 (for comparison)

### 3'-O-benzyl-5'-O-benzoyl-2'-deoxy-3-benzoyl-5-fluorouridine (R¹ = C₆H₅CH₂, R = R³ = C₆H₅CO)

Yield 94 %
M.p.-(oily)
¹H-NMR(CDCl₃) δ:
8.03-7.32 (16H, m, phenyl-H, C₆-H)
6.20 (1H, t, J = 6Hz, C_{1'}-H)
4.48-4.16 (6H, m, C_{3'}·_{4'}·_{5'}-H, 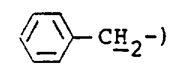 2.70-2.42, 2.25-1.95 (2H, m, C_{2'}-H)

### Example 50 (for comparison)

### 3'-O-benzyl-5'-O-phenoxycarbonyl-2'-deoxy-3-phenoxycarbonyl-5-fluorouridine (R¹= C₆H₅CH₂, R = R³ = phenoxycarbonyl)

Yield 48 %
M.p.-(oily)
¹H-NMR(DMSO-d₆) δ:
8.21 (1H, d, J = 7Hz, C₆-H)
7.57-7.16 (15H, m, phenyl-H)
6.20 (1H, t, J = 7Hz, C_{1'}-H)
4.59-4.28 (6H, m, C_{3'}·_{4'}·_{5'}-H, 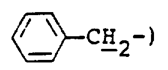 2.54-2.38 (2H, m, C_{2'}-H)

### Example 51 (for comparison)

### 3'-O-benzyl-5'-O-α-naphthylcarbonyl-2'-deoxy-3-α-naphthylcarbonyl-5-fluorouridine (R¹ = C₆H₅CH₂, R = R³ = α-naphthylcarbonyl)

Yield 29 %
M.p.-(oily)
¹H-NMR(CDCl₃) δ:
9.11-8.79 (2H, m, 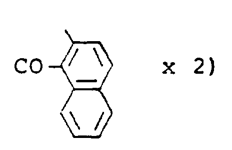 8.11-7.19 (13H, m, 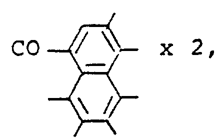 C₆-H)
6.20 (1H, t, J = 7Hz, C_{1'}-H)
4.71-4.10 (6H, m, C_{3'}·_{4'}·_{5'}-H, 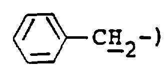 2.64-2.36, 2.15-1.85 (2H, m, C_{2'}-H)

### Example 52 (for comparison)

### 3'-O-benzyl-5'-O-(3-methylbenzoyl)-2'-deoxy-3-(3-methylbenzoyl)-5-fluorouridine (R¹ = C₆H₅CH₂, R = R³ = 3-methylbenzoyl)

Yield 18 %
M.p.-(oily)
¹H-NMR(CDCl₃) δ:
7.81-7.62 (5H, m, 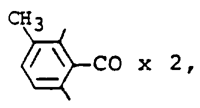 C₆-H)
7.43-7.24 (9H, m, 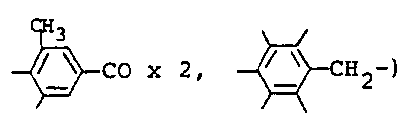
6.23 (1H, t, J = 6Hz, C_{1'}-H)
4.60-4.20 (6H, m, C_{3'}·_{4'}·_{5'}-H, 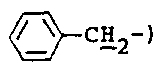 2.78-2.50, 2.24-1.93 (2H, m, C_{2'}-H)
2.39, 2.37 (each s, 3H,

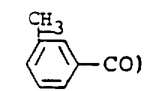

### Example 53 (for comparison)

### 3'-O-benzyl-5'-O-acetyl-2'-deoxy-3-hexanoyl-5-fluorouridine (R¹ = C₆H₅CH₂, R = CH₃CO, R³ = hexanoyl)

Yield 48 %
M.p.-(oily)
¹H-NMR(CDCl₃) δ:
7.66 (1H, d, J = 6Hz, C₆-H)
7.32 (5H, s, phenyl-H)
6.20 (1H, t, J = 6Hz, C_{1'}-H)
4.54 (2H, s, 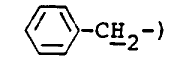 4.38-4.07 (4H, m, C_{3'}·_{4'}·_{5'}-H)
2.82 (2H, t, J = 9Hz, -CH₂-CO-)
2.59-2.44, 2.22-2.02 (5H, m, C_{2'}-H and CH₃CO-)
1.92-1.67 (2H, m,-CH₂CH₂CO-)
1.56-1.22 (4H, m, CH₃CH₂CH₂CH₂CH₂CO-)
0.90 (3H, t, J = 5Hz, CH₃CH₂)

### Example 54 (for comparison)

### Preparation of 3'-O-benzyl-5'-O-acetyl-2'-deoxy-5-fluorouridine (R¹ = C₆H₅CH₂, R = CH₃CO, R³ = H)

A 3.33 ml quantity of acetic anhydride was added to a solution of 3.95 g of 3'-O-benzyl-2'-deoxy-5-fluorouridine in 30 ml of pyridine, and the mixture was left to stand at 40°C overnight. The solvent was distilled off and the residue was dissolved in 30 ml of ethyl acetate. The solution was washed twice with 15 ml of water. The ethyl acetate layer was dried on anhydrous sodium sulfate and concentrated. The concentrate was placed on a silica gel column and eluted with chloroform, giving 3.62 g of the title compound in a yield of 81.5 %.
M.p. 87-88°C ¹H-NMR(DMSO-d₆) δ:
11.86 (1H, d, J = 4Hz, -NH-, disappeared by addition of D₂O)
7.93 (1H, d, J = 7Hz, C₆-H)
7.35 (5H, s, phenyl-H)
6.15 (1H, t, J = 6Hz, C_{1'}-H)
4.55 (2H, s, 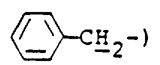 4.32-4.20 (4H, m, C_{3'}·_{4'}·_{5'}-H)
2.39-2.28 (2H, t, J = 6Hz, C_{2'}-H)
2.04 (3H, s, COCH₃)
Elementary Analysis: for C₁₈H₁₉FN₂O₆
Calcd. (%) C 57.14; H 5.06; N 7.40
Found (%) C 56.99; H 5.22; N 7.37

### Example 55 (for comparison)

### Preparation of 3'-O-acetyl-5'-O-benzyl-2'-deoxy-5-fluorouridine (R¹ = CH₃CO, R = C₆H₅CH₂, R³ = H)

Following the general procedure of Example 54 and using 1.00 g of 5'-O-benzyl-2'-deoxy-5-fluorouridine, 1.00 g of the title compound was prepared in a yield of 88.9 %.
M.p. 114-116°C
¹H-NMR(DMSO-d₆) δ:
11.85 (1H, bs, -NH-, disappeared by addition of D₂O)
7.95 (1H, d, J = 7Hz, C₆-H)
7.34 (5H, s, phenyl-H)
6.17 (1H, t, J = 6Hz, C_{1'}-H)
5.25-5.23 (1H, m, C_{3'}-H)
4.57 (2H, s, 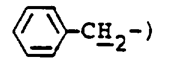 4.32-4.20 (1H, m, C_{4'}-H)
3.84-3.73 (2H, m, C_{5'}-H)
2.37-2.25 (2H, m, C_{2'}-H)
2.06 (3H, s, COCH₃)
Elementary Analysis: for C₁₈H₁₉FN₂O₆
Calcd. (%) C 57.14; H 5.06; N 7.40
Found (%) C 56.91; H 5.32; N 7.25

### Example 56 (for comparison)

### Preparation of 3'-O-benzyl-5'-O-chloroacetyl-2'-deoxy-5-fluorouridine (R¹ = C₆H₅CH₂, R = CH₂Cl-CO, R³ = H)

Chloroacetic anhydride was added to a solution of 0.20 g of 3'-O-benzyl-2'-deoxy-5-fluorouridine in 10 ml of pyridine, and the mixture was left to stand at room temperature overnight. Then the same subsequent procedures as in Example 54 were conducted, giving 0.11 g of the title compound as an oil in a yield of 45 %.
¹H-NMR(CDCl₃) δ:
10.22 (1H, bs, -NH-, disappeared by addition of D₂O)
7.60 (1H, d, J = 6Hz, C₆-H)
7.32 (5H, s, phenyl-H)
6.23 (1H, t, J = 6Hz, C_{1'}-H)
4.53 (2H, d, J = 3Hz, 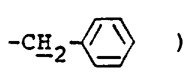 4.45 -4.08 (6H, m, C_{3'}·_{4'}·_{5'}-H, ClCH₂CO-)
2.69-2.06 (2H, m, C_{2'}-H)
Elementary Analysis: for C₁₈H₁₈ClFN₂O₆
Calcd. (%) C 52.37; H 4.39; N 6.79
Found (%) C 52.43; H 4.63; N 6.80

### Example 57 (for comparison)

### Preparation of 3'-O-benzyl-2'-deoxy-3-(2-tetrahydrofuranyl)-5-fluorouridine (R¹ = C₆H₅CH₅, R = H, R³ = 2-tetrahydrofuranyl)

Following the general procedure of Reference Example 2 and using 0.40 g of 3'-O-benzyl-2'-deoxy-5-fluorouridine, 0.37 g of the title compound was prepared as an oil in a yield of 77 %.
¹H-NMR(CDCl₃) δ:
8.01 (1H, d, J = 6Hz, C₆-H)
7.30 (5H, s, phenyl-H)
6.58 (1H, bt, J = 6Hz, 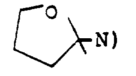 6.26 (1H, bt, J = 6Hz, C_{1'}-H)
4.51 (2H, s, 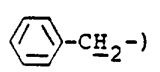 4.39-3.50 (7H, m, C_{3'}·_{4'}·_{5'}-H, C_{6'}-OH, 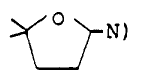 2.60-1.86 (6H, m, C_{2'}-H, 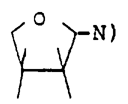 Elementary Analysis: for C₂₀H₂₃FN₂O₆
Calcd (%) C 59.11; H 5.70; N 6.89
Found (%) C 59.02; H 6.11; N 6.78

### Example 58

### Preparation of 5'-O-acetyl-3'-O-benzyl-2'-deoxy-5-fluoro-3-[3-(2-hydroxy-4-pyridyloxycarbonyl)benzoyl]-uridine

To a solution of 3.00 g of 5'-O-acetyl-3'-O-benzyl-2′-deoxy-5-fluorouridine in 100 ml of dry dioxane were added 2.40 g of isophthaloyl chloride and 8.5 ml of triethylamine, and the mixture was refluxed for 2 hours. Then, to the reaction mixture were added a suspension of 1.77 g of 4-hydroxy-2(1H)-pyridone and 9.60 ml of triethylamine in 100 ml of dry dioxane, and the mixture was refluxed for 2 hours. The insolubles were removed by filtration and the filtrate was concentrated. The residue was dissolved in 100 ml of ethyl acetate, and the solution was washed twice with 30 ml of saturated aqueous solution of sodium chloride. The ethyl acetate layer was dried on anhydrous sodium sulfate and concentrated. The residue was placed on a silica gel column to conduct a gradient elution using chloroform and mixtures of methanol (up to 2 %) and chloroform. The fractions corresponding to the title compound were collected and concentrated to dryness, giving 1.48 g of the title compound as a powder in a yield of 30 %.
¹H-NMR(CDCl₃)δ:
8.62-7.31 (11H, m, phenyl-H, C₆-H and C₆-H of the pyridine ring)
6.50 (1H, d, J = 2Hz, C₃-H of the pyridine ring)
6.31 (1H, dd, J₅·₆ = 7Hz, J₃·₅ = 2Hz, C₅-H of the pyridine ring)
6.23 (1H, coalesced in part with the signal of C₅-H of the pyridine ring, C_{1'}-H)
4.52 (2H, d, 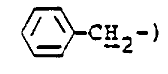 4.30-4.13 (4H, m, C_{3'}·_{4'}·_{5'}-H)
2.53-2.06 (5H, m, C_{2'}-H and COCH₃)

### Example 59

### Preparation of 5'-O-acetyl-3'-O-benzyl-3-[3-(5-chloro-4-hydroxy-2-pyridyloxycarbonyl)benzoyl]-2'-deoxy-5-fluorouridine

To a solution of 1.00 g of 5'-O-acetyl-3'-O-benzyl-2'-deoxy-5-fluorouridine in 50 ml of dry dioxane were added 0.80 g of isophthaloyl chloride and 2.95 ml of triethylamine, and the mixture was refluxed for 2 hours. The insolubles were removed by filtration and the filtrate was concentrated. The residue was dissolved in 50 ml of dry dichloromethane. To the solution was added 1.35 g of 2,4-bis(trimethylsilyloxy)-5-chloropyridine, and the mixture was stirred at room temperature overnight. The solvent was distilled off and the residue was dissolved in 50 ml of ethyl acetate. The solution was washed three times with saturated aqueous solution of hydrogencarbonate and washed three times with saturated aqueous solution of sodium chloride. The ethyl acetate layer was dried on anhydrous sodium sulfate and concentrated. The residue was placed on a silica gel column to conduct a gradient elution using chloroform and mixtures of methanol (up to 1 %) and chloroform. The fractions corresponding to the title compound were collected and concentrated to dryness, giving 0.30 g of the title compound in a yield of 17 % as a powder.
¹H-NMR(CDCl₃)δ:
8.61-7.31 (11H, m, phenyl-H, C₆-H and C₆-H of the pyridine ring)
6.82 (1H, s, C₃-H of the pyridine ring)
6.19 (1H, t, J = 6Hz, C_{1′}-H)
4.52 (2H, d, 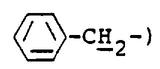 4.29-3.95 (4H, m, C_{3'}·_{4'}·_{5'}-H)
2.74-2.10 (5H, m, C_{2′}-H and COCH₃)

### Example 60

### Preparation of 5′-O-acetyl-3′-O-benzyl-2′-deoxy-3-[3-(1,2-dihydro-2-oxo-1-(2-tetrahydrofuranyl)-4-pyridyloxycarbonyl)benzoyl]-5-fluorouridine

Following the general procedure of Example 58 and using 4-hydroxy-1-(2-tetrahydrofuranyl)-2(1H)-pyridone in place of the 4-hydroxy-2(1H)-pyridone, the title compound was prepared as a powder in a yield of 41 %.
¹H-NMR(CDCl₃)δ:
8.61-7.65 (5H, m, 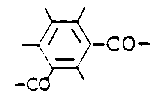 and C₆-H)
7.53 (1H, d, J = 8Hz, C₆-H of the pyridine ring)
7.29 (5H, s, 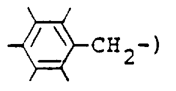 6.40 (1H, d, J = 2Hz, C₃-H of the pyridine ring)
6.27-6.12 (3H, m, C_{1′}-H, 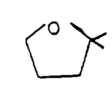 and C₅-H of the pyridine ring)
4.50 (2H, s, 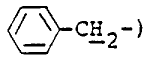 4.27-3.92 (6H, m, C_{3'}·_{4'}·_{5'}-H and 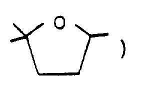 2.50-1.98 (9H, m, C_{2′}-H, COCH₃ and

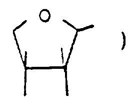

### Example 61

### Preparation of 5′-O-acetyl-3′-O-benzyl-3-[3-(5-chloro-1,2-dihydro-2-oxo-1-(2-tetrahydrofuranyl)-4-pyridyloxycarbonyl)benzoyl]-2′-deoxy-5-fluorouridine

Following the general procedure of Example 58 and using 3-chloro-4-hydroxy-1-(2-tetrahydrofuranyl)-2(1H)-pyridone in place of 4-hydroxy-2(1H)-pyridone, the title compound was prepared as a powder in a yield of 72 %.
¹H-NMR(CDCl₃) δ:
8.93-7.58 (6H, m, C₆-H, C₆-H of the pyridine ring
and 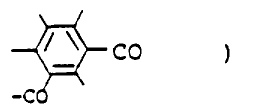 7.21 (5H, s, 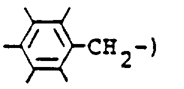 6.58 (1H, s, C₃-H of the pyridine ring)
6.20-6.08 (2H, m, C_{1'}-H and 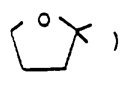 4.50 (2H, s, 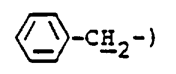 4.36-3.97 (6H, m, C_{3'}·_{4'}·_{5'}-H and 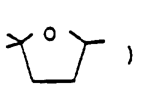 2.55-1.92 (9H, m, C_{2'}-H, COCH₃ and

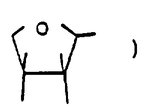

### Example 62

### Preparation of 5'-O-acetyl-3'-O-benzyl-2'-deoxy-5-fluoro3-[3-(1,2-dihydro-1-ethoxymethyl-2-oxo-4-pyridyloxycarbonyl)benzoyl]uridine

Following the general procedure of Example 58 and using 1-ethoxymethyl-4-hydroxy-2(1H)-pyridone in place of 4-hydroxy-2(1H)-pyridone, the title compound was prepared in a yield of 32 % as a powder.
¹H-NMR(CDCl₃)δ:
8.70-7.31 (11H, m, phenyl-H, C₆-H and C₆-H of the pyridine ring)
6.47 (1H, bs, C₃-H of the pyridine ring)
6.20-6.10 (2H, m, C_{1′}-H and C₅-H of the pyridine ring)
5.36 (2H, s, N-CH₂OC₂H₅)
4.52-4.00 (6H, m, C_{3'}·_{4'}·_{5'}-H and 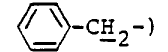 3.63 (2H, q, J = 7Hz, -OCH₂CH₃)
2.80-1.90 (5H, m, C_{2′}-H and COCH₃)
1.22 (3H, t, J = 7Hz, -OCH₂CH₃)

### Example 63

### Preparation of 5′-O-acetyl-3′-O-benzyl-3-[3-(5-chloro-1,2-dihydro-1-ethoxymethyl-2-oxo-4-pyridyloxycarbonyl)benzoyl]-2′-deoxy-5-fluorouridine

Following the general procedure of Example 58 and using 5-chloro-1-ethoxymethyl-4-hydroxy-2(1H)-pyridone in place of 4-hydroxy-2(1H)-pyridone, the title compound was prepared in a yield of 20 % as a powder.
¹H-NMR(CDCl₃)δ:
8.66-7.68 (5H, m, C₆-H and 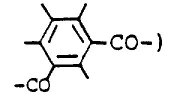 7.59 (1H, s, C₆-H of the pyridine ring)
7.30 (5H, s, 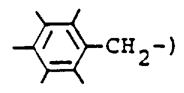 6.64 (1H, s, C₃-H of the pyridine ring)
6.21 (1H, t, J = 6Hz, C_{1′}-H)
5.31 (2H, s, N-CH₂OC₂H₅)
4.50 (2H, s, 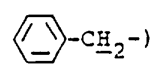 4.28-4.06 (4H, m, C_{3'}·_{4'}·_{5'}-H)
3.63 (2H, q, J = 7Hz, -O-CH₂CH₃)
2.58-2.02 (5H, m, C_{2′}-H and COCH₃)
1.22 (3H, t, J = 7Hz, -OCH₂CH₃)

### Example 64

### Preparation of 5′-O-acetyl-3-[3-(2-benzoyloxy-5-chloro-4-pyridyloxycarbonyl)benzoyl]-3′-O-benzyl-2′-deoxy-5-fluorouridine

Following the general procedure of Example 58 and using 2-benzoyloxy-5-chloro-4-hydroxypyridine in place of 4-hydroxy-2(1H)-pyridone, the title compound was prepared in a yield of 27 % as a powder.
¹H-NMR(CDCl₃)δ:
8.68-7.41 (12H, m, C₆-H, C₃·₆-H of the pyridine ring, 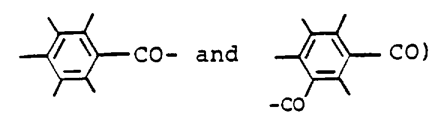 7.28 (5H, s, 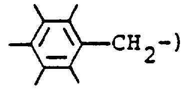 6.20 (1H, t, J = 6Hz, C_{1'}-H)
4.49 (2H, s, 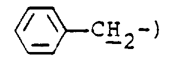 4.27-4.04 (4H, m, C_{3'}·_{4'}·_{5'},-H)
2.70-2.07 (5H, m, C_{2'}-H and COCH₃)

### Example 65

### Preparation of 5'-O-acetyl-3'-O-benzyl-2'-deoxy-3-[3-(1,2-dihydro-1-carbomethoxymethyicarbamoyl-2-oxo-4-pyridyloxycarbonyl)benzoyl]-5-fluorouridine

The general procedure of Example 58 was followed using 4-hydroxy-1-carbomethoxymethylcarbamoyl-2(1H)-pyridone in place of the 4-hydroxy-2(1H)-pyridone, thereby giving the title compound as a powder in a yield of 5.7 %.
¹H-NMR(CDCl₃) δ:
10.86 (1H, t, J = 6Hz, -CONHCH₂-)
8.61-7.60 (6H, m, C₆-H, 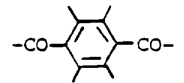 and C₆-H of the pyridine ring)
7.31 (5H, s, 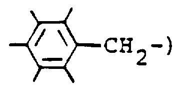 6.62 (1H, d, J = 2Hz, C₃-H of the pyridine ring)
6.44-6.14 (2H, m, C_{1'}-H and C₅-H of the pyridine ring) 4.52 (2H, d, 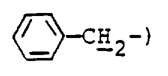
4.30-4.00 (6H, m, C_{3'}·_{4'}·₅'-H and -NHCH₂COOCH₃)
3.78 (3H, s, -COOCH₃)
2.76-1.92 (5H, m, C_{2′}-H and COCH₃)

### Example 66

### Preparation of 5′-O-acetyl-3′-O-benzyl-3-[4-[5-chloro-1,2-dihydro-2-oxo-1-(2-tetrahydrofuranyl)-4-pyridyloxycarbonyl]benzoyl]-2′-deoxy-5-fluorouridine

The general procedure of Example 58 was followed using terephthaloyl chloride in place of isophthaloyl chloride and using 5-chloro-4-hydroxy-1-(2-tetrahydrofuranyl)-2(1H)-pyridone in place of 4-hydroxy-2(1H)-pyridone, thereby producing the title compound as a powder in a yield of 38 %.
¹H-NMR(DMSO-d₆)δ:
8.50-8.20 (5H, m, C₆-H and 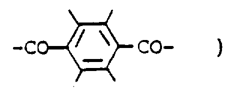 7.93 (1H, s, C₆-H of the pyridine ring)
7.34 (5H, s, 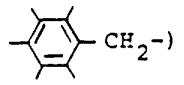 6.66 (1H, s, C₃-H of the pyridine ring)
6.20-6.10 (2H, m, C_{1′}-H and 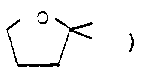 4.55-3.70 (8H, m, C_{3'}·_{4'}·_{5'}-H, 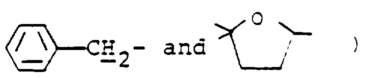 2.60-1.80 (9H, m, C_{2′}-H, 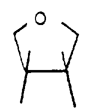 and COCH₃)

### Example 67

### Preparation of 3-benzoyl-3′-O-benzyl-5′-O-[3-(5-chloro-4-hydroxy-2-pyridyloxycarbonyl)benzoyl]-2′-deoxy-5-fluorouridine

The general procedure of Example 59 was followed using 3-benzoyl-3′-O-benzyl-2′-deoxy-5-fluorouridine in place of 5′-O-acetyl-3′-O-benzyl-2′-deoxy-5-fluorouridine, thereby producing the title compound as a powder in a yield of 15 %.
¹H-NMR(CDCl₃)δ:
8.73-7.34 (11H, m, C₆-H, 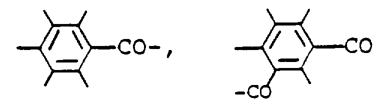 and C₆-H of the pyridine ring)
7.28 (5H, s, 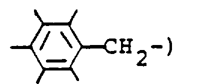 6.77 (1H, s, C₃-H of the pyridine ring)
6.21 (1H, t, J = 7Hz, C_{1′} -H)
4.70-4.20 (6H, m, C_{3'}·_{4'}·_{5'}-H and 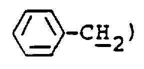 2.60-2.13 (2H, m, C_{2′}-H)

### Example 68

### Preparation of 3-benzoyl-3′-O-benzyl-5′-O-[3-[5-chloro-1,2-dihydro-2-oxo-1-(2-tetrahydrofuranyl)-4-pyridyloxycarbonyl]benzoyl]-2′-deoxy-5-fluorouridine

The general procedure of Example 58 was followed using 3-benzoyl-3′-O-benzyl-2′-deoxy-5-fluorouridine in place of 5′-O-acetyl-3′-O-benzyl-2′-deoxy-5-fluorouridine and using 5-chloro-4-hydroxy-1-(2-tetrahydrofuranyl)-2(1H)-pyridone in place of 4-hydroxy-2(1H)-pyridone, thereby producing the title compound as a powder in a yield of 46 %.
¹H-NMR(CDCl₃)δ:
8.77-7.34 (11H, m, C₆-H, 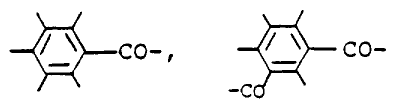 and C₆-H of the pyridine ring)
7.26 (5H, s, 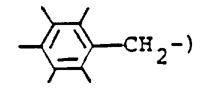 6.57 (1H, s, C₃-H of the pyridine ring)
6.21-6.06 (2H, m, C_{1′}-H and 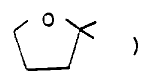 4.53 (2H, s, 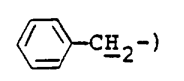 4.53-4.02 (6H, m, C_{3'}·_{4'}·_{5'}-H and 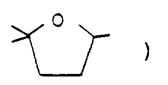 2.55-1.96 (6H, m, C_{2′}-H and

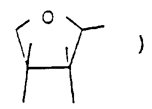

### Example 69

### Preparation of 5′-O-acetyl-3′-O-benzyl-2′-deoxy-3-[3-(1,2-dihydro-2-oxo-1-phthalidyl-4-pyridyloxycarbonyl)benzoyl]-5-fluorouridine

The general procedure of Example 58 was followed using 4-hydroxy-1-phthalidyl-2(1H)-pyridone in place of 4-hydroxy-2(1H)-pyridone, thereby producing the title compound in a yield of 19 %.
¹H-NMR(CDCl₃)δ:
8.60-7.57 (10H m, C₆-H, 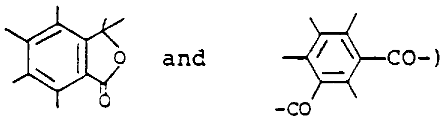 7.32 (5H, s, 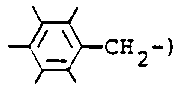 6.90 (1H, d, J = 8Hz, C₆-H of the pyridine ring)
6.60 (1H, d, J = 2Hz, C₃-H of the pyridine ring)
6.28-6.04 (2H, m, C_{1′}-H and C₅-H of the pyridine ring)
4.51 (2H, s, 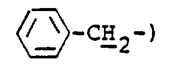 4.28-4.06 (4H, m, C_{3'}·_{4'}·_{5'}-H)
2.60-2.01 (5H, m, C_{2′}-H and COCH₃)

### Example 70

### Preparation of 5′-O-acetyl-3′-O-benzyl-3-(3-carbomethoxybenzoyl)-2′-deoxy-5-fluorouridine

The general procedure of Example 58 was followed using methanol in place of 4-hydroxy-2(1H)-pyridone, thereby giving the title compound as a powder in a yield of 69 %.
¹H-NMR(CDCl₃)δ:
8.54-7.47 (5H, m, C₆-H and 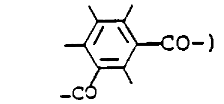 7.28 (5H, s, 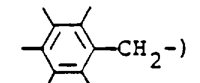 6.20 (1H, t, J = 6Hz, C_{1′}-H)
4.50 (2H, s, 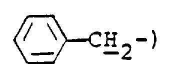 4.48-4.13 (4H, m, C_{3'}·_{4'}·_{5'}-H)
3.89 (3H, s, -COOCH₃)
2.50-1.93 (5H, m, C_{2′}-H and COCH₃)

### Example 71

### Preparation of 5′-O-acetyl-3′-O-benzyl-3-[3-(1-benzyloxymethyl-5-chloro-1,2-dihydro-2-oxo-4-pyridyloxycarbonyl)benzoyl]-2′-deoxy-5-fluorouridine

The general procedure of Example 58 was followed using 1-benzyloxymethyl-5-chloro-4-hydroxy-2(1H)-pyridone in place of 4-hydroxy-2(1H)-pyridone, thereby producing the title compound as a powder in a yield of 34 %.
¹H-NMR(CDCl₃)δ:
8.65-7.58 (6H, m, C₆-H, 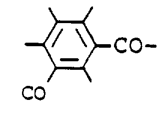 and C₆-H of the pyridine ring)
7.34 (10H, s, 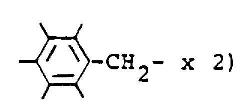 6.64 (1H, s, C₃-H of the pyridine ring)
6.22 (1H, t, J = 7Hz, C_{1′} -H)
5.42 (2H, s, -NCH₂O-)
4.66 (2H, s, 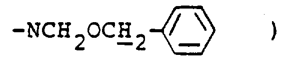 4.53 (2H, s, C₃, 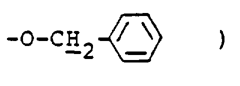 4.30-4.03 (4H, m, C_{3'}·_{4'}·_{5'}-H)
2.77-1.97 (5H, m, C_{2′}-H and COCH₃)

### Example 72

### Preparation of 5′-O-acetyl-3-[3-(2-acetoxy-5-chloro-4-pyridyloxycarbonyl)benzoyl]-3′-O-benzyl-2′-deoxy-5-fluorouridine

To a solution of 2.00 g of 5′-O-acetyl-3′-O-benzyl-2′-deoxy-5-fluorouridine in 100 ml of dry dioxane were added 1.60 g of isophthaloyl chloride and 1.47 ml of triethylamine, and the mixture was refluxed for 30 minutes. Then 4.40 ml of triethylamine was further added and the mixture was refluxed for 50 minutes. The salt formed was filtered off and the filtrate was concentrated under reduced pressure. The concentrate was dissolved in 100 ml of acetonitrile. To the solution were added 3.66 ml of triethylamine and 1.19 g of 2-acetoxy-5-chloro-4-hydroxypyridine, and the resulting mixture was stirred at room temperature for 1.5 hours. The solvent was distilled off and the residue was subjected to a silica gel column chromatography. The fractions corresponding to the title compound were collected and evaporated to dryness, giving 2.83 g of the title compound as a powder in a yield of 77 %.
¹H-NMR(CDCl₃) δ:
8.68 (6H, m, C₆-H, C₆-H of the pyridine ring and 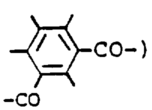 7.30 (5H, s, 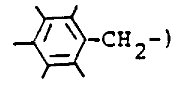 7.27 (1H, s, C₃-H of the pyridine ring)
6.20 (1H, t, J = 6Hz, C_{1'}-H)
4.51 (2H, d, 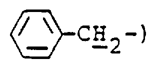 4.25-4.07 (4H, m, C_{3'}·_{4'}·_{5'}-H)
2.60-2.20 (5H, m, C_{2'}-H and -COCH₃ on the pyridine ring)
2.09 (3H, S, -COCH₃ at the 5'-position)

### Example 73

### Preparation of 5'-O-acetyl-3- [3-(4-benzoyloxy-5-chloro-2-pyridyloxycarbonyl)benzoyl]-3'-O-benzyl-2'-deoxy-5-fluorouridine

To a solution of 0.60 g of 5'-O-acetyl-3'-O-benzyl-2'-deoxy-5-fluorouridine in 30 ml of dry dioxane were added 0.42 g of isophthaloyl chloride and 1.11 ml of triethylamine, and the mixture was refluxed for 2 hours. Then 0.60 g of 4-benzoyloxy-5-chloro-2(1H)-pyridone and 1.11 ml of triethylamine were added and the resulting mixture was refluxed for 1 hour. The precipitate thus formed was filtered off, and the filtrate was concentrated. The residue was placed on a silica gel column and eluted with 40 % ethyl acetate-benzene, giving 0.45 g of the title compound in a yield of 37 %.
¹H-NMR(CDCl₃) δ:
8.66-7.28 (17H, m, phenyl-H, C₆-H and C₃·₆-H of the pyridine ring)
6.19 (1H, t, J = 6Hz, C_{1'}-H)
4.49 (2H, s, 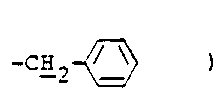 4.27-4.01 (4H, m, C_{3'}·_{4'}·_{5'}-H)
2.66-1.90 (5H, m, C_{2'}-H and COCH₃)

### Example 74

### Preparation of 5'-O-acetyl-3-[3-(4-acetyloxy-5-chloro-2-pyridyloxycarbonyl)benzoyl]-3'-O-benzyl-2'-deoxy-5-fluorouridine

The general procedure of Example 73 was followed using 4-acetoxy-5-chloro-2(1H)-pyridone in place of 4-benzoyloxy-5-chloro-2(1H)-pyridone, thereby producing the title compound in a yield of 51 %.
¹H-NMR (CDCl₃) δ:
8.65-7.17 (12H, m, phenyl-H, C₆-H and C₃·₆-H of the pyridine ring)
6.19 (1H, t, J = 6Hz, C_{1'}-H)
4.49 (2H, s, 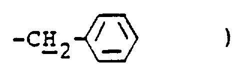 4.26-4.12 (4H, m, C_{3'}·_{4'}·₅'-H)
2.55-2.07 (8H, m, C_{2'}-H and COCH₃ x 2)

### Example 75

### Preparation of 5'-O-acetyl-3-[3-(4-benzoyloxy-2-pyridyloxycarbonyl)benzoyl]-3'-O-benzyl-2'-deoxy-5-fluorouridine

The general procedure of Example 73 was followed using 4-benzoyloxy,-2(1H)-pyridone in place of 4-benzoyloxy-5-chloro-2(1H)-pyridone, thereby producing the title compound in a yield of 55 %.
¹H-NMR(CDCl₃) δ:
8.66-7.25 (18H, m, phenyl-H, C₆-H and C₃·₅·₆-H of the pyridine ring)
6.21 (1H, t, J = 6Hz, C_{1'}-H)
4.51 (2H, s, 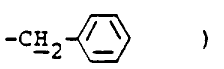 4.29-4.07 (4H, m, C_{3'}·_{4'}·_{5'}-H)
2.76-1.98 (5H, m, COCH₃ and C_{2'}-H)

### Example 76

### Preparation of 5'-O-acetyl-3-[3-(2-benzoyloxy-4-pyridyloxycarbonyl)benzoyl]-3'-O-benzyl-2'-deoxy-5-fluorouridine

The general procedure of Example 73 was followed using 2-benzoyloxy-4-hydroxypyridine in place of 4-benzoyloxy-5-chloro-2(1H)-pyridone, thereby producing the title compound in a yield of 42 %.
¹H-NMR(CDCl₃) δ:
8.66-7.23 (18H, m, phenyl-H, C₆-H and C₃·₅·₆-H of the pyridine ring)
6.22 (1H, t, J = 6Hz, C_{1'}-H)
4.52 (2H, s, 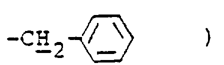 4.30-4.06 (4H, m, C_{3'}·_{4'}·_{5'}-H)
2.72-2.04 (5H, m, C_{2'}-H and COCH₃)

### Example 77

### Preparation of 5'-O-acetyl-3-[3-[2-(2-methylbenzoyloxyl-4-pyridyloxycarbonyl]benzoyl]-3'-O-benzyl-2'-deoxy-5-fluorouridine

The general procedure of Example 73 was followed using 2-(2-methylbenzoyloxy)-4-hydroxypyridine in place of 4-benzoyloxy-5-chloro-2(1H)-pyridone, thereby producing the title compound in a yield of 31 %.
¹H-NMR(CDCl₃) δ:
8.66-7.20 (17H, m, phenyl-H, C₆-H and C₃·₅·₆-H of the pyridine ring)
6.21 (1H, t, J = 6Hz, C_{1'}-H)
4.51 (2H, s, 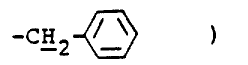 4.28-4.07 (4H, m, C_{3'}·_{4'}·_{5'}-H)
2.68-2.02 (8H, m, C_{2'}-H, 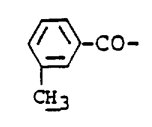 and COCH₃)

### Example 78

### Preparation of 5'-O-acetyl-3'-O-benzyl-3-[3-(5-chloro-2-ethoxycarbonyloxy-4-pyridyloxycarbonyl)benzoyl]-2'-deoxy-5-fluorouridine

The general procedure of Example 73 was followed using 5-chloro-2-ethoxycarbonyloxy-4-hydroxypyridine in place of 4-benzoyloxy-5-chloro-2(1H)-pyridone, thereby producing the title compound in a yield of 52 %.
¹H-NMR(CDCl₃) δ:
8.66-7.26 (12H, m, phenyl-H, C₆-H and C₃·₆-H of the pyridine ring)
6.22 (1H, t, J = 6Hz, C_{1'}-H)
4.53-4.08 (8H, m, 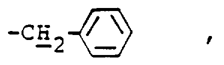 -O-CH₂CH₃ and C_{3'}·_{4'}·_{5'}-H)
2.75-2.11 (5H, m, C_{2'}-H and COCH₃)
1.40 (3H, t, J = 7Hz, -OCH₂CH₃)

### Example 79

### Preparation of 5'-O-acetyl-3-[3-(4-benzoyloxy-5-chloro-2-pyridyloxycarbonyl)benzoyl]-3'-O-(4-chlorobenzyl)-2'-deoxy-5-fluorouridine

The general procedure of Example 73 was followed using 5'-O-acetyl-3'-0-(4-chlorobenzyl)-2'-deoxy-5-fluorouridine in place of 5′-O-acetyl-3′-O-benzyl-2′-deoxy-5-fluorouridine, thereby producing the title compound in a yield of 50 %.
¹H-NMR (CDCl₃)δ:
8.65-7.22 (16H, m, phenyl-H, C₆-H and C₃·₆-H of the pyridine ring)
6.18 (1H, t, J = 6Hz, C_{1′}-H)
4.44 (2H, s, 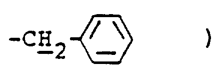 4.27-4.11 (4H, m, C_{3'}·_{4'}·_{5'}-H)
2.70-2.00 (5H, m, COCH₃ and C_{2′}-H)

### Example 80

### Preparation of 5′-O-acetyl-3-[3-(2-acetoxy-5-chloro-4-pyridyloxycarbonyl)benzoyl]-3′-O-(4-chlorobenzyl)-2′-deoxy-5-fluorouridine

The general procedure of Example 73 was followed using 5′-O-acetyl-3′-O-(4-chlorobenzyl)-2′-deoxy-5-fluorouridine in place of 5′-O-acetyl-3′-O-benzyl-2′-deoxy-5-fluorouridine and using 2-acetoxy-5-chloro-4-hydroxypyridine in place of 4-benzoyloxy-5-chloro-2(1H)-pyridone, thereby producing the title compound in a yield of 43 %.
¹H-NMR(CDCl₃)δ:
8.67-7.24 (11H, m, phenyl-H, C₆-H and C₃·₆-H of the pyridine ring)
6.20 (1H, t, J = 6Hz, C_{1'}-H)
4.47 (2H, s, 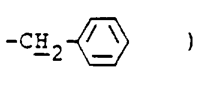 4.30-4.08 (4H, m, C_{3'}·_{4'}·_{5'}-H)
2.62-2.11 (8H, m, C_{2'}-H and COCH₃ x 2)

### Example 81

### Preparation of 5'-O-acetyl-3-[3-(4-acetoxy-5-chloro-2-pyridyloxycarbonyl)benzoyl]-3'-O-(4-chlorobenzyl)-2'-deoxy-5-fluorouridine

The general procedure of Example 73 was followed using 5'-O-acetyl-3'-O-(4-chlorobenzyl)-2'-deoxy-5-fluorouridine in place of 5'-O-acetyl-3'-O-benzyl-2'-deoxy-5-fluorouridine and using 4-acetoxy-5-chloro-2(1H)-pyridone in place of 4-benzoyloxy-5-chloro-2(1H)-pyridone, thereby producing the title compound in a yield of 52 %.
¹H-NMR(CDCl₃) δ:
8.65-7.18 (11H, m, phenyl-H, C₆-H and C₃·₆-H of the pyridine ring)
6.18 (1H, t, J = 6Hz, C_{1'}-H)
4.46 (2H, s, 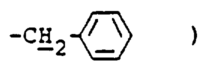 4.28-4.07 (4H, m, C_{3'}·_{4'}·_{5'}-H)
2.54-2.09 (8H, m, C_{2'}-H and COCH₃ x2)

### Example 82

### Preparation of 5'-O-acetyl-3-[3-(2-benzoyloxy-5-chloro-4-pyridyloxycarbonyl)benzoyl]-3'-O-(4-chlorobenzyl)-2'-deoxy-5-fluorouridine

The general procedure of Example 73 was followed using 5'-O-acetyl-3'-O-(4-chlorobenzyl)-2'-deoxy-5-fluorouridine in place of 5'-O-acetyl-3'-O-benzyl-2'-deoxy-5-fluorouridine and using 2-benzoyloxy-5-chloro-4-hydroxypyridine in place of 4-benzoyloxy-5-chloro-2(1H)-pyridone, thereby producing the title compound in a yield of 53 %.
¹H-NMR(CDCL₃) δ:
8.68-7.26 (16H, m, phenyl-H, C₆-H and C₃·₆-H of the pyridine ring)
6.20 (1H, t, J = 6Hz, C_{1'}-H)
4.46 (2H, s, 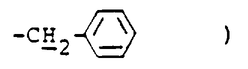 4.30-4.08 (4H, m, C_{3'}·_{4'}·_{5'}-H)
2.73-2.05 (5H, m, C_{2'}-H and COCH₃)

### Example 83

### Preparation of 5'-O-acetyl-3-[5-(2-acetoxy-5-chloro-4-pyridyloxycarbonyl)-3-pyridylcarbonyl]-3'-O-benzyl-2'-deoxy-5-fluorouridine

To a solution of 1.00 g of 5′-O-acetyl-3′-O-benzyl-2′-deoxy-5-fluorouridine in 50 ml of dry dioxane were added 3.26 ml of triethylamine and 0.80 g of pyridine-3,5-dicarbonyl chloride, and the mixture was refluxed for 1 hour. The precipitate thus formed was filtered off, and the filtrate was concentrated under reduced pressure. The concentrate was dissolved in 50 ml of acetonitrile. To the solution were added 1.83 ml of triethylamine and 0.59 g of 2-acetoxy-5-chloro-4-hydroxypyridine, and the resulting mixture was stirred at room temperature for 2.5 hours. The solvent was evaporated off under reduced pressure and the residue was placed on a silica gel column and eluted with chloroform, giving 0.22 g of the title compound in a yield of 12 %.
¹H-NMR(CDCl₃)δ:
9.57-8.88 (3H, m, 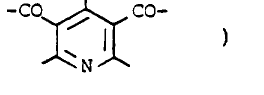 8.47 (1H, s, 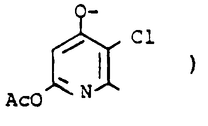 7.83 (1H, d, J = 6Hz, C₆-H)
7.30 (5H, s, phenyl-H)
7.27 (1H, s, 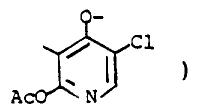 6.19 (1H, t, J = 6Hz, C_{1′} -H)
4.51 (2H, s, 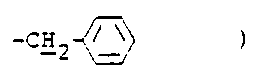 4.28-4.12 (4H, m, C_{3'}·_{4'}·_{5'}-H)
2.38-2.09 (8H, m, C_{2′}-H and COCH₃ x 2)

### Example 84

### Preparation of 5′-O-acetyl-3′-O-benzyl-3-[4-(5-chloro-4-hydroxy-2-pyridyloxycarbonyl)benzoyl]-2′-deoxy-5-fluorouridine

To a solution of 1.00 g of 5′-O-acetyl-3′-O-benzyl-2′-deoxy-5-fluorouridine in 50 ml of dry dioxane were added 2.88 ml of triethylamine and 0.80 g of terephthaloyl chloride, and the mixture was refluxed for 1.5 hours. The solvent was distilled off, and the residue was dissolved in 50 ml of acetonitrile. To the solution were added 1.53 g of 2,4-bis(trimethylsilyloxy)-5-chloropyridine, and the mixture was stirred overnight at room temperature. The solvent was distilled off, and the residue was placed on a silica gel column and eluted with 0.5 % ethanol-chloroform, giving 0.18 g of the title compound in a yield of 10 %.
¹H-NMR(CDCl₃)δ:
8.25 (1H, s, C₆-H of the pyridine ring)
8.17-7.93 (4H, m, 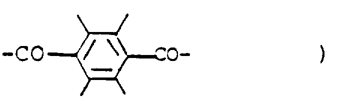 7.80 (1H, d, J = 6Hz, C₆-H)
7.28 (5H, s, 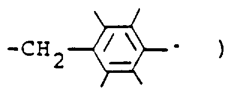 6.79 (1H, s, C₃-H of the pyridine ring)
6.20 (1H, t, J = 6Hz, C_{1′} -H)
4.51 (2H, s, 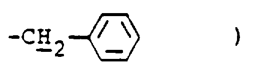 4.44-4.09 (4H, m, C_{3'}·_{4'}·_{5'}-H)
2.75-2.10 (5H, m, C_{2′}-H and COCH₃)

### Example 85

### Preparation of 3-[3-(4-acetoxy-5-chloro-2-pyridyloxycarbonyl)benzoyl]-3′-O-benzyl-5-fluoro-5′-O-nicotinoyluridine

The general procedure of Example 73 was followed using 3′-O-benzyl-2′-deoxy-5-fluoro-5′-O-nicotinoyluridine in place of 5′-O-acetyl-3′-O-benzyl-2′-deoxy-5-fluorouridine and using 4-acetoxy-5-chloro-2(1H)-pyridone in place of 4-benzoyloxy-5-chloro-2(1H)-pyridone, thereby producing the title compound in a yield of 41 %.
¹H-NMR(CDCl₃)δ:
9.21-7.29 (15H, m, phenyl-H, 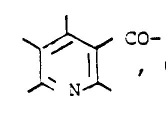 C₆-H and 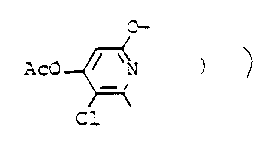 7.18 (1H, s, 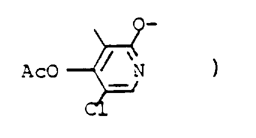 6.18 (1H, t, J = 6Hz, C_{1'}-H)
4.60-4.19 (6H, m, 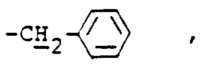 C_{3'}·_{4'}·_{5'}-H)
2.80-2.03 (5H, m, C_{2′}-H and COCH₃)

### Example 86

### Preparation of 3-[3-(4-acetoxy-5-chloro-2-pyridyloxycarbonyl)benzoyl]-3′-O-benzyl-2′-deoxy-5-fluoro-5′-O-(3,4-methylenedioxybenzoyl)uridine

The general procedure of Example 73 was followed using 3′-O-benzyl-2′-deoxy-5-fluoro-5′-O-(3,4-methylenedioxybenzoyl)uridine in place of 5′-O-acetyl-3′-O-benzyl-2′-deoxy-5-fluorouridine and using 4-acetoxy-5-chloro-2(1H)-pyridone in place of 4-benzoyloxy-5-chloro-2(1H)-pyridone, thereby producing the title compound in a yield of 42 %.
¹H-NMR(CDCl₃)δ:
8.65-8.17 and 7.79-7.52 (7H, m, 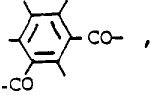 C₆-H, 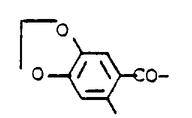 and C₆-H of the pyridine ring)
7.39 (1H, d, J = 2Hz, 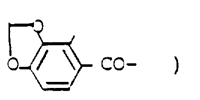 7.30 (5H, s, 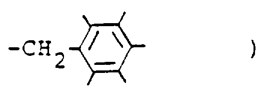 7.25 (1H, s, C₃-H of the pyridine ring)
6.84 (1H, d, J = 8Hz, 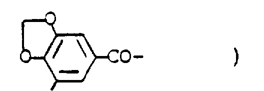 6.20 (1H, t, J = 6Hz, C_{1′}-H)
6.03 (2H, s, -OCH₂O-)
4.55-4.12 (6H, m, C_{3'}·_{4'}·_{5'}-H and 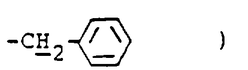 2.70-2.51 and 2.25-2.02 (2H, m, C_{2'}-H)
2.38 (3H, s, COCH₃)

### Example 87

### Preparation of 3-[3-(4-acetoxy-5-chloro-2-pyridyloxycarbonyl)benzoyl]-3′-O-benzyl-5′-O-(4-chlorobenzoyl)-2′-deoxy-5-fluorouridine

The general procedure of Example 73 was followed using 3′-O-benzyl-5′-O-(4-chlorobenzoyl)-2′-deoxy-5-fluorouridine in place of 5′-O-acetyl-3′-O-benzyl-2′-deoxy-5-fluorouridine and using 4-acetoxy-5-chloro-2(1H)-pyridone in place of 4-benzoyloxy-5-chloro-2(1H)-pyridone, thereby producing the title compound in a yield of 61 %.
¹H-NMR(CDCl₃) δ:
8.64-7.17 (16H, m, phenyl-H, C₆-H and C₃·₆-H of the pyridine ring)
6.19 (1H, t, J = 6Hz, C_{1'}-H)
4.55-4.18 (6H, m, C_{3'}·_{4'}·₅'-H and 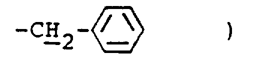 2.62-2.13 (5H, m, C_{2'}-H and -COCH₃)

### Example 88 (for comparison)

### Preparation of 3'-O-benzyl-2'-deoxy-5-fluoro-5'-O-(3,4-methylenedioxybenzoyl)uridine

The general procedure of Example 26 was followed, thereby producing the title compound in a yield of 72 %.
M.p. 169-171°C
¹H-NMR(CDCl₃) δ:
9.72 (1H, bs, -NH-, disappeared by addition of D₂O)
7.71-7.52 (2H, m, C₆-H and 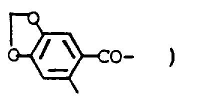 7.38 (1H, d, J = 2Hz, 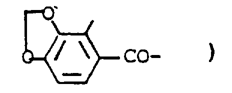 7.32 (5H, s, 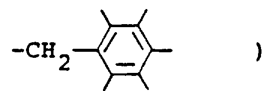 6.83 (1H, d, J = 8Hz, 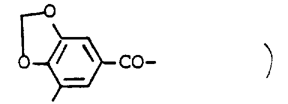 6.23 (1H, t, J = 6Hz, C_{1'}-H)
6.03 (2H, s, -OCH₂O-)
4.57-4.14 (6H, m, 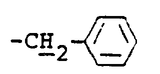 and C_{3'}·_{4'}·_{5'}-H)
2.75-2.49 and 2.19-1.89 (2H, m, C_{2'}-H)

### Example 89 (for comparison)

### Preparation of 3'-O-benzyl-5'-O-(4-chlorobenzoyl)-2'-deoxy-5-fluorouridine

The general procedure of Example 26 was followed, thereby producing the title compound in a yield of 53 %.
¹H-NMR(DMSO-d₆) δ:
11.9 (1H, bs, -NH-, disappeared by addition of D₂O)
7.95 (2H, d, J = 9Hz, 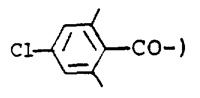 7.92 (1H, d, J = 6Hz, C₆-H)
7.59 (2H, d, J = 9Hz, 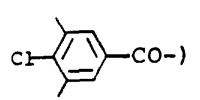 7.33 (5H, s, 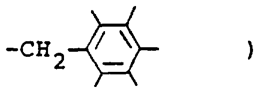 6.15 (1H, t, J = 6Hz, C_{1'}-H)
4.58-4.31 (6H, m, C_{3'}·_{4'}·_{5'}-H and 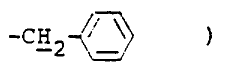 2.42-2.31 (2H, m, C_{2'}-H)

### Example 90 (for comparison)

### Preparation of 3'-O-(3-chlorobenzyl)-2'-deoxy-5-fluorouridine

A 2.00 g quantity of potassium hydroxide was dissolved in a mixture of 75 ml of water and 40 ml of dioxane. To the solution were added 1.00 g of 2'-deoxy-5-fluorouridine and 2.50 g of 3-chlorobenzyl chloride, and the resulting mixture was stirred at 45°C for 3 days. After the reaction, the same subsequent procedure as in Examples 4 and 5 was carried out, and the residue was placed on a silica gel column to conduct a gradient elution with chloroform and mixtures of methanol (up to 2 %) and chloroform, thereby producing 0.21 g of the title compound in a yield of 14 %.
M.p. 153-155°C.

### Example 91 (for comparison)

### Preparation of 3'-O-(2-chlorobenzyl)-2'-deoxy-5-fluorouridine

A 3.75 g quantity of potassium hydroxide was dissolved in a mixture of 150 ml of water and 40 ml of dioxane. To the solution were added 1.00 g of 2'-deoxy-5-fluorouridine and 10 ml of 2-chlorobenzyl chloride, and the resulting mixture was stirred at 30°C for 3 days. After the reaction, the same subsequent procedure as in Examples 4 and 5 was carried out, and the residue was placed on a silica gel column and eluted with 2 % methanol-chloroform, thereby producing 0.34 g of the title compound in a yield of 23 %.
M.p. 78-80°C.

### Example 92 (for comparison)

### Preparation of 2'-deoxy-5-fluoro-3'-O-(4-fluorobenzyl)-uridine

A 7.5 g quantity of potassium hydroxide was dissolved in a mixture of 300 ml of water and 80 ml of dioxane. To the solution were added 2.00 g of 2'-deoxy-5-fluorouridine and 4.9 ml of 4-fluorobenzyl chloride, and the resulting mixture was stirred at 35°C for 2 days. After the reaction, the same subsequent procedure as in Examples 4 and 5 was carried out, and the residue was placed on a silica gel column and eluted with 2 % methanol-chloroform, thereby producing 0.57 g of the title compound in a yield of 20 %.
M.p. 130-131°C.

### Example 93 (for comparison)

### Preparation of 2'-deoxy-5-fluoro-3'-O-(1-naphthylmethyl)-uridine

The general procedures of Examples 4 and 5 were followed using 1.3 g of potassium hydroxide, 1.00 g of 2'-deoxy-5-fluorouridine and 2.7 g of 1-naphthylmethyl bromide, thereby producing 0.28 g of the title compound in a yield of 18 %.
M.p. 159-160°C.

### Example 94 (for comparison)

### Preparation of 5'-O-acetyl-3-O-benzoyl-3'-O-(4-chlorobenzyl)-2'-deoxy-5-fluorouridine

To a solution of 0.25 g of 3'-0-(4-chlorobenzyl)-5'-O-acetyl-2'-deoxy-5-fluorouridine in 30 ml of dioxane were added 0.26 g of benzoyl chloride and 0.51 ml of triethylamine, and the mixture was stirred at 90°C for 2 hours. The reaction mixture was concentrated and the residue was dissolved in 50 ml of ethyl acetate. The solution was washed three times with a saturated aqueous solution of sodium hydrogen carbonate and dried over anhydrous sodium sulfate. The ethyl acetate was distilled off, and the residue was placed on a silica gel column and eluted with chloroform, thereby producing 0.29 g of the title compound in a yield of 92 %.
NMR(CDCl₃) δ:
7.98-7.87 (2H, m, 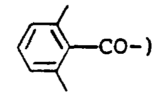 7.77 (1H, d, J = 6Hz, H₆)
7.69-7.32 (3H, m, 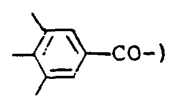 7.27 (4H, d, J = 3Hz, phenyl-H)
6.21 (1H, t, J = 4Hz, H_{1'})
4.90 (2H, d, J = 1Hz, 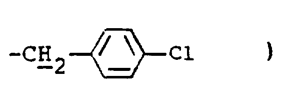 4.32-4.03 (4H, m, H_{3'},H_{4'},H_{5'})
2.72-1.96 (5H, m, H₂ and CH₃CO-)

### Example 95 (for comparison)

### Preparation of 5'-O-acetyl-3-(4-chlorobenzoyl)-3'-O-(4-chlorobenzyl)-2'-deoxy-5-fluorouridine

To a solution of 0.30 g of 3'-0-(4-chlorobenzyl)-5'-O-acetyl-2'-deoxy-5-fluorouridine in 30 ml of dioxane were added 0.38 g of 4-chlorobenzoyl chloride and 0.61 ml of triethylamine, and the mixture was stirred at 40°C for 3 hours. The insolubles were removed by filtration, and the residue was dissolved in 50 ml of ethyl acetate. The solution was washed with water and dried. The ethyl acetate was distilled off, and the residue was placed on a silica gel column and eluted with chloroform-n-hexane (3:2), thereby producing 0.33 g of the title compound in a yield of 82 %.
NMR(CDCl₃) δ:
7.86 (2H, d, J = 9Hz, 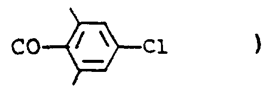 7.77 (1H, d, J = 7Hz, H₆)
7.48 (2H, d, J = 9Hz, 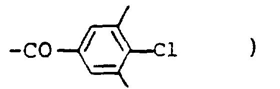 7.27 (4H, d, J = 4Hz, 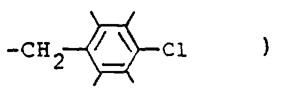 6.20 (1H, t, J = 6Hz, H_{1'})
4.49 (2H, s, 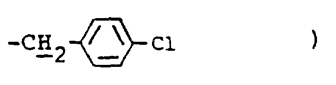 4.32-4.03 (4H, m, H_{3'}·H_{4'}·H_{5'})
2.75-1.95 (5H, m, H_{2'} and CH₃CO-)

### Example 96 (for comparison)

### Preparation of 5'-O-acetyl-3-(4-n-propoxybenzoyl)-3'-O-(4-chlorobenzyl)-2'-deoxy-5-fluorouridine

To a solution of 0.30 g of 3'-O-(4-chlorobenzyl)-5'-O-acetyl-2'-deoxy-5-fluorouridine in 30 ml of dioxane were added 0.44 g of 4-n-propoxybenzoyl chloride and 0.61 ml of triethylamine, and the mixture was stirred at 70°C for 3 hours. The insolubles were removed by filtration, and the residue was dissolved in ethyl acetate. The solution was washed with water and dried. The ethyl acetate was distilled off, and the residue was placed on a silica gel column and eluted with chloroform-petroleum ether (1:1), thereby producing 0.15 g of the title compound in a yield of 36 %.
NMR(CDCl₃) δ:
7.87 (2H, d, J = 9Hz, 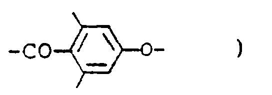 7,74 (1H, d, J = 6Hz, H₆)
7.27 (4H, d, J = 3Hz, 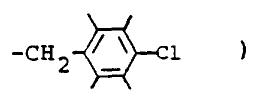 6.21 (1H, t, J = 4Hz, H_{1'})
4.49 (2H, s, 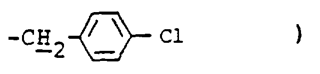 4.31-3.93 (6H, m, H_{3'}·H_{4'}·H_{5'} and -O-CH₂CH₂CH₃)
2.72-1.65 (7H, m, H₂, CH₃CO- and -O-CH₂CH₂CH₃)
1.04 (3H, t, J = 7Hz, -OCH₂CH₂CH₃)

### Example 97 (for comparison)

### Preparation of 3-benzoyl-3'-O-(4-chlcrobenzyl)-2'-deoxy-5-fluorouridine

The general procedure of Example 41 was followed using 0.50 g of 3'-O-(4-chlorobenzyl)-2'-deoxy-5-fluorouridine, thereby producing 0.46 g of the title compound as a powder in a yield of 72 %.
M.p.-(powder)
NMR(CDCl₃) δ:
8.19 (1H, d, J = 6Hz, H₆)
7.90 (2H, bd, J = 7Hz, 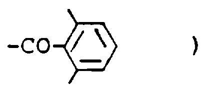 7.65-7.35 (3H, m, 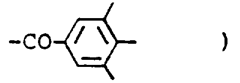 7.24 (4H, s, 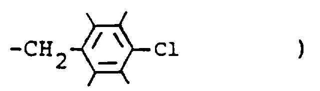 6.24 (1H, t, J = 6Hz, H_{1'})
4.42 (2H, s, 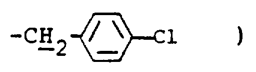 4.24-4.15 (2H, m, H_{3'}, H_{4'})
3.95-3.60 (2H, m, H_{5'})
2.59-1.98 (2H, m, H_{2'})

### Example 98 (for comparison)

### Preparation of 3-benzoyl-5'-O-benzoyl-3'-O-(4-chlorobenzyl)-2'-deoxy-5-fluorouridine

A 0.23 ml quantity of benzoyl chloride was added to a solution of 0.25 g of 3'-O-(4-chlorobenzyl)-2'-deoxy-5-fluorouridine in pyridine. The mixture was stirred at room temperature for 2 hours. The solvent was distilled off. To the residue were added ethyl acetate and water to separate the ethyl acetate layer. The ethyl acetate layer was dried over anhydrous sodium sulfate and concentrated. The concentrate was subjected to silica gel column chromatography using chloroform as an eluent, giving 0.27 g of the title compound in a yield of 70 %.
NMR(CDCl₃) δ:
8.05-7.85 (4H, m, 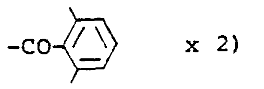 7.71 (1H, d, J = 6Hz, H₆)
7.63-7.32 (6H, m, 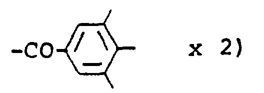 7.25 (4H, s, 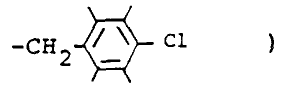 6.21 (1H, t, J = 4Hz, H_{1'})
4.63-4.20 (6H, m, H_{3'}, H_{4'}, H_{5'} and 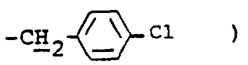 2.77-2.02 (2H, m, H_{2'})

### Example 99 (for comparison)

### Preparation of 3'-O-benzyl-2'-deoxy-5-fluoro-3-nicotinoyl-5'-O-nicotinoyluridine

A 0.40 g quantity of nicotinoyl chloride hydrochloride and 1.0 ml of triethylamine were added to a solution of 0.50 g of 3'-O-benzyl-2'-deoxy-5-fluorouridine in 40 ml of dioxane. The mixture was refluxed for 6 hours. The solvent was distilled off and the residue was subjected to silica gel column chromatography using 1 % methanol-chloroform as an eluent, giving 0.23 g of the title compound in a yield of 29 %.
NMR(DMSO-d₆) δ:
9.27 and 9.11 (each 1H, d, 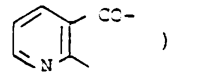 8.97-8.81 (2H, m, 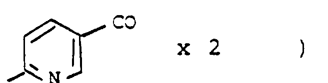 8.57-8.16 (3H, m, H₆ and 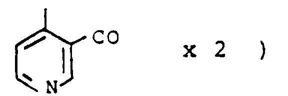 7.73-7.50 (2H, m, 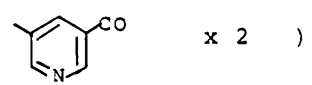 7.32 (5H, s, 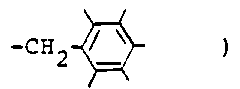 6.10 (1H, m, J = 6Hz, H_{1'})
4.59 (2H, s, 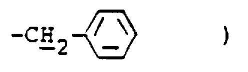 4.54-4.33 (4H, m, H_{3'}, H_{4'}, H_{5'})
2.37-2.20 (2H, m, H_{2'})

### Example 100

### Preparation of 3-[3-(4-acetoxy-5-chloro-2-pyridyloxycarbonyl)benzoyl]-3'-O-benzyl-5-fluoro-2'-deoxy-5'-O-(1-naphthoyl)uridine

The general procedure of Example 73 was followed using 0.50 g of 3'-O-benzyl-2'-deoxy-5-fluoro-5'-O-(1-naphthoyl)uridine, thereby producing 0.05 g of the title compound as a powder - a yield of 6 %.
NMR(CDCl₃)δ:
8.89-7.36 (13H, m, 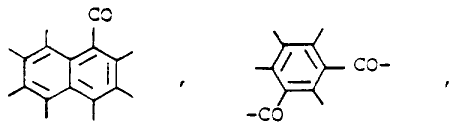 H₆ and H₆ of the pyridine ring)
7.30 (5H, s, 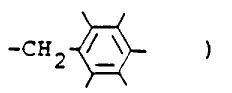 7.16 (1H, s, H₃ of the pyridine ring)
6.24 (1H, t, J = 6Hz, H_{1′} )
4.70-4.24 (6H, m, H_{3′}, H_{4′}, H_{5′} and 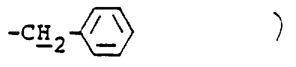 2.75-2.03 (5H, m, H_{2′} and CH₃CO-)

### Example 101

### Preparation of 3-[3-(4-acetoxy-5-chloro-2-pyridyloxycarbonyl)benzoyl]-3′-O-benzyl-2′-deoxy-5-fluorouridine

Trimethylchlorosilane (1.50 ml) and 4.20 ml of triethylamine were added to a solution of 1.00 g of 3′-O-benzyl-2′-deoxy-5-fluorouridine in 30 ml of dried dioxane. The mixture was stirred at room temperature for 20 minutes. The precipitate obtained was separated by filtration and the filtrate was concentrated. The concentrate was dissolved in 30 ml of dried dioxane. To the solution were added 724 mg of isophthaloyl chloride and 2.47 ml of triethylamine and the mixture was stirred at 100°C for 45 minutes.

The reaction mixture was cooled to room temperature and 0.84 g of 4-acetyloxy-5-chloro-2-pyridone was added to the reaction mixture. The resulting mixture was stirred at room temperature for 2 hours. The precipitate obtained was removed by filtration, the filtrate was concentrated, the concentrate was dissolved in 50 ml of ethyl acetate, and the solution was washed with 30 ml of a saturated aqueous solution of sodium chloride three times (3 x 30 ml). The ethyl acetate layer was dried over anhydrous sodium sulfate and concentrated. To the concentrate were added 50 ml of methanol and 1.0 ml of acetic acid. The mixture was stirred at room temperature for 16 hours. The solvent was distilled off and the residue was subjected to silica gel column chromatography using as an eluent 1 % acetone-chloroform, giving 0.74 g of the title compound in a yield of 38 %.
NMR(CDCl₃)δ:
8.66-8.18 (3H, m, 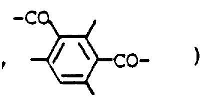 8.45 (1H, s, H₆ of the pyridine ring)
8.11 (1H, d, H₆, J = 7Hz)
7.68 (1H, t, 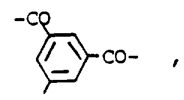 J = 8Hz)
7.31 (5H, s, 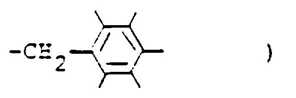 7.18 (1H, s, H₃ of the pyridine ring)
6.24 (1H, t, H_{1′} , J = 4Hz)
4.52 (1H, d, 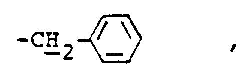 J = 1Hz)
4.29-4.11 (2H, m, H_{3′}, H_{4′})
4.04-3.67 (2H, m, H_{5′})
2.57-2.04 (2H, m, H_{2′})
2.39 (3H, s, CH₃CO-)

### Example 102

### Preparation of 3′-O-benzyl-3-[3-(5-chloro-1,2-dihydro-2-oxo-pyridyloxycarbonyl)benzoyl]-2′-deoxy-5-fluorouridine

Silylation and introduction of isophthaloyl group were conducted using 0.50 g of 3′-O-benzyl-2′-deoxy-5-fluorouridine in the same manner as in Example 101. The insolubles were removed by filtration. The solvent was distilled off and the residue was dissolved in 100 ml of pyridine. To the solution was added 0.35 g of 5-chloro-4-hydroxy-2-pyridone. The mixture was left to stand at room temperature overnight. The pyridine was distilled off and the residue was subjected to silica gel column chromatography to conduct gradient elution using
chloroform and mixtures of methanol (up to 4 %) and chloroform, giving 0.23 g of the title compound in a yield of 25 %.
NMR(DMSO-d₆)δ:
12.04 (1H, bs, -NH-)
8.62-8.26 (4H, m, H₆ and 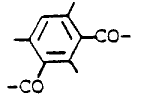 7.92-7.74 (2H, m, 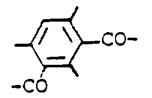 and H₆ of the pyridine ring)
7.33 (5H, s, 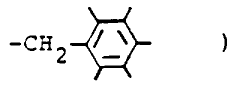 6.63 (1H, s, H₃ of the pyridine ring)
6.11 (1H, t, J = 6Hz, H_{1′} )
5.29 (1H, t, J = 5Hz, 5′-OH)
4.54 (2H, s, 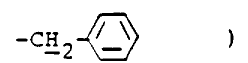 4.35-4.08 (2H, m, H_{3′}, H_{4′})
3.70-3.61 (2H, m, H_{5′})
2.51-2.08 (2H, m, H_{2′})

### Example 103

### Preparation of 3-[3-(4-benzoyloxy-5-chloro-2-pyridyloxycarbonyl)benzoyl]-3′-O-benzyl-2′-deoxy-5-fluorouridine

The general procedure of Example 101 was followed using 0.50 g of 3′-O-benzyl-2′-deoxy-5-fluorouridine, thereby producing 0.29 g of the title compound as a powder in a yield of 27 %.
NMR(CDCl₃)δ:
8.68-8.13 (7H, m, 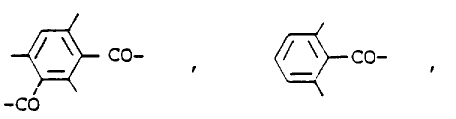 H₆ and H₆ of the pyridine ring)
7.81-7.42 (4H, m, 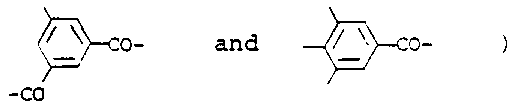 7.39 (1H, s, H₃ of the pyridine ring)
7.30 (5H, s 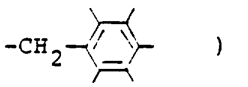 6.21 (1H, t, J = 4Hz, H_{1′})
4.55 (2H, d, J = 1Hz, 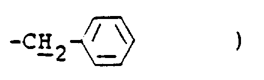 4.46-4.12 (2H, m, H_{3′}, H_{4′})
4.03-3.66 (2H, m, H_{5′})
2.67-2.07 (2H, m, H_{2′})

### Example 104

### Preparation of 3′-O-benzyl-3-[3-(5-chloro-4-hydroxy-2-pyridyloxycarbonyl)benzoyl]-2′-deoxy-5-fluorouridine

Silylation and introduction of isophthaloyl group were conducted using 0.50 g of 3′-O-benzyl-2′-deoxy-5-fluorouridine in the same manner as in Example 101. The insolubles were removed by filtration and the solvent was distilled off. The residue was dissolved in 50 ml of dichloromethane. To the solution were added 0.57 g of 2,4-bis (trimethylsilyloxy)-5-chloropyridine and 0.10 ml of stannic chloride. The mixture was stirred at room temperature for 6 hours. The reaction mixture was neutralized with triethylamine and concentrated. The concentrate was extracted with ethyl acetate, and the extract was washed with water, dried and concentrated. The concentrate was suspended in 40 ml of methanol and the suspension was stirred at room temperature overnight. The solvent was distilled off and the residue was subjected to silica gel column chromatography using as an eluent 1 % methanol-chloroform, giving 0.19 g of the title compound as a powder in a yield of 21 %.
NMR(CDCl₃)δ:
8.58-8.16 (4H, m, H₆ of the pyridine ring, 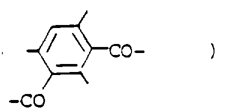 8.05 (1H, d, J = 6Hz, H₆)
7.62 (1H, t, J = 8Hz, 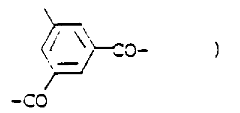 7.29 (5H, s, phenyl-H)
6.78 (1H, s, H₃ of the pyridine ring)
6.19 (1H, t, J = 6Hz, H_{1′})
4.49 (2H, s, 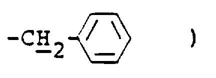 4.32-3.78 (4H, m, H_{3′}, H_{4′}, H_{5′} )
2.49-2.09 (2H, m, H_{2′})

### Example 105

### Preparation of 3-[3-(4-benzoyloxy-5-chloro-2-pyridyloxycarbonyl)benzoyl]-3′-O-(4-chlorobenzyl)-2′-deoxy-5-fluorouridine

The same reaction as in Example 101 was repeated using 3′-O-(4-chlorobenzyl)-2′-deoxy-5-fluorouridine and 4-benzoyloxy-5-chloro-2-pyridone.

A reaction for removing the silyl group was conducted by dissolving the residue of the reaction mixture in 5 ml of chloroform, adding 10 ml of methanol to the solution and leaving the mixture to stand at room temperature for 16 hours. The solvent was distilled off and the residue was subjected to silica gel column chromatography using as an eluent 1 % acetone-chloroform, giving 0.24 g of the title compound as a powder in a yield of 24 %.
NMR(CDCl₃)δ:
8.67-8.17 (6H, m, 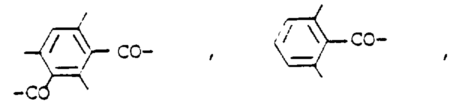 and H₆ of the pyridine ring)
8.11 (1H, d, H₆, J = 6Hz)
7.78-7.43 (4H, m, 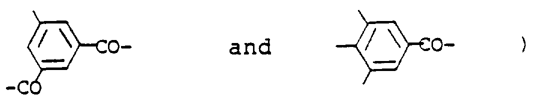 7.39 (1H, s, H₃ of the pyridine ring)
7.26 (4H, d, 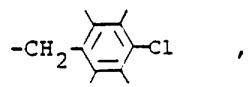 J = 2Hz)
6.24 (1H, t, J = 4Hz, H_{1′} )
4.48 (2H, s, 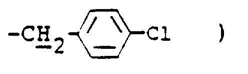 4.36-4.15 (2H, m, H_{3′}, H_{5′})
4.10-3.73 (2H, m, H_{5′})
2.70-2.10 (2H, m, H_{2′})

### Example 106

### Preparation of 3′-O-benzyl-3-[3-(5-chloro-4-dimethylaminobenzoyloxy-2-pyridyloxycarbonyl)benzoyl]-2′-deoxy-5-fluorouridine

The general procedure of Example 101 was followed using 1.00 g of 3'-O-benzyl-2'-deoxy-5-fluorouridine, thereby producing 0.48 g of the title compound as a powder in a yield of 21 %.
NMR(CDCl₃) δ:
8.69-8.46 (4H, m, H₆ of the pyridine ring, 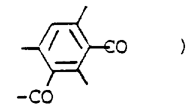 8.00-7.76 (4H, m, H₆, 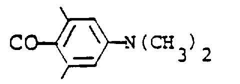 and 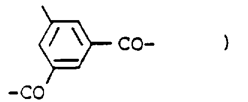 7.73 (1H, s, H₃ of the pyridine ring)
7.32 (5H, s, 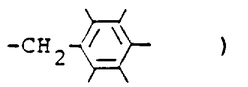 6.82 (2H, d, J = 9Hz, 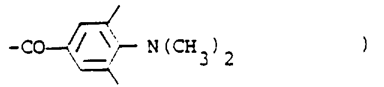 6.12 (1H, t, J = 7Hz, H_{1'})
5.30 (1H, t, J = 5Hz, 5'-OH)
4.53 (2H, s, 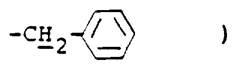 4.42-3.63 (4H, m, H_{3'}, H_{4'}, H_{5'})
3.07 (6H, s, -N(CH₃)₂)
2.56-2.20 (2H, m, H_{2'})

### Example 107

### Preparation of 3-[3-(4-acetoxy-2-pyridyloxycarbonyl)benzoyl]-5′-O-acetyl-3′-O-benzyl-2′-deoxy-5-fluorouridine

The general procedure of Example 73 was followed, thereby producing the title compound in a yield of 46 %.
NMR(CDCl₃)δ:
8.66-8.11 (4H, m, H₆ of the pyridine ring, 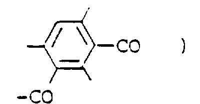 7.80 (1H, d, J = 6Hz, H₆)
7.68 (1H, t, J = 8Hz, 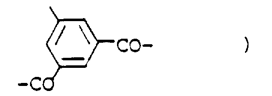 7.31 (5H, s, phenyl-H)
7.22-7.09 (2H, m, H₃ and H₅ of the pyridine ring)
6.20 (1H, t, J = 6Hz, H_{1′})
4.52 and 4.50 (each 1H, s, 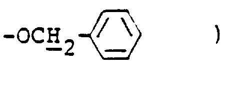 4.44-4.08 (4H, m, H_{3′}, H_{4′}, H_{5′} )
2.69-2.02 (8H, m, H₂′ and CH₃CO- x 2)

### Example 108

### Preparation of 5′-O-acetyl-3′-O-benzyl-3-[3-(5-chloro-1,2-dihydro-2-oxo-4-pyridyloxycarbonyl)benzoyl]-2′-deoxy-5-fluorouridine

Isophthaloyl chloride (0.40 g) and 1.0 ml of triethylamine were added to a solution of 0.50 g of 5′-O-acetyl-3′-O-benzyl-2′-deoxy-5-fluorouridine in 50 ml of dioxane. The mixture was refluxed for 1 hour. After the mixture was left to stand for cooling, the insolubles were removed by filtration and the filtrate was concentrated. The concentrate was dissolved in 100 ml of pyridine. To the solution was added 0.38 g of 5-chloro-4-hydroxy-2-pyridone. The mixture was stirred at room temperature overnight. The solvent was distilled off and the residue was subjected to silica gel column chromatography using as an eluent 2 % methanol-chloroform, giving 0.23 g of the title compound in a yield of 27 %.
NMR(DMSO-d₆)δ:
12.00 (1H, bs, -NH-)
8.67-7.88 (6H, m, H₆, 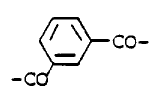 and H₆ of the pyridine ring)
7.33 (5H, s, 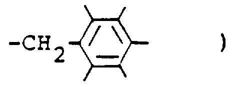 6.63 (1H, s, H₃ of the pyridine ring)
6.10 (1H, t, J = 6Hz, H_{1′} )
4.54 (2H, s, 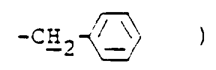 4.24 (4H, bs, H_{3′}, H_{4′}, H_{5′})
2.46-2.01 (5H, m, H_{2′} and CH₃CO-)

### Example 109

### Preparation of 5′-O-acetyl-3′-O-benzyl-3-[3-(5-chloro-4-n-hexanoyloxy-2-pyridyloxycarbonyl)benzoyl]-2′-deoxy-5-fluorouridine

The general procedure of Example 73 was followed, thereby producing the title compound as a powder in a yield of 75 %.
NMR(CDCl₃)δ:
8.63-8.06 (4H, m, 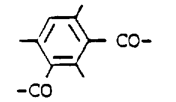 and H₆ of the pyridine ring)
7.83-7.60 (2H, m, 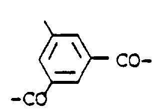 and H₆)
7.31 (5H, s, 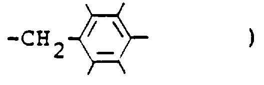 7.18 (1H, s, H₃ of the pyridine ring)
6.21 (1H, t, J = 6Hz, H_{1′} )
4.52 (2H, s, 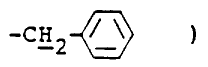 4.44-4.09 (4H, m, H_{3'}, H_{4'}, H_{5'})
2.65 (2H, t, J = 7Hz, -COCH₂CH₂-)
2.22-0.93 (14H, m, COCH₃-, H₂, and -COCH₂(CH₂)₃CH₃)

### Example 110

### Preparation of 5'-O-acetyl-3'-O-benzyl-3-[3-(5-chloro-4-octadecanoyloxy-2-pyridyloxycarbonyl)benzoyl]-2'-deoxy-5-fluorouridine

The general procedure of Example 73 was followed, thereby producing the title compound as a powder in a yield of 17 %.
NMR(CDCl₃) δ:
8.60-8.05 (4H, m, 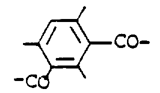
and H₆ of the pyridine ring)
7.82-7.57 (2H, m, 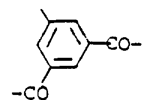 and H₆)
7.31 (5H, s, 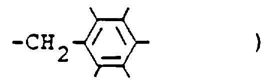 7.18 (1H, s, H₃ of the pyridine ring)
6.19 (1H, t, J = 6Hz, H_{1'})
4.52 (2H, s, 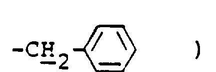 4.30-4.10 (4H, m, H_{3'}, H_{4'}, H_{5'})
2.67 (2H, t, J = 7Hz, -COCH₂CH₂-)
2.58-2.05 (5H, m, COCH₃- and H_{2'})
1.26 (3OH, bs, -COCH₂(CH₂)₁₅CH₃)
0.88 (3H, t, J = 7Hz, -CH₂CH₃)

### Example 111

### Preparation of 5'-O-acetyl-3'-O-benzyl-3-[3-[5-chloro-4-(2-furoyloxy)-2-pyridyloxycarbonyl]benzoyl]-2'-deoxy-5-fluorouridine

The general procedure of Example 73 was followed, thereby producing the title compound in a yield of 57 %.
NMR(CDCl₃) δ:
8.66-8.21 (5H, m, 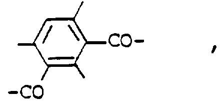 H₆ of the pyridine ring and H₅ of the furan ring)
7.84-7.69 (2H, m, 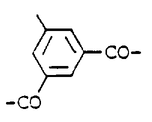 and H₆)
7.49 (1H, dd, J_{3,4} = 4Hz, J_{3,5} = 1Hz, H₃ of the furan ring)
7.36 (1H, s, H₃ of the pyridine ring)
7.31 (5H, s, 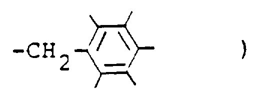 6.64 (1H, dd, J_{3,4} = 4Hz, J_{4,5} = 2Hz, H₄ of the furan ring)
6.21 (1H, t, J = 6Hz, H_{1'})
4.53 and 4.51 (each 1H, s, 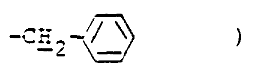 4.30-4.09 (4H, m, H₃', H_{4'}, H_{5'})
2.58-2.09 (5H, m, H₂, and CH₃CO-)

### Example 112

### Preparation of 5'-O-acetyl-3'-O-benzyl-3-[3-[5-chloro-4-(2-thenoyloxy)-2-pyridyloxycarbonyl]benzoyl]-2'-deoxy-5-fluorouridine

The general procedure of Example 73 was followed, thereby producing the title compound in a yield of 59 %.
NMR(CDCl₃) δ:
8.65-7.58 (8H, m, 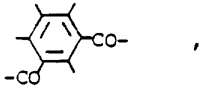 H₆ of the pyridine ring, H₃ of the thiophene ring, H₅ of the thiophene ring and H₆)
7.38 (1H, s, H₃ of the pyridine ring)
7.30-7.15 (6H, m, 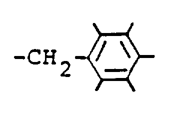 and H₄ of the thiophene ring)
6.21 (1H, t, J = 6Hz, H_{1'})
4.52 and 4.50 (each 1H, s, 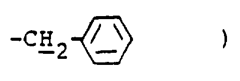 4.28-4.09 (4H, m, H_{3'}, H_{4'}, H_{5'})
2.72-2.05 (5H, m, H₂, and CH₃CO-)

### Example 113

### Preparation of 5'-O-acetyl-3'-O-benzyl-3-[3-[5-chloro-4-(3,4,5-trimethoxybenzoyloxy)-2-pyridyloxycarbonyl]-benzoyl]-2'-deoxy-5-fluorouridine

The general procedure of Example 73 was followed, thereby producing the title compound as a powder in a yield of 71 %.
NMR(CDCl₃) δ:
8.67-8.18 (4H, m, 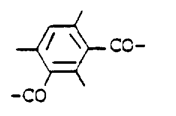 and H₆ of the pyridine ring)
7.84-7.60 (2H, m, 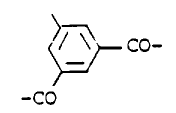 and H₆)
7.47 (2H, s, 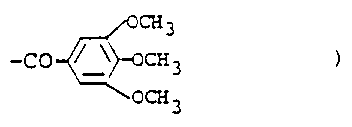 7.38 (1H, s, H₃ of the pyridine ring)
7.31 (5H, s, 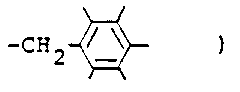 6.21 (lH, t, J = 6Hz, H_{1'})
4.53 and 4.50 (each 1H, s, 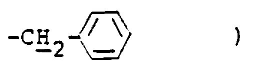 4.29-4.08 (4H, m, H_{3'}, H_{4'} H_{5'})
3.96 and 3.93 (9H, each s, -OCH₃ x 3)
2.50-2.05 (5H, m, H₂, and CH₃CO-)

### Example 114 (for comparison)

### Preparation of 2'-deoxy-5-fluoro-3'-O-(4-carboxybenzyl)-uridine [R = R³ = H, R¹ = 4-carboxybenzyl group]

Potassium hydroxide (4.30 g) and 1.70 g of 4-methoxycarbonylbenzyl bromide were added to a solution of 3.00 g of 2'-deoxy-5-fluoro-5'-O-trityluridine in 200 ml of dioxane. The mixture was stirred at room temperature for a day. The reaction mixture was concentrated under a reduced pressure. To the concentrate was added 200 ml of water and the mixture was stirred at room temperature for 15 minutes. The reaction mixture was rendered acidic with acetic acid, and extracted with ethyl acetate. The organic layer was dried over magnesium sulfate and concentrated under a reduced pressure. The concentrate was dissolved in 50 ml of a 80 % aqueous solution of acetic acid. The solution was heated to 100°C for 2 hours. The solvent was distilled off and the residue was applied to silica gel column chromatography using 3 % methanol-chloroform as an eluent, affording 650 mg of the title compound in a yield of 28 %.
¹H-NMR(DMSO-d₆) δ:
11.83 (1H, bs, N₃-H, disappeared by addition of D₂O)
8.19 (1H, d, J = 7Hz, C₆-H)
7.94 (2H, d, J = 8Hz, 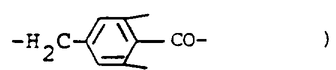 7.46 (2H, d, J = 8Hz, 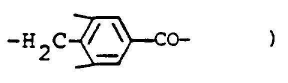 6.14 (1H, t, J = 6Hz, C_{1'}-H)
5.20 (1H, bs, C_{5'}-OH, disappeared by addition of D₂O)
4.63 (2H, s, 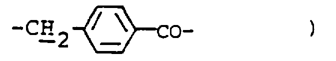
4.22-3.64 (4H, m, C_{3'}·_{4'}·_{5'}-H)
2.35-2.04 (2H, m, C_{2'}-H)

### Example 115 (for comparison)

### Preparation of 2'-deoxy-3'-O-benzyl-5-fluorouridine [R¹ = C₆H₅CH₂, R = R³ = H]

In 50 ml of dioxane was dissolved 10 g of 2'-deoxy-5'-O-trityl-5-fluorouridine. To the solution were added 2.9 ml of benzyl bromide and 14.6 g of particles of potassium hydroxide. The mixture was stirred at room temperature for 1 hour. Thereto was added 40 ml of water. The mixture was adjusted to a pH of about 3 to about 4 and extracted with ethyl acetate. The ethyl acetate layer was washed with an aqueous solution of sodium chloride and dried over anhydrous sodium sulfate. The sodium sulfate was separated by filtration and the solvent was distilled off, giving as an intermediate 16.0 g of 2'-deoxy-3'-O-benzyl-5'-O-trityl-5-fluorouridine.

The compound thus obtained was dissolved in 80 ml of a 80 % solution of acetic acid to undergo reaction at 50 to 60°C for 2 hours. The reaction mixture was cooled with ice for 1 hour and 5.1 g of trityl alcohol was removed by filtration. The mother liquor was concentrated and ethanol was added to the concentrate. The mixture was stirred and the crystals thus precipitated were separated by filtration and dried, giving 5.3 g of the title compound in a yield of 75.7 %.
M.p. 138-139°C

The analysis by ¹H-NMR showed that the compound obtained above was identical with the compound prepared in Example 2.

### Example 116 (for comparison)

### Preparation of 2'-deoxy-3'-O-benzyl-5-fluorouridine [R¹ = C₆H₅CH₂, R = R³ = H]

The general procedure of Example 115 was followed using 2'-deoxy-5'-O-(diphenyl-p-methoxyphenyl)-methyl-5-fluorouridine, thereby producing the title compound in a yield of 63 %. M.p. 138-139°C.

The analysis by ¹H-NMR showed that the compound obtained above was identical with the compound prepared in Example 115.

### Example 117 (for comparison)

### Preparation of 2'-deoxy-3'-O-benzyl-5-fluorouridine [R¹ = C₆H₅CH₂, R = R³ = H]

A 10 g quantity of 2'-deoxy-5'-O-trityl-5-fluorouridine was dissolved in 100 ml of dioxane. To the solution were added 2.9 ml of benzyl chloride, 6.9 g of particles of potassium hydroxide and 3.56 g of sodium iodide. The mixture was stirred at 40°C for 4 hours, and 2.9 ml of benzyl chloride and 1.15 g of potassium hydroxide were added and stirred for 1 hour. Thereto was added water to dissolve the potassium hydroxide in water. The solution was adjusted to a pH of about 3 with acetic acid and extracted with ethylene dichloride. The extract was washed with water and the ethylene dichloride layer was dried over anhydrous sodium sulfate. The sodium sulfate was separated by filtration and the solvent was distilled off, giving 8 g of 2'-deoxy-3'-O-benzyl-5'-O-trityl-5-fluorouridine as an oil.

The compound thus obtained was dissolved in 80 ml of a 80 % solution of acetic acid to undergo reaction at 50 to 60°C for 2 hours. The reaction mixture was cooled with ice for 1 hour. The trityl alcohol was separated by filtration and the filtrate was concentrated. The concentrate was recrystallized from ethanol, giving a first crop of crystals of the title compound in a yield of 70.6 %.

The ethanol mother liquor was concentrated and the concentrate was recrystallized from a small amount of ethanol, affording a second crop of crystals of the title compound in a yield of 15.9 %.
Overall yield 86.5 %
M.p. 138-139°C

The analysis by ¹H-NMR showed that the product was identical with the compound prepared in Example 115.

### Example 118 (for comparison)

### Preparation of 2'-deoxy-3'-O-benzyl-5-fluorouridine [R¹ = C₆H₅CH₂, R = R³ = H]

In 50 ml of dioxane was dissolved 10 g of 2'-deoxy-5'-O-trityl-5-fluorouridine. To the solution were added 2.9 ml of benzyl bromide and 14.6 g of particles of potassium hydroxide. The mixture was stirred at room temperature for 1 hour. The solvent was distilled off and the residue was dissolved in 80 ml of a 80 % solution of acetic acid to undergo reaction at 50 to 60°C for 2 hours. The reaction mixture was cooled with ice for 1 hour, the trityl alcohol was separated by filtration and the mother liquor was concentrated. To the concentrate was added ethanol and the mixture was stirred. The crystals precipitated were separated by filtration and dried, affording 5.0 g of the title compound in a yield of 72 %.
M.p. 138-139°C

The analysis by ¹H-NMR showed that the compound thus prepared was identical with the compound obtained in Example 115.

### Examples 119 to 147 (for comparison)

The general procedure of Example 115 was followed, thereby producing compounds identical with those prepared in Examples 1, 3 to 25, 90 to 93 and 114, respectively.

### Examples 148 to 176 (for comparison)

The general procedure of Example 117 was followed, thereby producing compounds identical with those prepared in Examples 1, 3 to 25, 90 to 93 and 114, respectively.

### Example 177 (for comparison)

### Preparation of 3'-O-benzyl-2'-deoxy-5-fluoro-5'-O-stearoyluridine

The title compound was prepared as an oil by carrying out the same reaction and subsequent treatment as in Example 26. Yield 78 %.
¹H-NMR(CDCl₃) δ:
7.65 (1H, d, J = 6Hz, C₆-H)
7.32 (5H, s, phenyl-H)
6.23 (1H, t, J = 6Hz, C_{1'}-H)
4.54 (2H, d, J = 2Hz, 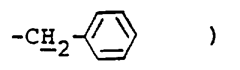 4.29-4.01 (4H, m, C_{3'}·_{4'}·_{5'}-H)
2.33-1.83 (4H, m, C_{2'}-H and -OCOCH₂-)
1.25 (30H, bs, -(CH₂)₁₅-)
0.88 (3H, t, -CH₃)

### Example 178 (for comparison)

### Preparation of 5'-O-cyclohexanoyl-2'-deoxy-3'-O-(2,4-dichlorobenzyl)-5-fluorouridine

The title compound was prepared as an oil by carrying out the same reaction and subsequent treatment as in Example 26. Yield 77 %.
¹H-NMR(CDCl₃) δ:
9.50 (1H, b, NH)
7.67 (1H, d, J = 6Hz, C₆-H)
7.40-7.16 (3H, m, phenyl-H)
6.25 (1H, t, J = 6Hz, C_{1'}-H)
4.58 (2H, s, 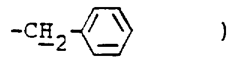 4.37-4.08 (4H, m, C_{3'}·_{4'}·_{5'}-H)
2.77-2.51 and 2.48-1.04
(13H, m, C_{2'}-H and cyclohexyl-H)

### Example 179 (for comparison)

### Preparation of 2'-deoxy-5-fluoro-3'-O-(4-methoxybenzyl)-5'-O-(2-thenoyl)uridine

The title compound was prepared as a powder by carrying out the same reaction and subsequent treatment as in Example 26. Yield 91 %.
¹H-NMR(CDCl₃) δ:
9.20 (1H, bs. NH)
7.82 (1H, dd, J_{4,5} = 4Hz, J_{3,5} = 2Hz, 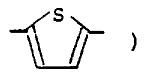 7.63 (1H, d, J = 6Hz, C₆-H)
7.59 (1H, dd, J_{3,4}=5Hz, J_{4,5}=4Hz, 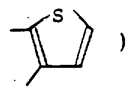 7.29-7.08 (3H, m, 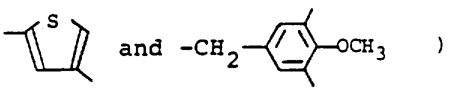 6.87 (2H, d, J = 9Hz, 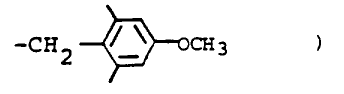 6.25 (1H, t, J = 6Hz, C_{1'}-H)
4.56-4.10 (6H, m, C_{3'}·_{4'}·_{5'}-H and 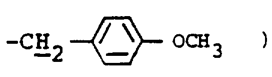 3.78 (3H, s, -OCH₃)
2.74-2.48 and 2.22-1.78 (2H, m, C_{2'}-H)

### Example 180 (for comparison)

### Preparation of 2'-deoxy-5-fluoro-3'-(3-methylbenzyl)-5'-O-(2-furoyl)uridine

The title compound was prepared as a powder by carrying out the same reaction and subsequent treatment as in Example 26. Yield 85 %. ¹H-NMR(CDCl₃) δ:
9.14 (1H, bs, NH)
7.93 (1H, d, J = 6Hz, C₆-H)
7.58 (1H, bs, 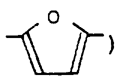 7.29-7.10 (5H, m, furanyl-H, 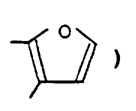 6.55 (1H, dd, J_{3,4}=4Hz, J_{4,5}=2Hz, 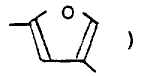 6.35 (1H, t, J = 6Hz, C_{1'}-H)
4.77-4.10 (6H, m, C_{3'}·_{4'}·_{5'}-H and -CH₂
2.71-2.44 and 2.34-1.97 (5H, m, C_{2'}-H and CH₃)

### Example 181 (for comparison)

### Preparation of 3'-O-benzyl-5'-O-crotonoyl-2'-deoxy-5-fluorouridine

The title compound was prepared as a powder in a yield of 75 % by carrying out the same reaction and treatment as in Example 54.
¹H-NMR(CDCl₃) δ:
8.63 (1H, bs, NH)
7.67 (1H, d, J = 6Hz, C₆-H)
7.32 (5H, s, phenyl-H)
7.14-6.89 (1H, m, -CH=CHCH₃)
6.24 (1H, t, J = 6Hz, C_{1'}-H)
5.84 (1H, dd, J_{α,β} = 16Hz, J_{α,γ} = 2Hz, CH=CHCH₃)
4.54 (2H, s, 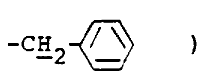 4.42-4.05 (4H, m, C_{3'}·_{4'}·_{5'}-H)
2.73-2.44 and 2.20-2.02 (2H, m, C_{2'}-H)
1.90 (3H, dd, J_{β,γ} = 7Hz, J_{α,γ} = 2Hz, -CH=CHCH₃)

### Example 182 (for comparison)

### Preparation of 3'-(2-bromobenzyl)-2'-deoxy-5'-O-ethoxyacetyl-5-fluorouridine

A 1.24 g quantity of DCC was added to a solution of 1 g of 3'-(2-bromobenzyl)-2'-deoxy-5-fluorouridine and 0.63 g of ethoxy acetate in 10 ml of pyridine. The mixture was stirred at room temperature for 24 hours. The insolubles were separated by filtration and the filtrate was concentrated. The concentrate was purified with isopropanol-ether, giving 1.06 g of the title compound as a powder in a yield of 80 %.
¹H-NMR(CDCl₃) δ:
9.49 (1H, bs, NH)
7.74 (1H, d, J = 6Hz, C₆-H)
7.60-7.06 (4H, m, phenyl-H)
6.34 (1H, t, J = 6Hz, C_{1'}-H)
4.60 (2H, s, 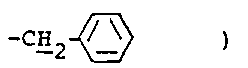 4.39-4.13 (6H, m, C_{3'}·_{4'}·_{5'}-H and -COCH₂O-)
3.59 (2H, q, J = 7Hz, -OCH₂CH₃)
2.73-2.48, 2.31-2.00 (2H, m, C_{2'}-H)
1.22 (3H, t, J = 7Hz, -OCH₂CH₃)

### Example 183 (for comparison)

### Preparation of 2'-deoxy-5-fluoro-3'-0-(2,4,6-trimethylbenzyl)uridine

A 1.66 g quantity of 2,4,6-trimethylbenzyl chloride was added to a solution of 4.00 g of 2'-deoxy-5-fluoro-5'-O-trityluridine, 2.30 g of potassium hydroxide and 1.47 g of sodium iodide in 50 ml of dried dioxane. The mixture was stirred at 60°C for 3 hours. The solvent was distilled off, and ethyl acetate and water were added to the residue. The ethyl acetate layer was washed with water and dried over anhydrous sodium sulfate. The ethyl acetate was distilled off and the residue was dissolved in 50 ml of a 80 % solution of acetic acid. The resulting solution was left to stand at 70°C for 2 hours. The reaction mixture was concentrated and water was added to the concentrate. The mixture was rendered weakly basic with an aqueous solution of sodium hydroxide and washed with ether. The aqueous layer was made weakly acidic with a 6N solution of hydrochloric acid and extracted with ethyl acetate. The ethyl acetate layer was washed with water, dried over anhydrous sodium sulfate and concentrated. Ether was added to the concentrate to deposit solids and the solids were recrystallized from ethanol, giving 1.69 g of the title compound in a yield of 55 %.
M.p. 179-181°C
¹H-NMR(DMSO-d₆)δ:
11.82 (1H, bs, NH)
8.20 (1H, d, J = 7Hz, C₆-H)
6.83 (2H, s, 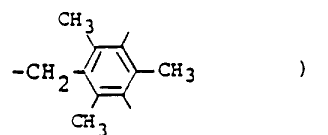 6.07 (1H, bt, J = 6Hz, C_{1′}-H)
5.19 (1H, bt, J = 5Hz, 5′-OH)
4.47 (2H, s, 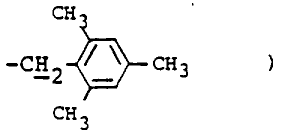 4.20-4.10 (1H, m, C_{3'}-H)
4.02-3.91 (1H, m, C_{4'}-H)
3.69-3.57 (2H, m, C_{5'}-H)
2.29-2.12 (11H, m, CH₃ x 3 and C_{2'}-H)

### Example 184

### Preparation of 2'-deoxy-3-[3-[6-(2,4-dichlorobenzoyloxy)-2-pyridyloxycarbonyl]benzoyl]-5-fluoro-3'-O-(3-methylbenzyl)-5'-O-(2-furoyl)uridine

The title compound was prepared as a powder by carrying out the same reaction and subsequent treatment as in Example 73. Yield 24 %.
¹H-NMR(CDCl₃) δ:
8.66 (1H, t, J = 2Hz, 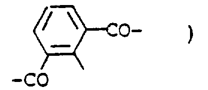 8.50 and 8.26 (2H, each dt, J = 8Hz, J = 2Hz, 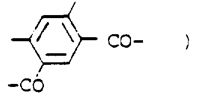 8.14-7.92 (3H, m, 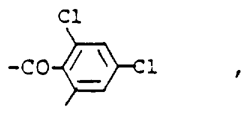 C₆-H, C₄-H of the pyridine ring)
7.69 (1H, t, J = 8Hz, 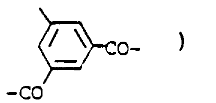 7.60 (1H, bs, C₅-H of furanyl)
7.53 (1H, d, J = 2Hz, 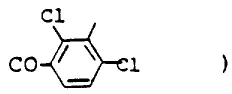 7.36 (1H, dd, J = 8Hz, J = 2Hz, 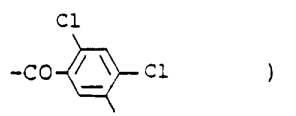 7.30-7.11 (7H, m, 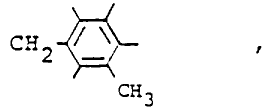 C_{3,5}-H of the pyridine ring and C₃-H of furanyl)
6.55 (1H, dd, J = 4Hz, J = 2Hz, C₄-H of furanyl)
6.34 (1H, t, J = 6Hz, C_{1′}-H)
4.75-4.15 (6H, m, C_{3'}·_{4'}·_{5'}-H and 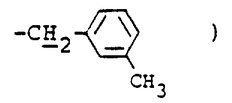 2.77-2.01 (5H, C_{2′}-H and CH₃)

### Example 185

### Preparation of 5′-O-cyclohexanoyl-2′-deoxy-3′-O-(2,4-dichlorobenzyl)-5-fluoro-3-[3-[3-cyano-6-(3-methylbenzoyloxy-2-pyridyloxycarbonyl]benzoyl]uridine

The title compound was prepared as a powder by carrying out the same reaction and subsequent treatment as in Example 73. Yield 13 %.
¹H-NMR(CDCl₃)δ:
8.66 (1H, t, J = 2Hz, 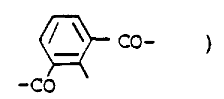 8.52 and 8.35-8.18 (3H, m, 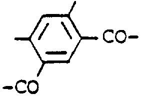 and C₄-H of the pyridine ring)
8.04-7.92 (2H, m, 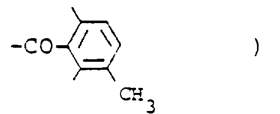 7.86-7.65 (2H, m, C₆-H and 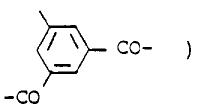 7.49-7.16 (6H, m, C₅-H of the pyridine ring, 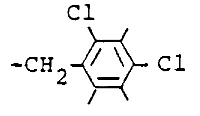 and 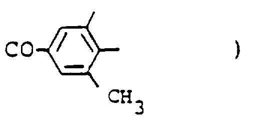 6.22 (1H, t, J = 6Hz, C_{1'}-H)
4.55 (2H, s, 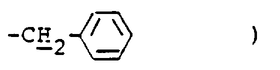 4.42-4.09 (4H, m, C_{3'}·_{4'}·_{5'}-H)
2.85-1.10 (16H, m, C_{2'}-H, CH₃, cyclohexyl)

### Example 186

### Preparation of 3'-O-benzyl-3-[3-(5-chloro-1,2-dihydro-1-ethoxymethyl-2-oxo-4-pyridyloxycarbonyl)benzoyl]-2'-deoxy-5-fluorouridine

The title compound was prepared as a powder by carrying out the same reaction and subsequent treatment as in Example 101. Yield 49 %.
¹H-NMR(CDCl₃) δ:
8.67-8.19 (4H, m, C₆-H and 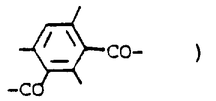 7.77-7.61 (2H, m, 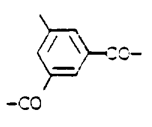 and C₆-H of the pyridine ring)
7.30 (5H, s, 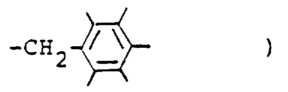 6.66 (1H, s, C₃-H of the pyridine ring)
6.20 (1H, t, J = 6Hz, C_{1'}-H)
5.34 (2H, s, N-CH₂O-)
4.52 (2H, s, 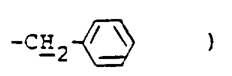 4.32-3.81 (4H, m, C_{3'}·_{4'}·_{5'}-H)
3.64 (2H, q, J = 7Hz, -OCH₂CH₃)
3.10 (1H, bs, 5'-OH)
2.52-2.19 (2H, m, C_{2'}-H)
1.24 (3H, t, J = 7Hz, OCH₂CH₃)

### Example 187

### Preparation of 3-[3-(6-benzoyloxy-3-chloro-2-pyridyloxycarbonyl)benzoyl]-3'-O-benzyl-2'-deoxy-5-fluorouridine

The title compound was prepared as a powder by carrying out the same reaction and subsequent treatment as in Example 101. Yield 39 %.
¹H-NMR(CDCl₃) δ:
8.71-8.10 (6H, m, C₆-H and 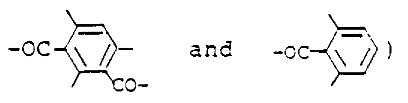 7.97 (1H, d, J = 8Hz, C₄-H of the pyridine ring)
7.76-7.40 (4H, m, 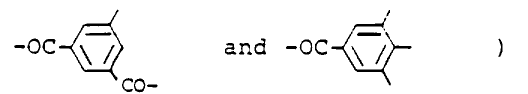 7.28 (5H, s, 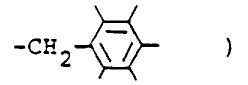 7.25 (1H, d, J = 8Hz, C₅-H of the pyridine ring)
6.23 (1H, t, J = 6Hz, C_{1′}-H)
4.48 (2H, s, 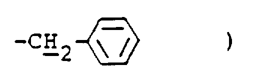 4.26-3.66 (4H, m, C_{3'}·_{4'}·_{5'}-H)
3.07 (1H, bs, C_{5′}-OH)
2.52-2.12 (2H, m, C_{2′}-H)

### Example 188

### Preparation of 3-[3-(6-benzoyloxy-3-chloro-2-pyridyloxycarbonyl)benzoyl]-3′-O-(4-chloro-benzyl)-2′-deoxy-5-fluorouridine

The title compound was prepared as a powder by carrying out the same reaction and subsequent treatment as in Example 101. Yield 45 %.
¹H-NMR(CDCl₃)δ:
8.69-8.11 (6H, m, C₆-H and 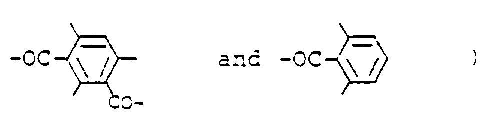 7.98 (1H, d, J = 8Hz, C₄-H of the pyridine ring)
7.76-7.39 (4H, m, 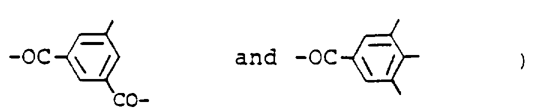 7.30-7.11 (5H, m, 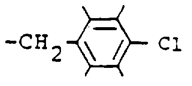 and C₅-H of the pyridine ring)
6.22 (1H, t, J = 6Hz, C_{1′}-H)
4.42 (2H, s, 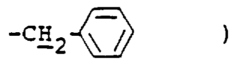 4.23-3.75 (4H, m, C_{3'}·_{4'}·_{5'}-H)
3.06 (1H, bs, C_{5'}-OH)
2.66-2.02 (2H, m, C_{2'}-H)

### Example 189

### Preparation of 3-[4-(6-benzoyloxy-3-cyano-2-pyridyloxycarbonyl)-3-furoyl]-3′-O-benzyl-2′-deoxy-5-fluorouridine

The title compound was prepared as a powder by carrying out the same reaction and subsequent treatment as in Example 101. Yield 31 %.
¹H-NMR(CDCl₃)δ:
8.35 (2H, s, 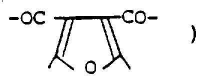 8.18-7.94 (4H, m, C₆-H, C₄-H of the pyridine ring and 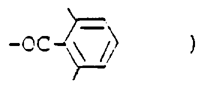 7.63-7.38 (4H, m, C₅-H of the pyridine ring
and 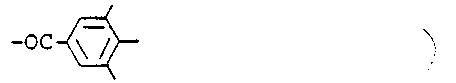 7.25 (5H, s, 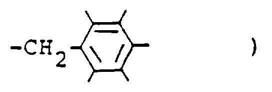 6.12 (1H, t, J = 6Hz, C_{1'}-H)
4.39 and 4.37 (each 1H, s, 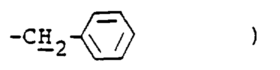 4.25-3.55 (4H, m, C_{3'}·_{4'}·_{5'}-H)
2.78 (1H, bs, C_{5'}-OH)
2.60-2.11 (2H, m, C_{2'}-H)

### Example 190

### Preparation of 3-[3-(6-benzoyloxy-3-cyano-2-pyridyloxycarbonyl)benzoyl]-3'-O-benzyl-2'-deoxy-5-fluorouridine

The title compound was prepared as a powder by carrying out the same reaction and subsequent treatment as in Example 101. Yield 33 %.
¹H-NMR(CDCl₃) δ:
8.71-8.12 (7H, m, C₆-H, C₄-H of the pyridine
ring and 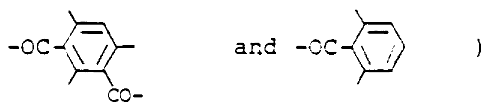 7.78-7.37 (5H, m, C₅-H of the pyridine ring and 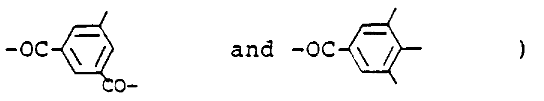 7.29 (5H, s, 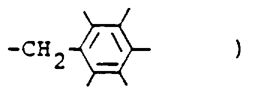 6.23 (1H, t, J = 6Hz, C_{1′}-H)
4.49 (2H, s, 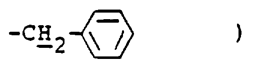 4.29-3.66 (4H, m, C_{3'}·_{4'}·_{5'}-H)
3.47 (1H, bs, C_{5'}-OH)
2.68-2.19 (2H, m, C_{2'}-H)

### Example 191

### Preparation of 3-[3-(6-benzoyloxy-3-cyano-2-pyridyloxycarbonyl)benzoyl]-3′-O-(2-chlorobenzyl)-2′-deoxy-5-fluorouridine

The title compound was prepared as a powder by carrying out the same reaction and subsequent treatment as in Example 101. Yield 43 %.
¹H-NMR(CDCl₃)δ:
8.72-8.12 (7H, m, 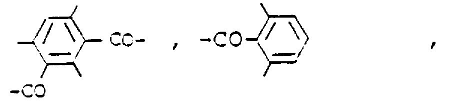 C₆-H, C₄-H of the pyridine ring)
7.78-7.15 (9H, m, 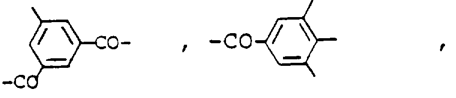 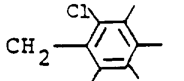 and C₅-H of the pyridine ring)
6.26 (1H, bt, J = 6Hz, C_{1'}-H)
4.57 (2H, s, 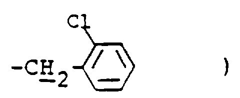 4.33-4.17 (2H, m, C_{3'}·_{4'}-H)
4.01-3.71 (2H, m, C_{5'}-H)
2.86 (1H, bs, 5'-OH)
2.68-2.14 (2H, m, C_{2'}-H)

### Example 192

### Preparation of 3-[3-(6-benzoyloxy-3-cyano-2-pyridyloxycarbonyl)benzoyl]-2'-deoxy-5-fluoro-3'-O-(4-fluorobenzyl)uridine

The title compound was prepared as a powder by carrying out the same reaction and subsequent treatment as in Example 101. Yield 57 %.
¹H-NMR(CDCl₃) δ:
8.70-8.10 (7H, m, 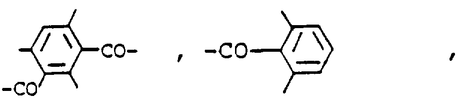
C₆-H, C₄-H of the pyridine ring)
7.79-6.8 (6H, m, 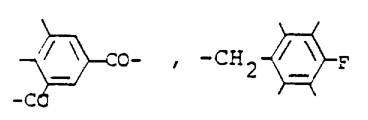 and C₅-H of the pyridine ring)
6.22 (1H, bt, J = 6Hz, C_{1'}-H)
4.45 (2H, s, 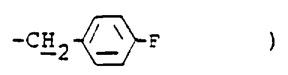 4.31-4.10 (2H, m, C_{3'}·_{4'}-H)
4.00-3.63 (2H, m, C_{5'}-H)
2.80-2.00 (3H, m, 5'-OH, C_{2'}-H)

### Example 193

### Preparation of 3-[3-(6-benzoyloxy-3-chloro-2-pyridyloxycarbonyl)benzoyl]-2'-deoxy-5-fluoro-3'-O-(2,4,6-trimethylbenzyl) uridine

The title compound was prepared as a powder by carrying out the same reaction and subsequent treatment as in Example 101. Yield 47 %.
¹H-NMR(CDCl₃) δ:
8.67-8.12 (6H, m, 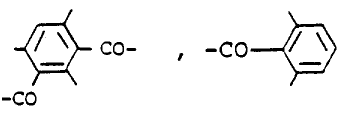 and C₆-H)
7.97 (1H, d, J = 8Hz, C₄-H of the pyridine ring)
7.77-7.37 (4H, m, 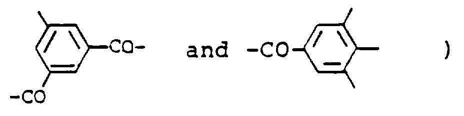
7.25 (1H, d, J = 8Hz, C₅-H of the pyridine ring)
6.82 (2H, s, 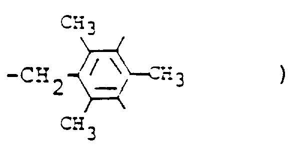 6.27 (1H, bt, J = 6Hz, C_{1′}-H)
4.48 (2H, s, 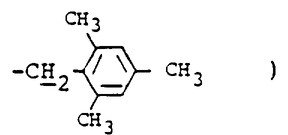 4.27-4.02 (2H, m, C_{3'}·_{4'}-H)
4.00-3.71 (2H, m, C_{5′}-H)
2.72-2.05 (12H, m, 5′-OH, C_{2′}-H, CH₃ x 3)

### Example 194

### Preparation of 3′-O-benzyl-2′-deoxy-5-fluoro-3-[3-(1,6-dihydroxy-6-oxo-2-pyridyloxy)benzoyl]uridine

The title compound was prepared as a powder by carrying out the same reaction and subsequent treatment as in Example 104. Yield 33 %.
¹H-NMR(DMSO-d₆)δ:
11.45 (1H, bs, NH)
8.66-8.44 (4H, m, 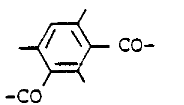 and C₄-H of the pyridone ring)
7.94-7.73 (2H, m, 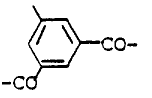 and C₆-H)
7.33 (5H, s, phenyl-H)
6.81 and 6.64 (2H, each d, J = 8Hz, C_{3,5}-H of the pyridone ring)
6.11 (1H, t, J = 7Hz, C_{1'}-H)
5.29 (1H, t, J = 6Hz, 5'-OH)
4.54 (2H, s, 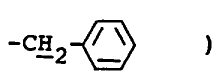 4.29-4.10 (2H, m, C_{3'}·_{4'}-H)
3.74-3.63 (2H, m, C_{5'}-H)
2.45-2.23 (2H, m, C_{2'}-H)

### Example 195

### Preparation of 3-[3-(6-benzoyloxy-3-cyano-2-pyridyloxycarbonyl)benzoyl] -3'-O-benzyl-2'-deoxy-5-fluorouridine

Triethylamine (2.88 ml) and 1.01 g of isophthaloyl chloride were added to a solution of 1.00 g of 6-benzoyloxy-3-cyano-2-hydroxypyridine in 50 ml of dioxane. The mixture was stirred at room temperature for 1.5 hours.

Aside from the above procedure, 2.88 ml of triethylamine and 0.69 ml of trimethylsilyl chloride were added to a solution of 1.40 g of 3'-O-benzyl-2'-deoxy-5-fluorouridine in 50 ml of dioxane. The mixture was stirred at room temperature for 1 hour. The reaction mixture was filtered and concentrated. The concentrate was added to the reaction mixture prepared above and the resulting mixture was stirred at 80°C for 30 minutes. The reaction mixture was filtered and concentrated. The residue was-dissolved in 20 ml of acetone. Thereto was added 5 ml of water. The mixture was stirred at 75°C for 1 hour. The acetone was distilled off and the residue was extracted with 50 ml of ethyl acetate. The organic layer was washed with water, dried over magnesium sulfate and concentrated. The concentrate was subjected to florisil column chromatography using as an eluent benzene-ethyl acetate (4:1), giving 80 mg of the title compound in a yield of 3 %.

Analysis by thin layer chromatography and liquid chromatography showed that the compound obtained was identical with the compound prepared in Example 190.

### Example 196 (for comparison)

### Preparation of 2'-deoxy-5-fluoro-3'-O-(4-methoxycarbonylbenzyl)uridine

The general procedure of Example 114 was followed, thereby producing the title compound.
M.p. 169-170°C
¹H-NMR(CDCl₃) δ:
9.85 (1H, bs, N₃-H)
8.04-7.94 (3H, m, C₆-H and 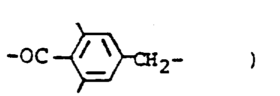
7.37 (2H, d, J = 8Hz, 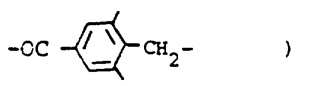 6.24 (1H, t, J = 6Hz, C_{1'} -H)
4.58 (2H, s, 3'-O-CH₂-)
4.31-3.70 (7H, m, C_{3'}·_{4'}·_{5'}-H and -COCH₃)
3.19 (1H, bs, C_{5'}-OH)
2.66-2.04 (2H, m, C_{2'}-H)

### Example 197 (for comparison)

### Preparation of 2'-deoxy-3'-O-cinnamyl-5-fluorouridine

The general procedure of Example 114 was followed, thereby producing the title compound in a yield of 23 %.
¹H-NMR(DMSO-d₆) δ:
11.75 (1H, bs, N₃-H)
8.20 (1H, d, J = 7Hz, C₆-H)
7.53-7.24 (5H, m, 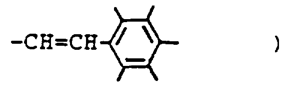 6.64 (1H, d, J = 16Hz, 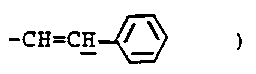 6.47-6.08 (2H, m, C_{1'}-H and 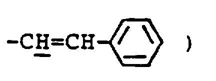 5.20 (1H, bs, C_{5'}-OH)
4.17 (2H, d, J = 5Hz, C_{3'}-O-CH₂-)
4.20-4.03 (2H, m, C_{3'}·_{4'}-H)
3.74-3.59 (2H, m, C_{5'}-H)
2.34-2.21 (2H, m, C_{2'}-H)

### Example 198 (for comparison)

### Preparation of 2'-deoxy-3'-O-(4-dimethylaminobenzyl)-5-fluorouridine

A 0.86 ml quantity of pyridine and 1.19 ml of phosphorus tribromide were added to a solution of 1.60 g of 4-dimethylaminobenzyl alcohol in 20 ml of benzene, and the mixture was stirred at 80°C for 1 hour. The reaction mixture was concentrated and the concentrate was dissolved in 50 ml of a 2:1 mixture of water and acetonitrile. The resulting mixture was adjusted to a pH of 11 by addition of potassium hydroxide. Thereto was added 1.00 g of 2'-deoxy-5-fluorouridine and the mixture was stirred at room temperature for 2 days.

The reaction mixture was poured into ice water and extracted with ethyl acetate. The organic layer was dried over magnesium sulfate and concentrated. The concentrate was placed on silica gel column and eluted with 1 % methanol-chloroform, thereby giving 70 mg of the title compound in a yield of 5 %.
¹H-NMR(DMSO-d₆) δ:
11.98 (1H, bs, N₃-H)
8.18 (1H, d, J = 7Hz, C₆-H)
7.15 (2H, d, J = 9Hz, 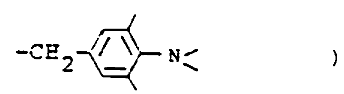 6.69 (2H, d, J = 9Hz, 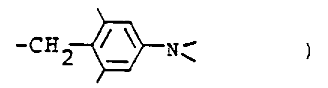 6.09 (1H, t, J = 6Rz, C_{1'}-H)
5.18 (1H, bs, C_{5'}-OH)
4.38 (2H, s, 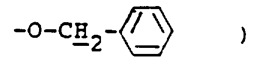 4.18-3.96 (2H, m, C_{3'·4'}-H)
3.70-3.58 (2H, m, C_{5'}-H)
2.88 (6H, s, CH₃ x 2)
2.28-2.09 (2H, m, C_{2'}-H)

### Example 199 (for comparison)

### Preparation of 2'-deoxy-5-fluoro-3'-O-(3-phenylpropyl)-uridine

To a solution of 500 mg of the 2'-deoxy-3'-O-cinnamyl-5-fluoro-5'-O-trityluridine prepared as the intermediate in Example 197 in 30 ml of methanol was added 50 mg of 5 % palladium-carbon, and the catalytic reduction was conducted at room temperature for 1 hour.

The reaction mixture was filtered and concentrated. The concentrate was dissolved in 20 ml of 80 % acetic acid and the solution was stirred at 65°C for 2 hours. The reaction mixture was concentrated, and the concentrate was placed on a silica gel column and eluted with 1% methanol-chloroform, thereby giving 190 mg of the title compound in a yield of 63 %.
¹H-NMR (DMSO-d₆)δ:
11.80 (1H, bs, N₃-H)
8.19 (1H, d, J = 7Hz, C₆-H)
7.22 (5H, s, 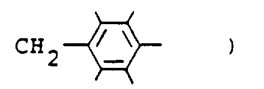 6.12 (1H, t, J = 6Hz, C_{1′}-H)
5.18 (1H, t, J = 5Hz, C_{5′}-OH)
4.06-3.93 (2H, m, C_{3'·4'}-H)
3.66-3.62 (2H, m, C_{5′}-H)
3.41 (2H, t, J = 6Hz, C_{3′}-O-CH₂-)
2.72-1.66 (6H, m, C_{2′}-H and

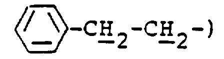

### Pharmacological Test I

(a) Sarcoma-180 subcultured in an ascites of ICR mice was diluted with a physiological saline solution and subcutaneously transplanted into the backs of ICR mice in an amount of 2 X 10⁷ each. Twenty-four hours after the transplantation, a test compound suspended in a 5 % solution of gum arabic was orally administered to each of mice once a day for 7 consecutive days.

The solid tumor was extirpated from under the dorsal skin of mice on the 10th day after the transplantation to measure the weight of the tumor. There was determined the ratio (T/C) of the weight of tumor (T) cut out from the group of mice treated with the test compound to the weight of tumor (C) from the group of mice not treated therewith. The 50 % tumor inhibition dose (ED₅₀ value) in which T/C is 0.5 was determined from the dose-response curve of dosage and the ratio (T/C). Table 1 shows the results.

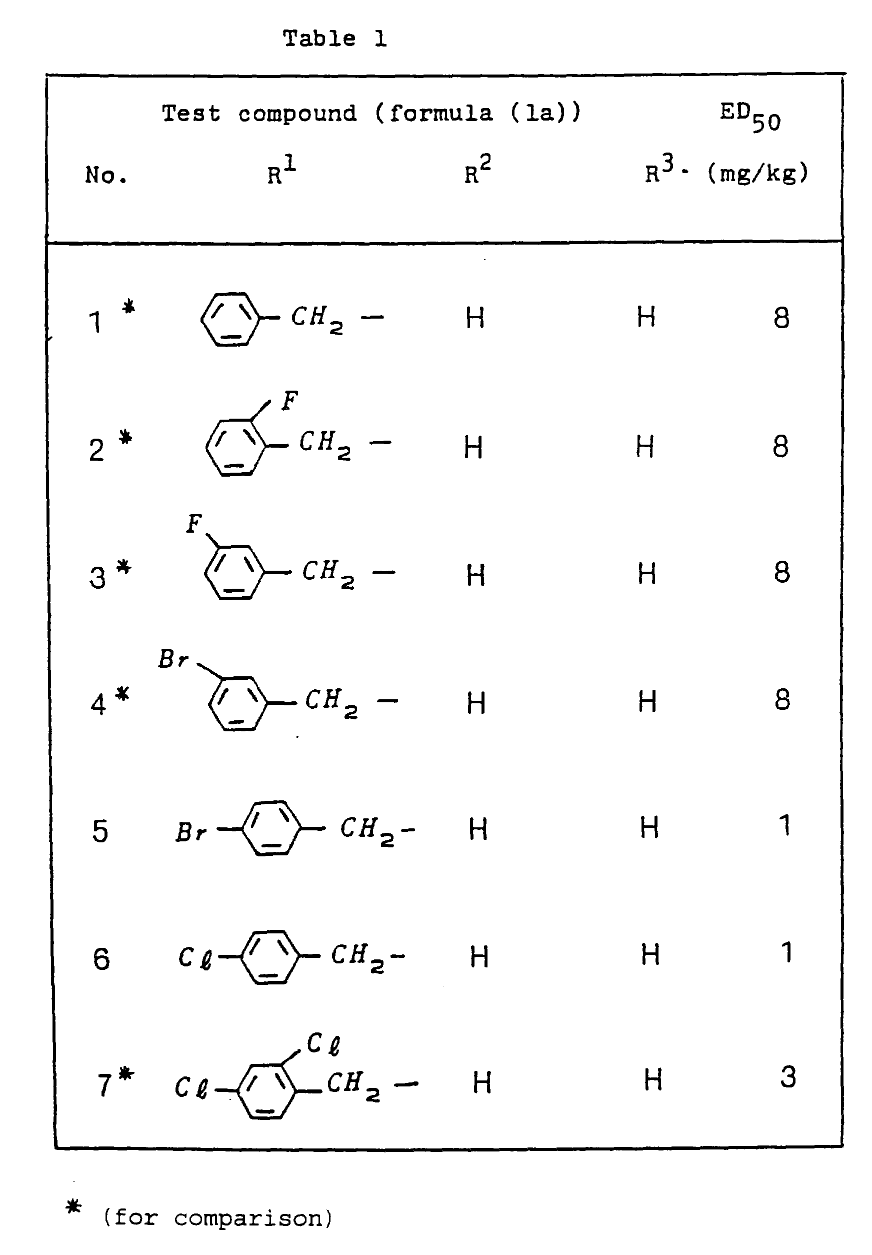

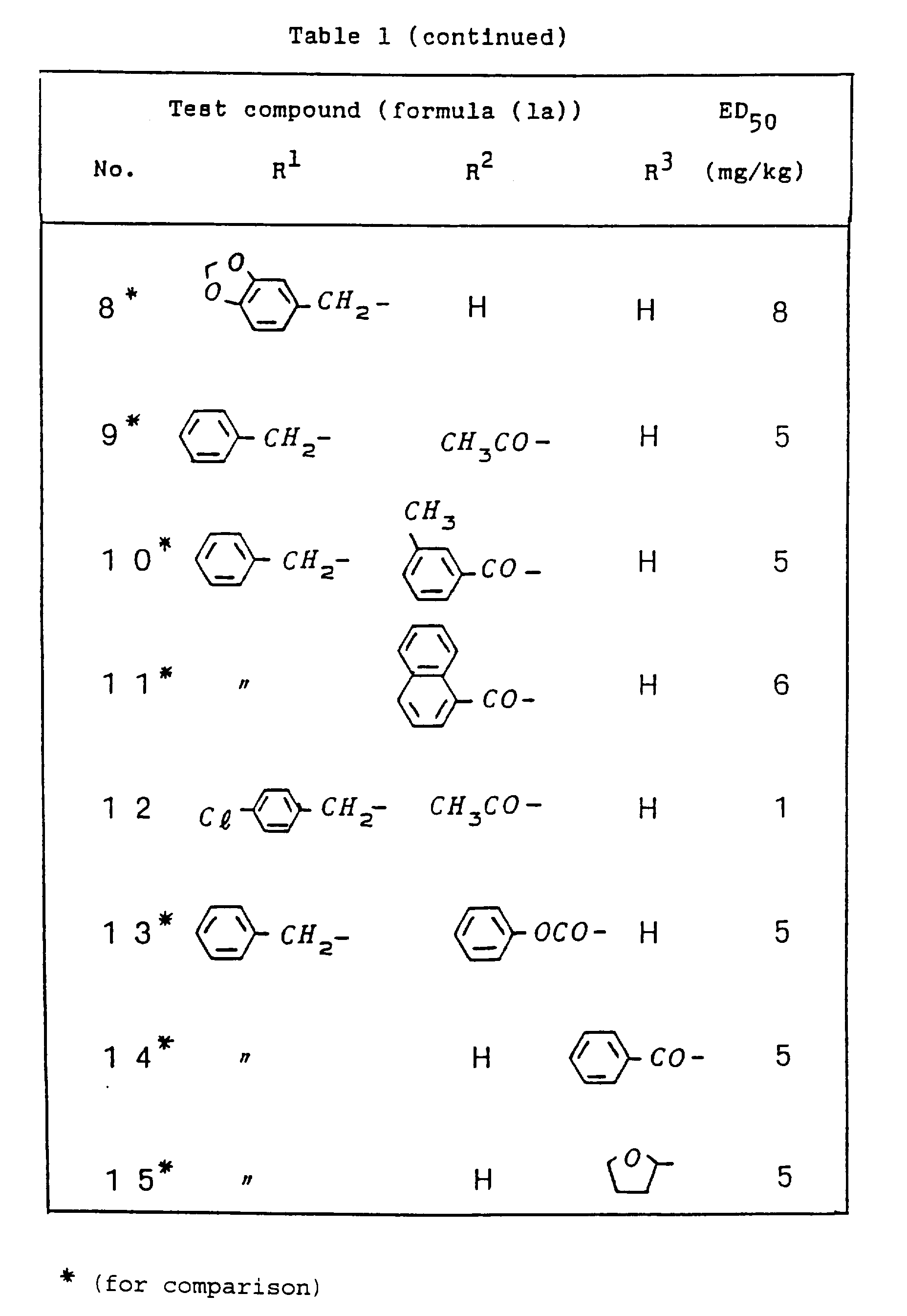

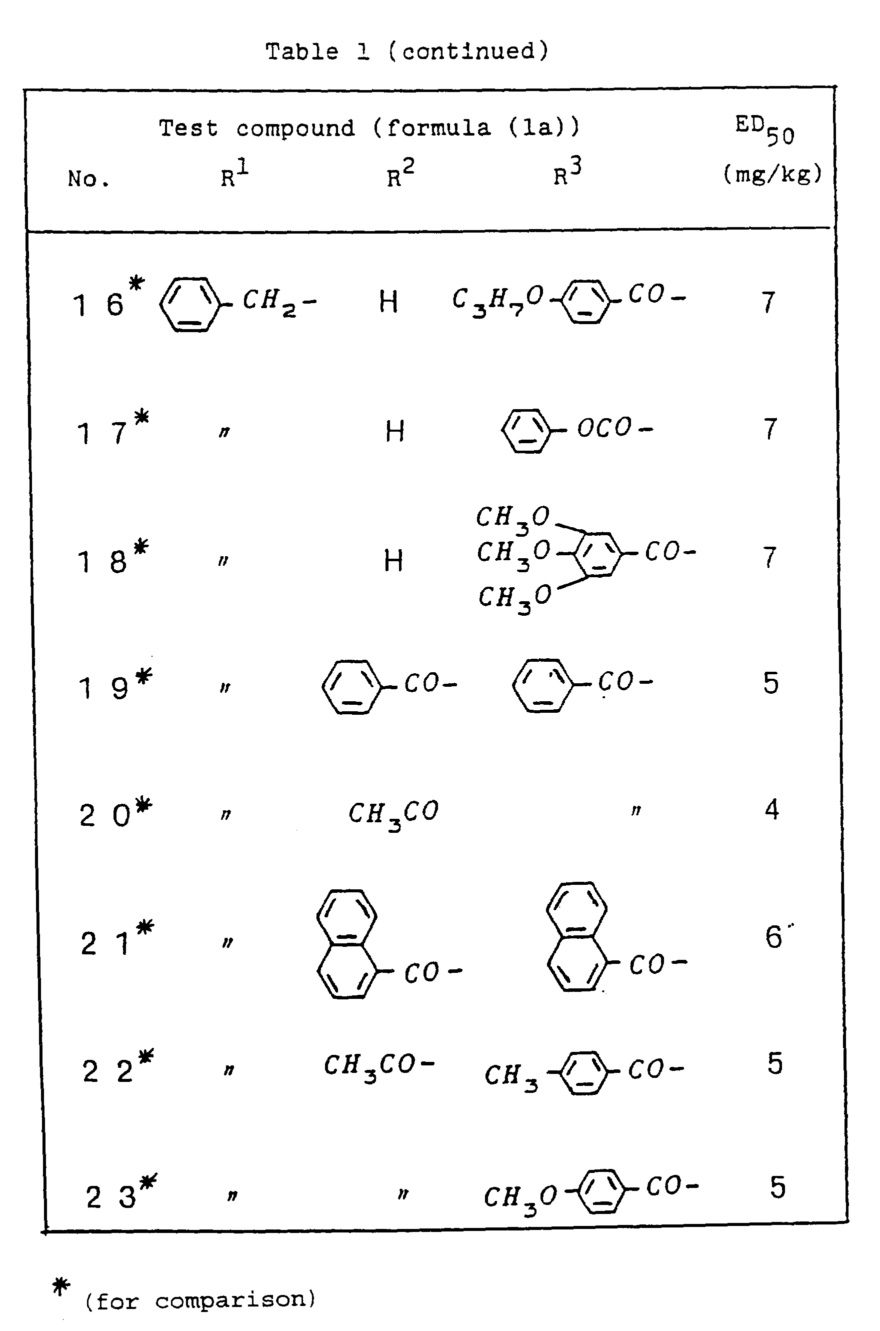

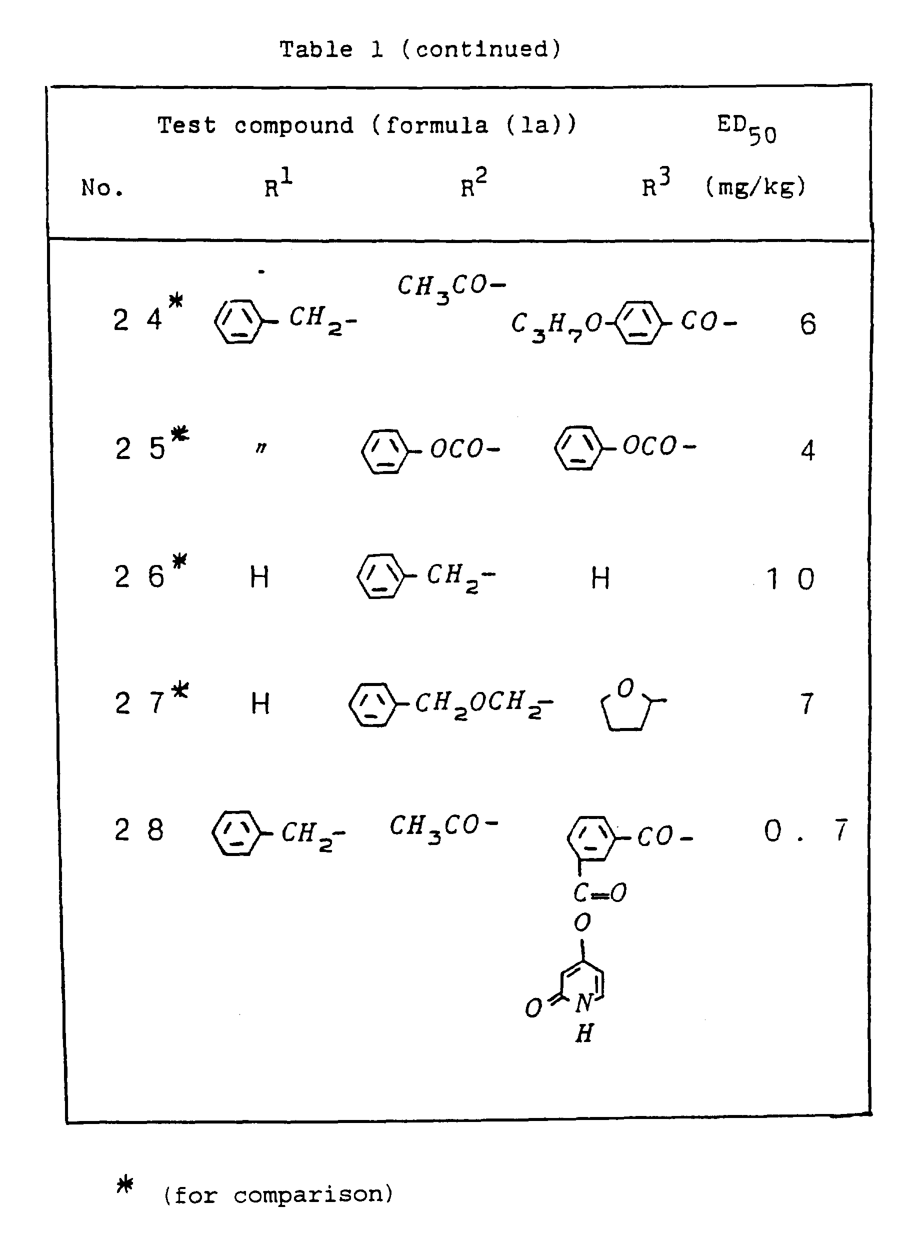

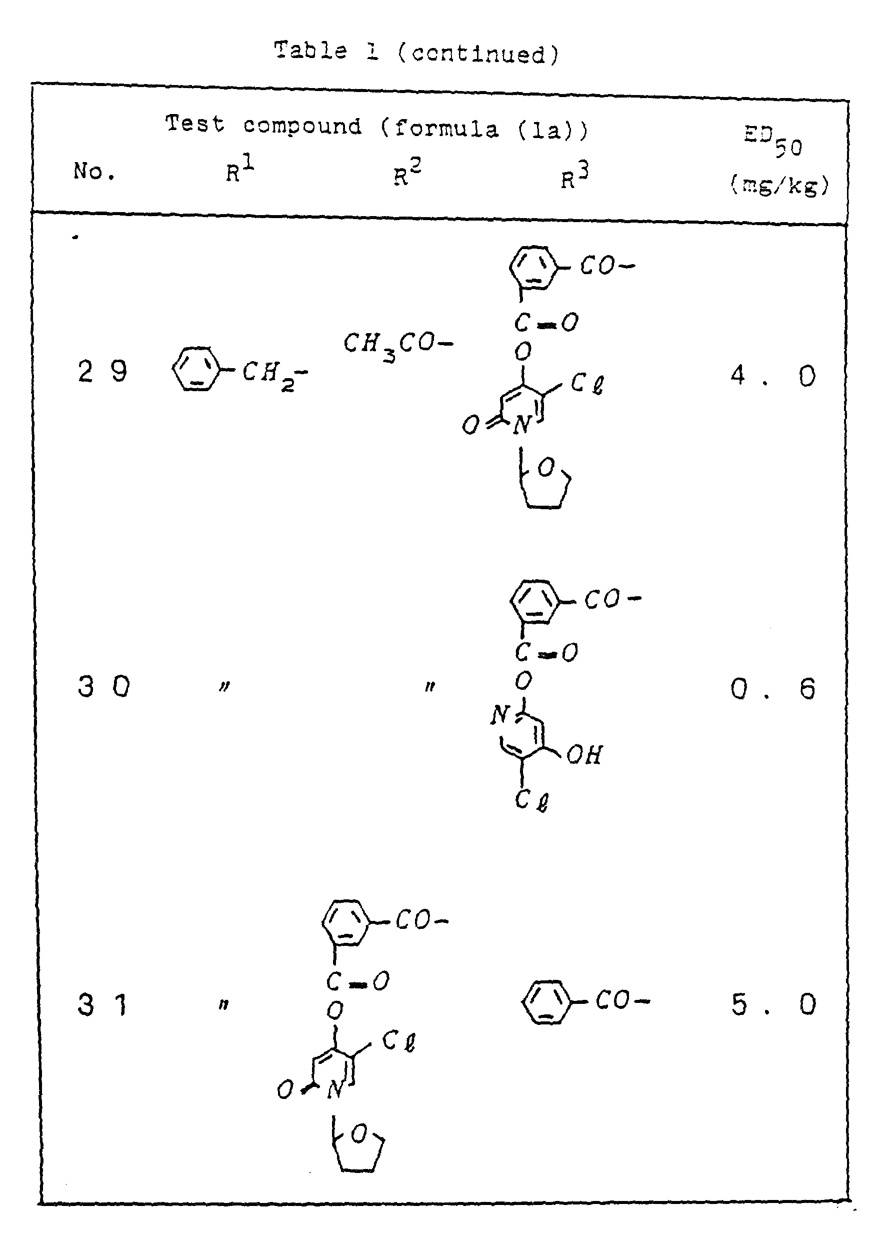

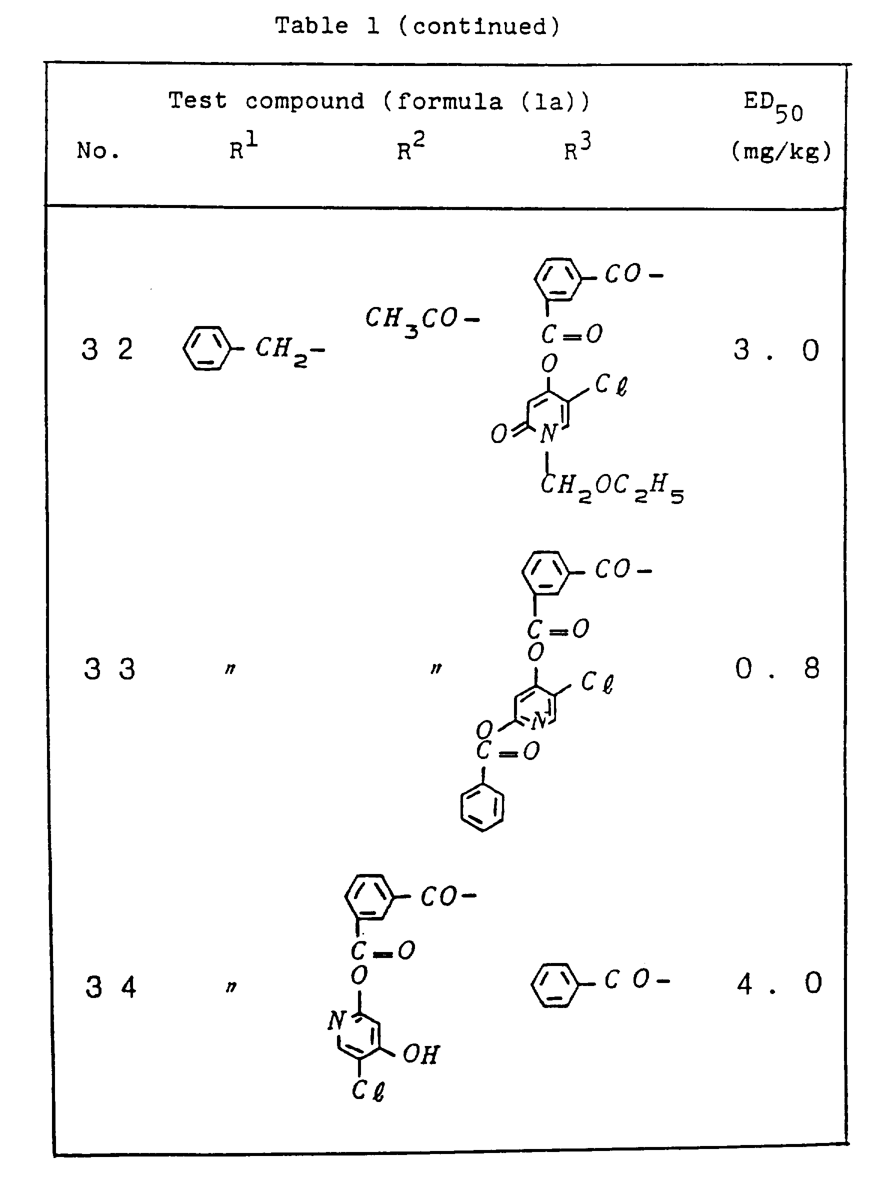

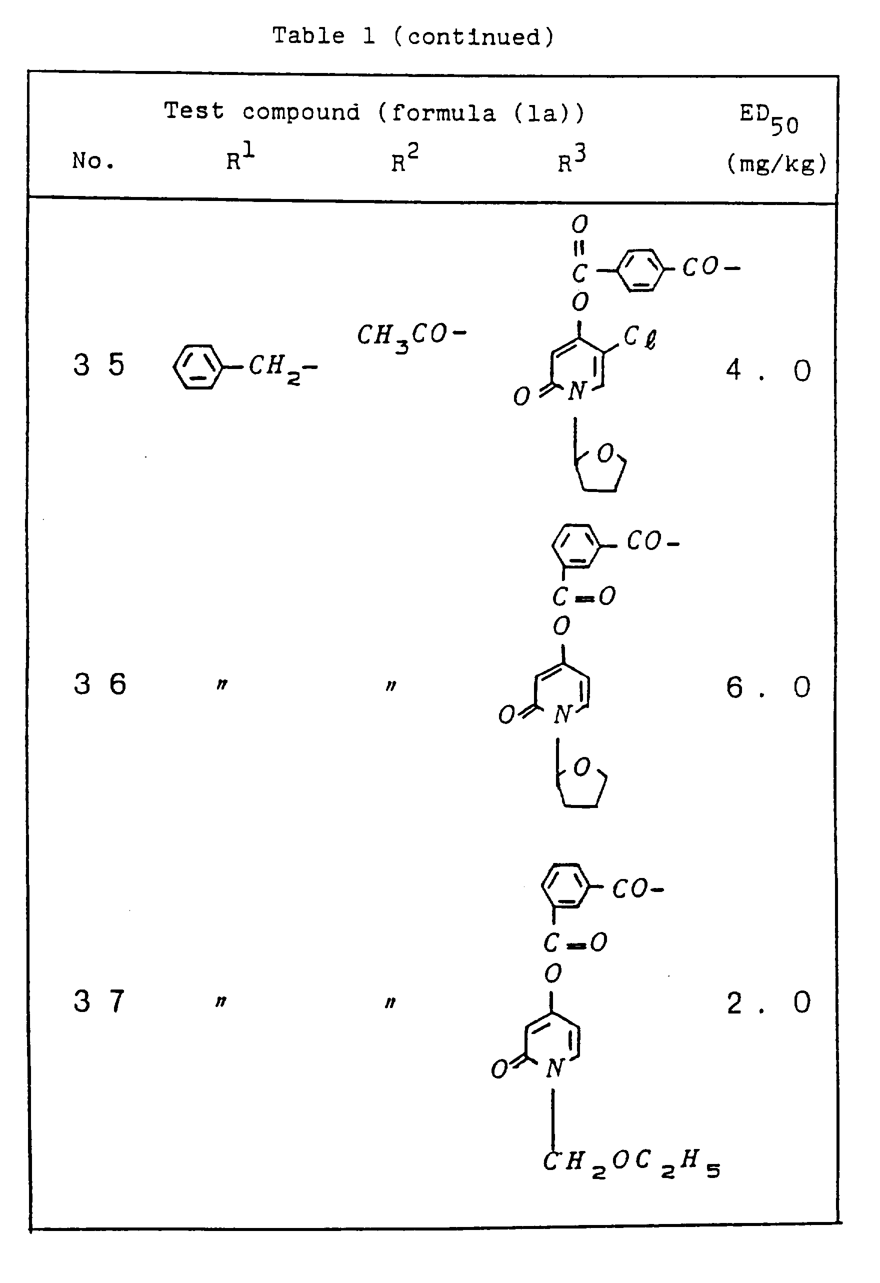

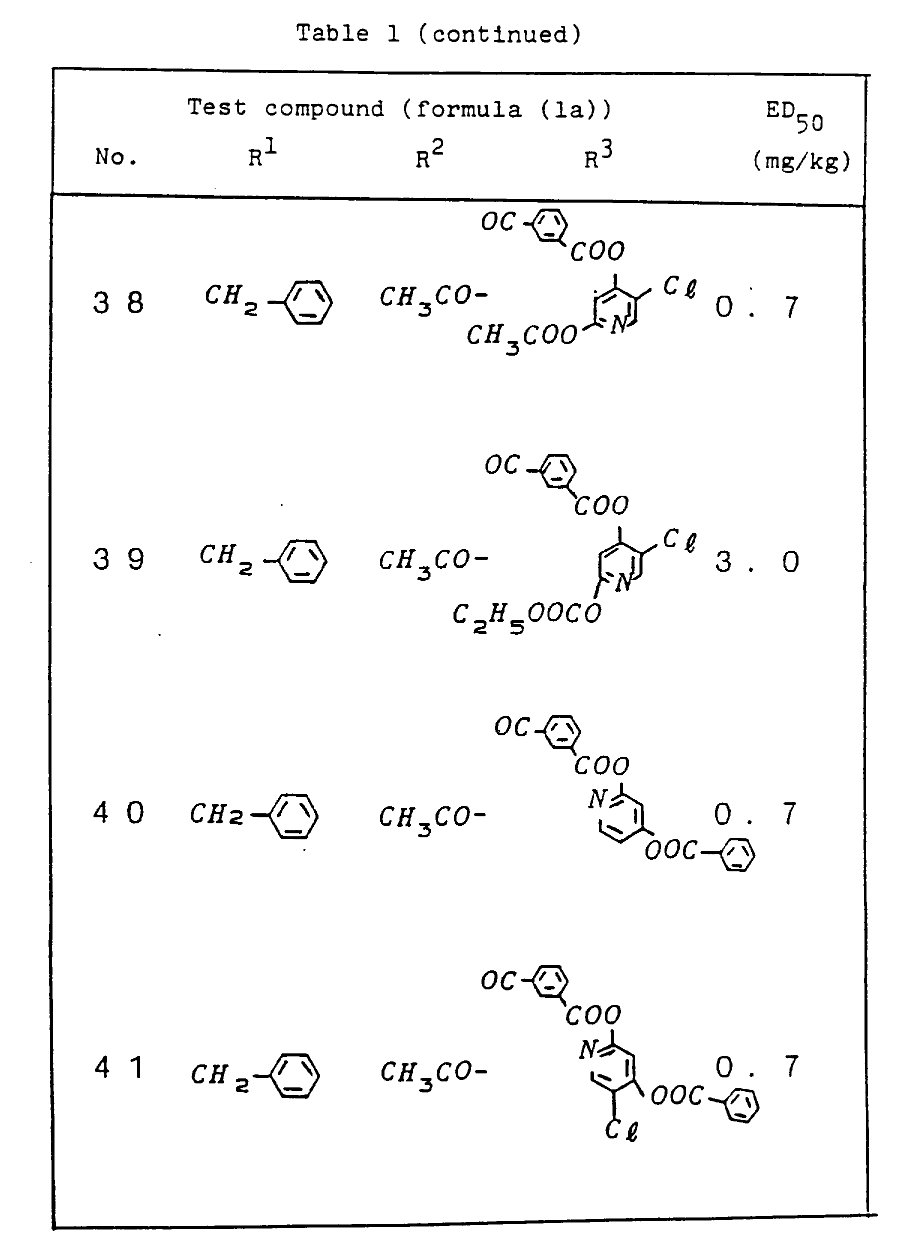

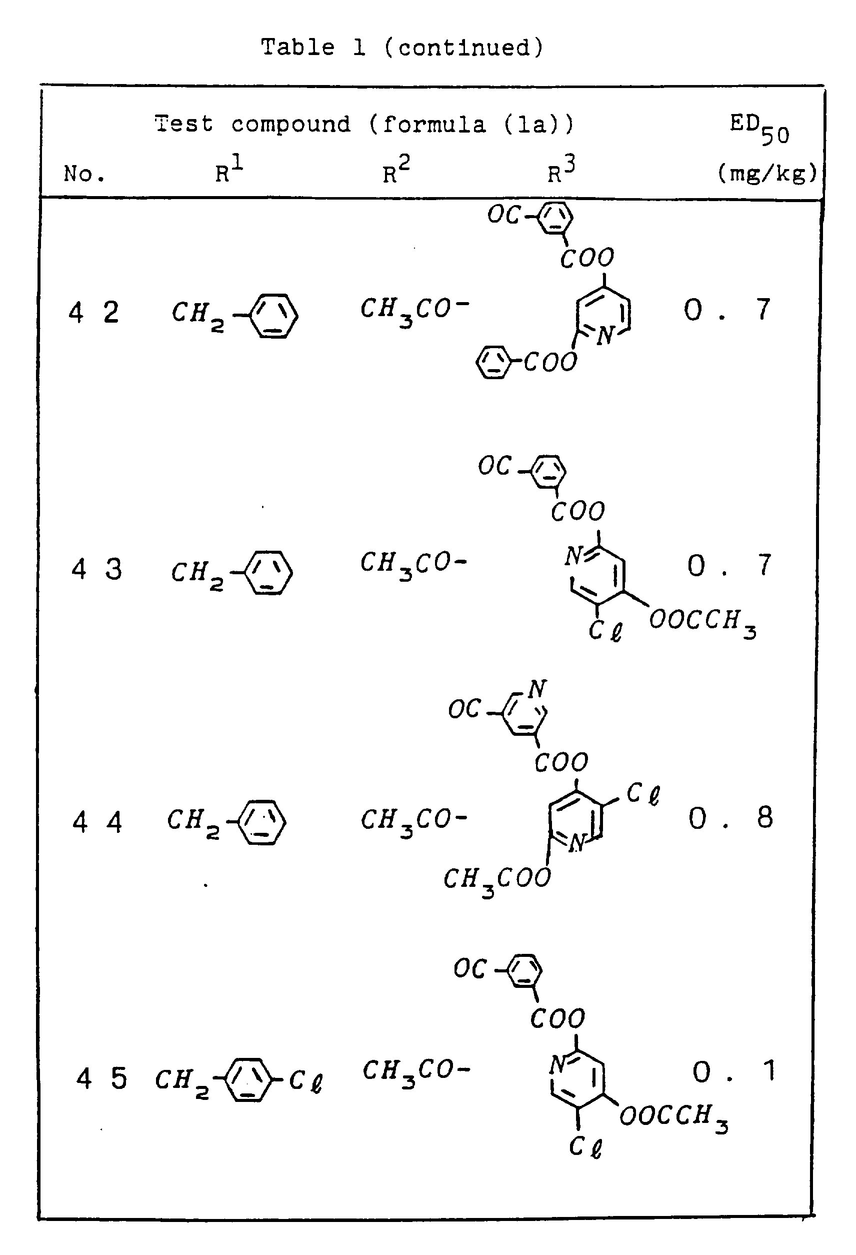

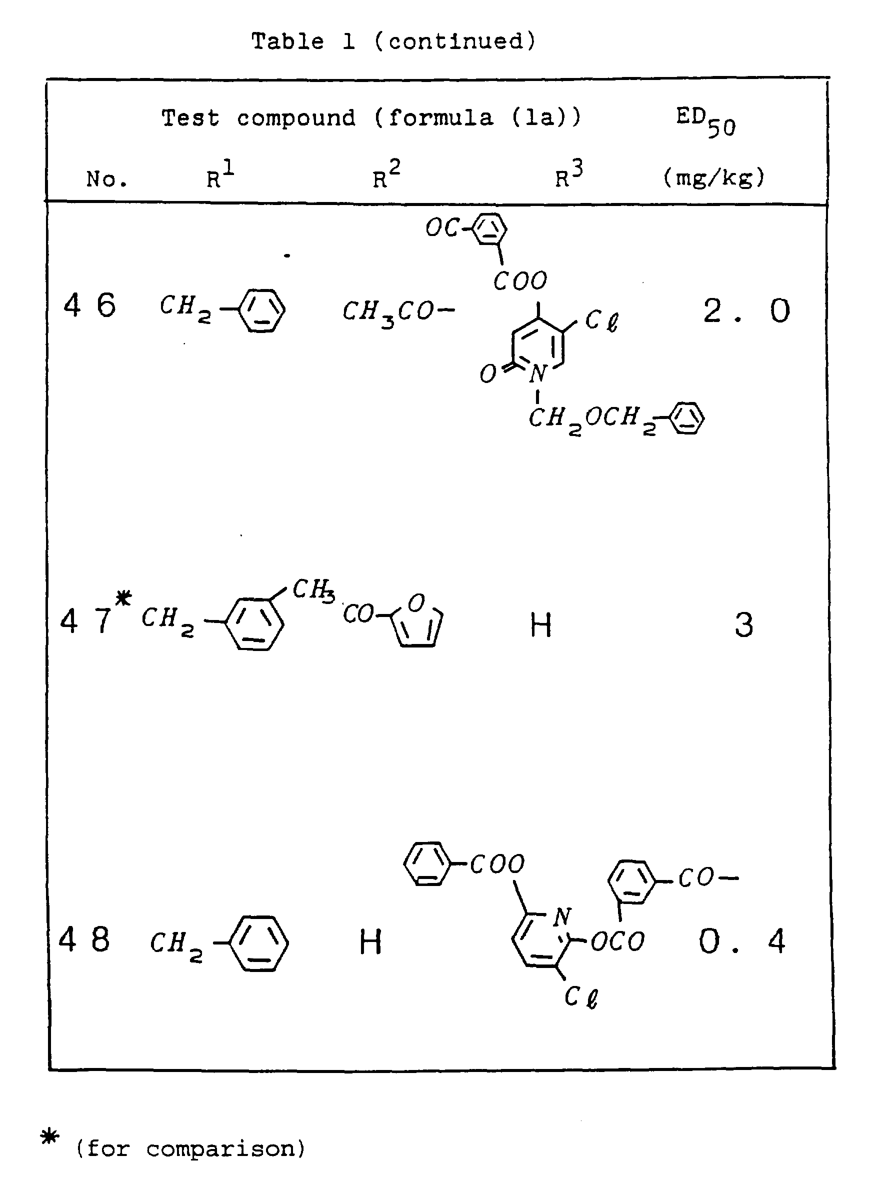

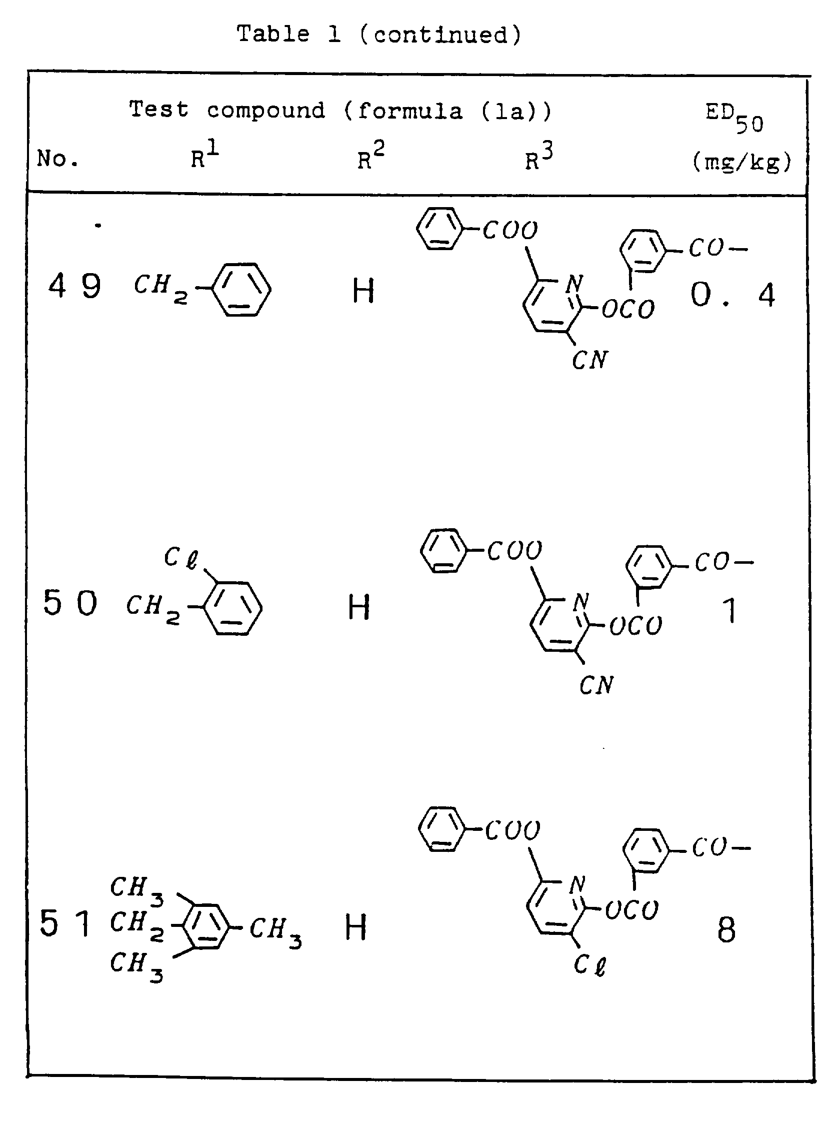

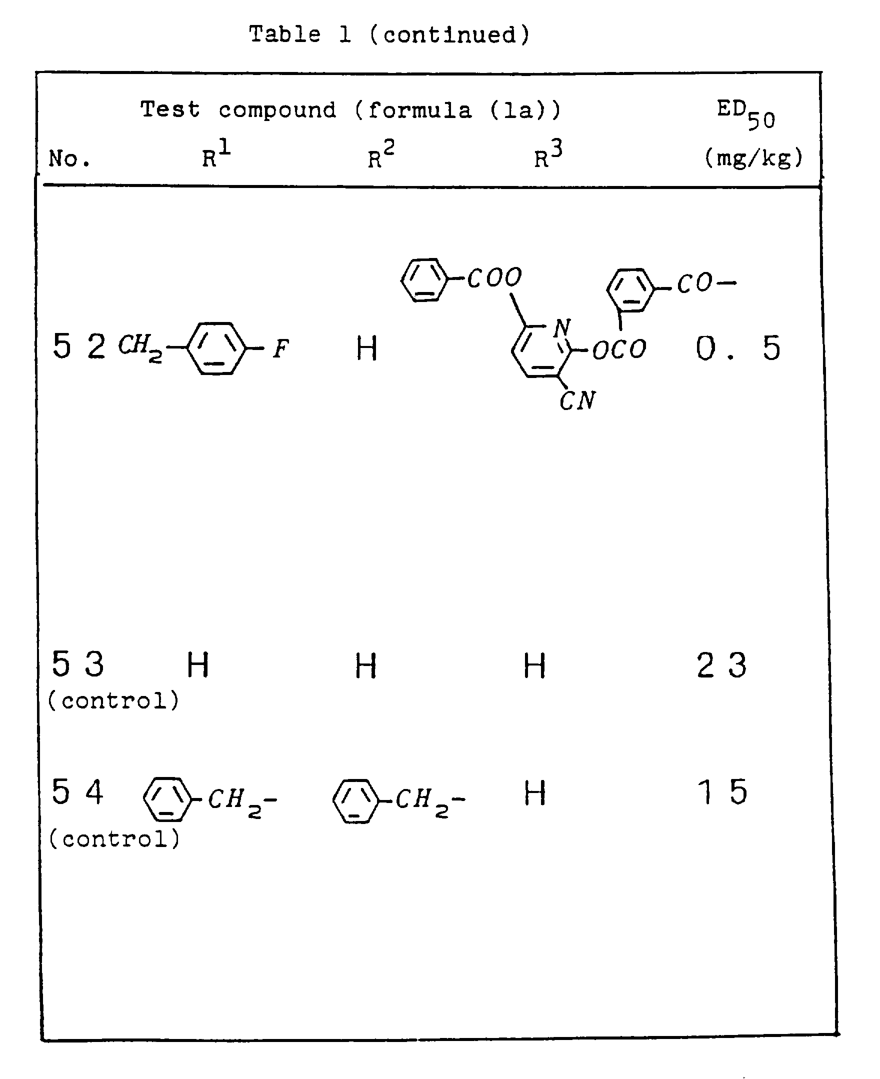

The compounds according to the present invention can be formulated following the Preparation Examples stated below:

**Table 2**

| Test Compound | LD₅₀ (mg/kg) |
|---|---|
| Compound of Example 2 (for comparison) | Over 1000 |
| Compound of Example 54 (for comparison) | Over 1000 |

### Preparation Example 1 (for comparison)

| | |
|---|---|
| Compound of Example 54 (for comparison) | 50 mg |
| Lactose | 97 mg |
| Crystalline cellulose | 50 mg |
| Magnesium stearate | 3 mg |

Capsules (200 mg each) were prepared which each had the foregoing composition.

### Preparation Example 2 (for comparison)

| | |
|---|---|
| Compound of Example 54 (for comparison) | 10 mg |
| Lactose | 184 mg |
| Crystalline cellulose | 100 mg |
| Magnesium stearate | 6 mg |

Capsules (300 mg each) were prepared which each had the foregoing composition.

### Preparation Example 3 (for comparison)

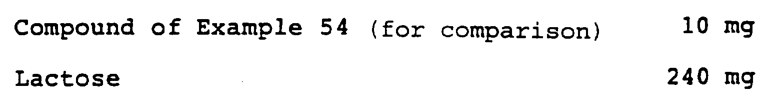

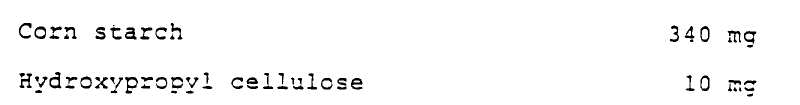

Granules (600 mg each wrapper) were prepared which each had the foregoing composition.

### Preparation Example 4

| | |
|---|---|
| Compound of Example 54 | 10 mg |
| Macrogol 300 | 500 mg |
| Distilled water for injection | (Suitable amount) |

An injection solution (5 ml per ampoule) was prepared which had the foregoing composition.

### Preparation Example 5

A thousand tablets for oral administration were prepared which each contained 10 mg of the compound obtained in Example 54 and which each had the following composition.

| | |
|---|---|
| Compound of Example 54 | 10 g |
| Lactose (Japanese Pharmacopeia) | 45 g |
| Corn starch (Japanese Pharmacopeia) | 25 g |
| Crystalline cellulose (Japanese Pharmacopeia) | 25 g |
| Methyl cellulose (Japanese Pharmacopeia) | 1.5 g |
| Magnesium stearate (Japanese Pharmacopeia) | 1 g |

The compound of Example 54, lactose, corn starch and crystalline cellulose were thoroughly mixed and the mixture was granulated with a 5 % aqueous solution of methyl cellulose. The granules thus obtained were passed through a 200-mesh sieve and carefully dried. The dry granules were passed through 200-mesh sieve and mixed with magnesium stearate. The mixture was pressed into tablets.

## Claims

1. A compound represented by the formula 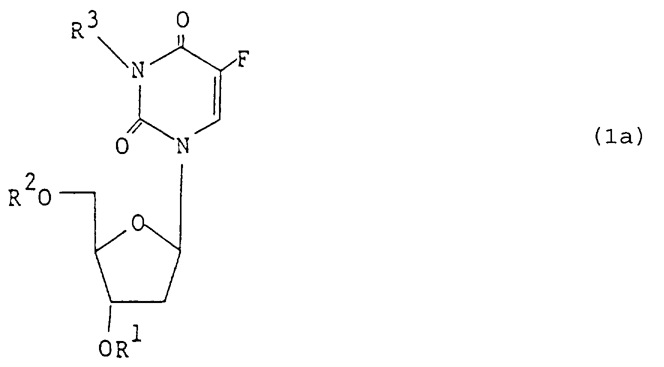 wherein one of R¹ and R is phenyl-lower alkyl group optionally having substituent selected from the group consisting of lower alkyl group, lower alkoxy group, halogen atom, carboxyl group, lower alkoxycarbonyl group and di(lower alkyl)amino group on the phenyl ring, phenyl-lower alkyl group having lower alkylenedioxy or phenyl group as the substituent on the phenyl ring, phenyl-lower alkenyl group or naphthyl-lower alkyl group, the other of R¹ and R is hydrogen atom or acyl group, and R³ is hydrogen atom, acyl group or tetrahydrofuranyl group, with the proviso that at least one of residues R¹, R and R³ is an acyl group, represented by the formula 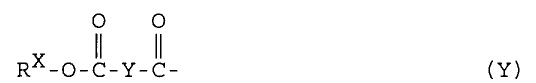 wherein R^{x} is pyridyl groups optionally substituted with 1 to 4 substituents selected from the group consisting of hydroxy group, oxo group, halogen atom, amino group, carboxyl group, cyano group, nitro group, carbamoyl group, lower alkylcarbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group and Y is arylene group.

2. A compound according to claim 1 wherein the acyl group represented by R¹, R and R³ is selected from the group consisting of:
(i) C₁-C₂₀ alkanoyl groups optionally substituted with the substituent selected from the group consisting of halogen atom, hydroxy group, lower alkoxy group, aryloxy group, substituted or unsubstituted aryl group, phenyl-lower alkoxycarbonyl group and lower alkylcarbamoyl group;
(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group;
(iii) 5- or 6- membered unsaturated hetero ring-carbonyl groups having nitrogen atom, sulfur atom or oxygen atom as the hetero atom;
(iv) carbonic acid ester residue such as aryloxycarbonyl groups, straight or branched-chain or cyclic alkoxycarbonyl groups;
(v) substituted or unsubstituted cycloalkylcarbonyl groups;
(vi) lower alkenyl (or lower alkynyl) carbonyl groups;
(vii) lower alkenyl (or lower alkynyl) oxycarbonyl groups; and
(viii) pyridyloxycarbonylarylenecarbonyl groups represented by the formula 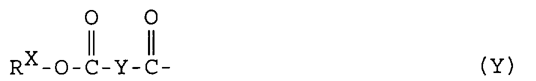 wherein R^{x} is pyridyl groups optionally substituted with 1 to 4 substituents selected from the group consisting of hydroxy group, oxo group, halogen atom, amino group, carboxy group, cyano group, nitro group, carbamoyl group, lower alkylcarbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group having acyl defined above under the items (i) to (vii) in the acyl moiety, and Y is arylene group.

3. A compound according to claim 2 wherein R¹ is phenyl-lower alkyl group optionally having substituent selected from the group consisting of lower alkyl group, lower alkoxy group, halogen atom, carboxyl group, lower alkoxycarbonyl group and di (lower alkyl)amino group on the phenyl ring, phenyl-lower alkyl group having lower alkylenedioxy or phenyl group as the substituent on the phenyl ring, phenyl-lower alkenyl group or naphthyl-lower alkyl group.

4. A compound according to claim 2 wherein R is phenyl-lower alkyl group optionally having substituent selected from the group consisting of lower alkyl group, lower alkoxy group, halogen atom, carboxyl group, lower alkoxycarbonyl group and di(lower alkyl)amino group on the phenyl ring, phenyl-lower alkyl group having lower alkylenedioxy or phenyl group as the substituent on the phenyl ring, phenyl-lower alkenyl group or naphthyl-lower alkyl group.

5. A compound according to claim 3 wherein R³ is an acyl group selected from the group consisting of those defined above under the items (i) to (viii).

6. A compound according to claim 3 wherein R³ is hydrogen atom or tetrahydrofuranyl group.

7. A compound according to claim 4 wherein R³ is an acyl group represented by the formula 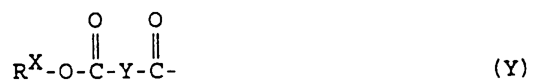 wherein R^{x} and Y are as defined above.

8. A compound according to claim 5 wherein R³ is an acyl group selected from the group consisting of those defined above under the items (i) to (vii).

9. A compound according to claim 8 wherein R¹ is benzyl group optionally having substituent selected from the group consisting of lower alkyl group, lower alkoxy group, halogen atom, carboxyl group, lower alkoxycarbonyl group and di(lower alkyl)amino group on the phenyl ring, or benzyl group having lower alkylenedioxy or phenyl group as the substituent on the phenyl ring.

10. A compound according to claim 5 wherein R³ is an acyl group represented by the formula 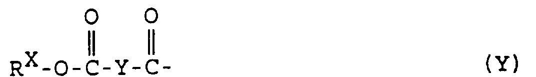 wherein R^{x} is a pyridyl group optionally substituented with 1 to 4 substituents selected from the group consisting of hydroxy group, oxo group, halogen atom, amino group, carboxyl group, cyano group, nitro group, carbamoyl group, lower alkylcarbamoyl group, carboxy-lower alkylcarbamoyl group, lower alkoxycarbonyl-lower alkylcarbamoyl group, phenyl carbamoyl group optionally substituted with 1 to 3 substituents selected from the group consisting of halogen atom, lower alkoxy group and lower alkyl group on the phenyl ring, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group having acyl defined above under the items (i) to (vii) in the acyl moiety, and Y is arylene group.

11. A compound according to claim 10 wherein R is hydrogen atom.

12. A compound according to claim 10 wherein R is an acyl group selected from the group consisting of those defined above under the items (i) to (vii).

13. A compound according to claim 11 or 12 wherein the group 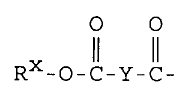 is a group represented by the formula 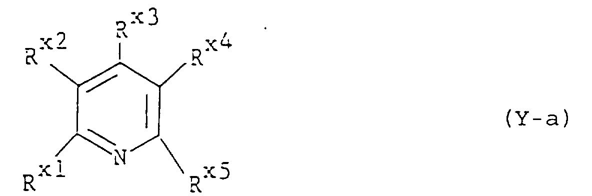 wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy; when at least one of R^{x1}, R^{x3} and R^{x5} is free hydroxy, the structure of 1-position on the pydridine ring can be 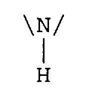 due to the keto-enol tautomerism, said hydrogen attached to the nitrogen being optionally substituted with a substituent selected from the group consisting of lower alkyl, tetrahydrofuranyl, tetrahydropyranyl, lower alkoxy-lower alkyl, phthalidyl, carbamoyl, lower alkoxycarbonyl-lower alkylcarbamoyl, phenyl-lower alkoxy-lower alkyl, phenylcarbamoyl which may have 1 to 3 substituents selected from the group consisting of halogen, lower alkoxy and lower alkyl on the phenyl ring, lower alkylcarbamoyl, carboxy-lower alkylcarbamoyl, lower alkylthio-lower alkyl and lower alkenyl, provided that at least one of R^{x1}, R^{x3} and R^{x5} represents hydroxy group and that the hydrogen of one hydroxyl group represented by R^{x1}, R^{x3} or R^{x5} is substituted with a group 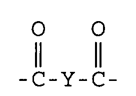 wherein Y is as defined above.

14. A compound according to claim 13 wherein the group 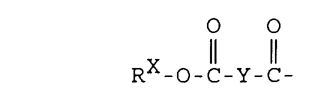 is a group represented by the formula 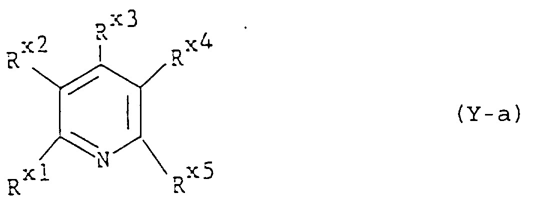 wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy, provided that at least one of R^{x1}, R^{x3} and R^{x5} represents hydroxy group and that the hydrogen of one hydroxyl group represented by R^{x1}, R^{x3} or R^{x5} is substituted with a group 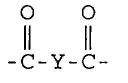 wherein Y is as defined above.

15. A compound according to claim 14 wherein R^{x1} is hydroxy, benzoyloxy, C₁-C₆ alkanoyloxy or furoyloxy, R^{x2} is hydrogen atom, one of R^{x3} and R^{x5} is hydrogen atom and the other of R^{x3} and R^{x5} is hydroxy, benzoyloxy, C₁-C₆ alkanoyloxy or furoyloxy, and R^{x4} is cyano group or halogen atom, provided that at least one of R^{x1}, R^{x3} and R^{x5} is hydroxy and that the hydrogen of one hydroxyl group represented by R^{x1}, R^{x3} or R^{x5} is substituted with a group 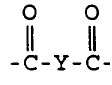 wherein Y is as defined above; and R¹ is benzyl group optionally having substituent selected from the group consisting of lower alkyl group, lower alkoxy group, halogen atom, carboxyl group, lower alkoxycarbonyl group and di(lower alkyl)amino group on the phenyl ring, or benzyl group having lower alkylenedioxy or phenyl group as the substituent on the phenyl ring.

16. A compound according to claim 15 wherein R¹ is benzyl group optionally substituted with halogen atom.

17. A compound according to claim 1 which is 3-[3-(6-benzoyloxy-3-cyano-2-pyridyloxycarbonyl)benzoyl]-3'-O-benzyl-2'-deoxy-5-fluorouridine.

18. A compound according to claim 1 which is selected from the group consisting of 3-[4-(6-benzoyloxy-3-cyano-2-pyridyloxycarbonyl)-3-furoyl]-3'-O-benzyl-2'-deoxy-5-fluorouridine, 3-[3-(6-benzoyloxy-3-cyano-2-pyridyloxycarbonyl)benzoyl]-3'-O-benzyl-2'-deoxy-5'-O-acetyl-5-fluorouridine, 3-[3-(6-benzoyloxy-3-cyano-2-pyridyloxycarbonyl)benzoyl]-3'-O-(p-fluorobenzyl)-2'-deoxy-5-fluorouridine and 3-[3-(6-benzoyloxy-3-cyano-2-pyridyloxycarbonyl)benzoyl]-3'-O-(o-chlorobenzyl)-2'-deoxy-5-fluorouridine.

19. A compound according to claim 1 which is selected from the group consisting of 3-[3-(6-benzoyloxy-3-chloro-2-pyridyloxycarbonyl)benzoyl]-3'-O-(p-chlorobenzyl)-2'-deoxy-5-fluorouridine, 3-[3-(6-benzoyloxy-3-chloro-2-pyridyloxycarbonyl)benzoyl]-3'-O-benzyl-2'-deoxy-5-fluorouridine, 3-[3-(4-benzoyloxy-5-chloro-2-pyridyloxycarbonyl)benzoyl]-3'-O-benzyl-2'-deoxy-5-fluorouridine, 3-[3-(4-acetyloxy-5-chloro-2-pyridyloxycarbonyl)benzoyl]-3'-O-benzyl-2'-deoxy-5-fluorouridine and 3-[3-(4-furoyloxy-5-chloro-2-pyridyloxycarbonyl)-benzoyl]-3'-O-benzyl-2'-deoxy-5-fluorouridine.

20. A compound having the formula (la) according to claim 1 for use in the treatment of cancer.

21. A pharmaceutical composition comprising a compound having the formula (la) according to claim 1 and a pharmaceutically acceptable carrier.

22. A compound according to claim 1 wherein R³ is an acyl group represented by the formula 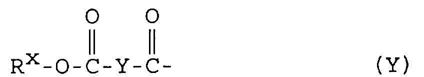 wherein R^{x} and Y are as defined in claim 2.

23. A compound according to claim 22 wherein one of R¹ and R is an acyl group selected from the group consisting of those defined under the items (i) to (viii) of claim 2.

24. A compound according to claim 23 wherein R¹ is phenyl-lower alkyl group optionally having substituent selected from the group consisting of lower alkyl group, lower alkoxy group, halogen atom, carboxyl group, lower alkoxycarbonyl group and di(lower alkyl)amino group on the phenyl ring, phenyl-lower alkyl group having lower alkylenedioxy or phenyl group as the substituent on the phenyl ring, phenyl-lower alkenyl group or naphthyl-lower alkyl group.

25. A compound according to claim 23 wherein R is phenyl-lower alkyl group optionally having substituent selected from the group consisting of lower alkyl group, lower alkoxy group, halogen atom, carboxyl group, lower alkoxycarbonyl group and di(lower alkyl)amino group on the phenyl ring, phenyl-lower alkyl group having lower alkylenedioxy or phenyl group as the substituent on the phenyl ring, phenyl-lower alkenyl group or naphthyl-lower alkyl group.

26. A compound selected from the group consisting of 3'-O-(4-bromobenzyl)-2'-deoxy-5-fluorouridine, 3'-O-(4-chlorobenzyl)-2'-deoxy-5-fluorouridine and 3'-O-(4-chlorobenzyl)-5'-O-acetyl-2'-deoxy-5-fluorouridine.

## Patentansprüche

1. Verbindung der Formel 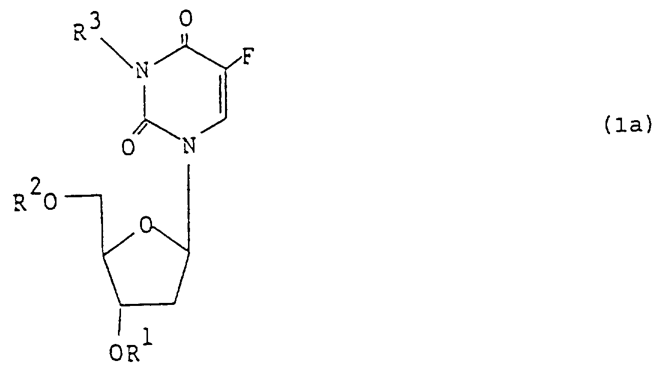 in der einer der Reste R¹ und R ein Phenylniederalkylrest, der gegebenenfalls einen Substituenten, ausgewählt aus einem Niederalkyl-, Niederalkoxy-, Halogen-, Carboxyl-, Niederalkoxycarbonyl- und Di(niederalkyl)aminorest, am Phenylring aufweist, ein Phenylniederalkylrest mit einem Alkylendioxyrest oder einer Phenylgruppe als Substituent am Phenylring, ein Phenylniederalkenyl- oder Naphthylniederalkylrest ist, der andere der Reste R¹ und R ein Wasserstoffatom oder Acylrest ist und R³ ein Wasserstoffatom, Acylrest oder eine Tetrahydrofuranylgruppe ist, mit der Maßgabe, daß mindestens einer der Reste R¹, R und R³ ein Acylrest, wiedergegeben durch die Formel 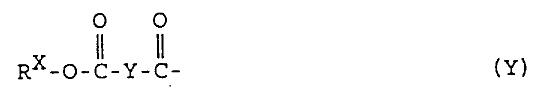 ist, in der R^{x} Pyridylgruppen sind, die gegebenenfalls mit 1 bis 4 Substituenten substituiert sind, ausgewählt aus einer Hydroxyl-, Oxogruppe, einem Halogenatom, einer Amino-, Carboxyl-, Cyano-, Nitro-, Carbamoylgruppe, einem Niederalkylcarbamoyl-, Carboxyniederalkylcarbamoyl-, Niederalkoxycarbonylniederalkylcarbamoyl-, Phenylcarbamoylrest, der gegebenenfalls mit 1 bis 3 Substituenten, ausgewählt aus einem Halogenatom, Niederalkoxy- und Niederalkylrest am Phenylring substituiert ist, einem Niederalkyl-, Niederalkenyl-, Niederalkoxycarbonyl-, Tetrahydrofuranyl-, Tetrahydropyranyl-, Niederalkoxyniederalkyl-, Niederalkylthioniederalkyl-, Phenylniederalkoxyniederalkyl-, Phthalidyl- und Acyloxyrest, und Y ein Arylenrest ist.

2. Verbindung nach Anspruch 1, in der der durch R¹, R und R³ wiedergegebene Acylrest ausgewählt ist aus den Resten, bestehend aus:
(i) C₁-C₂₀-Alkanoylresten, gegebenenfalls substituiert mit einem Substituenten, ausgewählt aus einem Halogenatom, einer Hydroxylgruppe, einem Niederalkoxy-, Aryloxy-, substituierten oder unsubstituierten Aryl-, Phenylniederalkoxycarbonyl- und Niederalkylcarbamoylrest,
(ii) Arylcarbonylresten, gegebenenfalls substituiert mit Niederalkylendioxyresten oder mit 1 bis 3 Substituenten, ausgewählt aus einem Halogenatom, Niederalkyl-, Niederalkoxy-, Carboxy-, Niederalkoxycarbonyl-, Nitro-, Cyano-, Phenylniederalkoxycarbonyl-, Hydroxyl-, Guanidyl-, Phenylniederalkoxy-, Amino- und niederalkylsubstituiertem Aminorest,
(iii) 5- oder 6-gliedrigen ungesättigten Heteroringcarbonylresten mit einem Stickstoff-, Schwefel- oder Sauerstoffatom als Heteroatom,
(iv) einem Carbonsäureesterrest, wie Aryloxycarbonylresten, linearen oder verzweigten oder cyclischen Alkoxycarbonylresten,
(v) substituierten oder unsubstituierten Cycloalkylcarbonylresten,
(vi) Niederalkenyl- (oder Niederalkinyl-)carbonylresten,
(vii) Niederalkenyl- (oder Niederalkinyl-)oxycarbonylresten, und
(viii) Pyridyloxycarbonylarylencarbonylresten, wiedergegeben durch die Formel 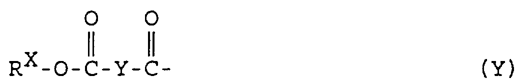 in der R^{x} Pyridylgruppen sind, die gegebenenfalls mit 1 bis 4 Substituenten substituiert sind, ausgewählt aus einer Hydroxyl-, Oxogruppe, einem Halogenatom, einer Amino-, Carboxyl-, Cyano-, Nitro-, Carbamoylgruppe, einem Niederalkylcarbamoyl-, Carboxyniederalkylcarbamoyl-, Niederalkoxycarbonylniederalkylcarbamoyl-, Phenylcarbamoylrest, der gegebenenfalls mit 1 bis 3 Substituenten, ausgewählt aus einem Halogenatom, Niederalkoxy- und Niederalkylrest am Phenylring substituiert ist, einem Niederalkyl-, Niederalkenyl-, Niederalkoxycarbonyl-, Tetrahydrofuranyl-, Tetrahydropyranyl-, Niederalkoxyniederalkyl-, Niederalkylthioniederalkyl-, Phenylniederalkoxyniederalkyl-, Phthalidylund Acyloxyrest, wobei der Acylrest vorstehend unter den Punkten (i) bis (vii) in der Acyleinheit definiert wurde und Y ein Arylenrest ist.

3. Verbindung nach Anspruch 2, in der R¹ ein Phenylniederalkylrest, der gegebenenfalls einen Substituenten, ausgewählt aus einem Niederalkyl-, Niederalkoxyrest, Halogenatom, einer Carboxylgruppe, einem Niederalkoxycarbonyl- und Di(niederalkyl)aminorest am Phenylring aufweist, ein Phenylniederalkylrest mit einem Niederalkylendioxyrest oder einer Phenylgruppe als Substituenten am Phenylring, ein Phenylniederalkenyl- oder Naphthylniederalkylrest ist.

4. Verbindung nach Anspruch 2, in der R ein Phenylniederalkylrest, der gegebenenfalls einen Substituenten, ausgewählt aus einem Niederalkyl-, Niederalkoxyrest, Halogenatom, einer Carboxylgruppe, einem Niederalkoxycarbonyl- und Di(niederalkyl)aminorest am Phenylring aufweist, ein Phenylniederalkylrest mit einem Niederalkylendioxyrest oder einer Phenylgruppe als Substituenten am Phenylring, ein Phenylniederalkenyl- oder Naphthylniederalkylrest ist.

5. Verbindung nach Anspruch 3, in der R³ ein Acylrest, ausgewählt aus den vorstehend unter den Punkten (i) bis (viii) definierten ist.

6. Verbindung nach Anspruch 3, in der R³ ein Wasserstoffatom oder eine Tetrahydrofuranylgruppe ist.

7. Verbindung nach Anspruch 4, in der R³ ein Acylrest, wiedergegeben durch die Formel 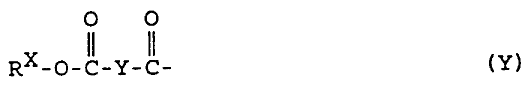 ist, in der R^{x} und Y die vorstehend angegebene Bedeutung haben.

8. Verbindung nach Anspruch 5, in der R³ ein Acylrest, ausgewählt aus den vorstehend unter den Punkten (i) bis (vii) definierten, ist.

9. Verbindung nach Anspruch 8, in der R¹ eine Benzylgruppe, die gegebenenfalls einen Substituenten, ausgewählt aus einem Niederalkyl-, Niederalkoxyrest, Halogenatom, einer Carboxylgruppe, einem Niederalkoxycarbonyl- und Di(niederalkyl)aminorest am Phenylring aufweist, oder eine Benzylgruppe mit einem Niederalkylendioxyrest oder einer Phenylgruppe als Substituent am Phenylring ist.

10. Verbindung nach Anspruch 5, in der R³ ein Acylrest, wiedergegeben durch die Formel 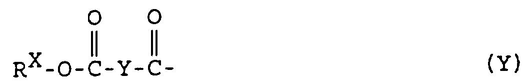 ist, in der R^{x} eine Pyridylgruppe ist, die gegebenenfalls mit 1 bis 4 Substituenten substituiert ist, ausgewählt aus einer Hydroxyl-, Oxogruppe, einem Halogenatom, einer Amino-, Carboxyl-, Cyano-, Nitro-, Carbamoylgruppe, einem Niederalkylcarbamoyl-, Carboxyniederalkylcarbamoyl-, Niederalkoxycarbonylniederalkylcarbamoyl-, Phenylcarbamoylrest, der gegebenenfalls mit 1 bis 3 Substituenten, ausgewählt aus einem Halogenatom, Niederalkoxy- und Niederalkylrest am Phenylring substituiert ist, einem Niederalkyl-, Niederalkenyl-, Niederalkoxycarbonyl-, Tetrahydrofuranyl-, Tetrahydropyranyl-, Niederalkoxyniederalkyl-, Niederalkylthioniederalkyl-, Phenylniederalkoxyniederalkyl-, Phthalidyl- und Acyloxyrest, wobei der Acylrest vorstehend unter den Punkten (i) bis (vii) in der Acyleinheit definiert wurde und Y ein Arylenrest ist.

11. Verbindung nach Anspruch 10, in der R ein Wasserstoffatom ist.

12. Verbindung nach Anspruch 10, in der R ein Acylrest, ausgewählt aus den vorstehend unter den Punkten (i) bis (vii) definierten, ist.

13. Verbindung nach Anspruch 11 oder 12, in der der Rest 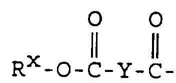 ein Rest, wiedergegeben durch die Formel 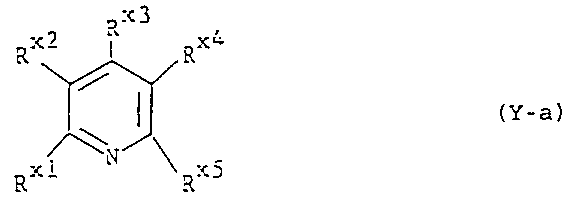 ist, in der R^{x1} eine Hydroxylgruppe oder ein Acyloxyrest ist, R^{x2} und R^{x4} jeweils Wasserstoff-, Halogen-, Amino-, Carboxyl-, Carbamoyl-, Cyano-, Nitro-, Niederalkyl-, Niederalkenyl- oder Niederalkoxycarbonylreste sind, R^{x3} und R^{x5} jeweils ein Wasserstoffatom, eine Hydroxylgruppe oder ein Acyloxyrest sind, wenn mindestens einer der Reste R^{x1}, R^{x3} und R^{x5} eine freie Hydroxylgruppe ist, die Struktur der 1-Stellung am Pyridinring durch die Keto-Enol-Tautomerie 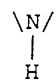 sein kann, wobei das an das Stickstoffatom gebundene Wasserstoffatom gegebenenfalls durch einen Substituenten ersetzt ist, ausgewählt aus einem Niederalkyl-, Tetrahydrofuranyl-, Tetrahydropyranyl-, Niederalkoxyniederalkyl-, Phthalidyl-, Carbamoyl-, Niederalkoxycarbonylniederalkylcarbamoyl-, Phenylniederalkoxyniederalkyl-, Phenylcarbamoylrest, der 1 bis 3 Substituenten, ausgewählt aus einem Halogenatom, Niederalkoxy- und Niederalkylrest am Phenylring aufweisen kann, einem Niederakylcarbamoyl-, Carboxyniederalkylcarbamoyl-, Niederalkylthioniederalkyl- und Niederalkyletlres, mit der Maßgabe, daß mindestens einer der Reste R^{x1}, R^{x3} und R^{x5} eine Hydroxylgruppe darstellt und daß das Wasserstoffatom der einen durch R^{x1}, R^{x3} oder R^{x5} wiedergegebenen Hydroxylgruppe durch einen Rest 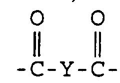 ersetzt ist, in dem Y die vorstehend angegebene Bedeutung hat.

14. Verbindung nach Anspruch 13, in der der Rest 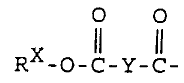 ein durch Formel 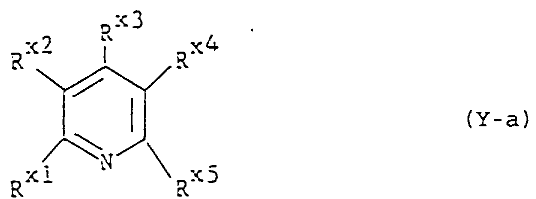 wiedergegebener Rest ist, in der R^{x1} eine Hydroxylgruppe oder ein Acyloxyrest ist, R^{x2} und R^{x4} jeweils Wasserstoff-, Halogen-, Amino-, Carboxyl-, Carbamoyl-, Cyano-, Nitro-, Niederalkyl-, Niederalkenyl- oder Niederalkoxycarbonylreste sind, R^{x3} und R^{x5} jeweils ein Wasserstoffatom, eine Hydroxylgruppe oder ein Acyloxyrest sind, mit der Maßgabe, daß mindestens einer der Reste R^{x1}, R^{x3} und R^{x5} eine Hydroxylgruppe darstellt und daß das Wasserstoffatom der einen durch R^{x1}, R^{x3} oder R^{x5} wiedergegebenen Hydroxylgruppe durch einen Rest 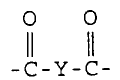 ersetzt ist, in dem Y die vorstehend angegebene Bedeutung hat.

15. Verbindung nach Anspruch 14, in der R^{x1} eine Hydroxyl-, Benzoyloxy-, C₁-C₆-Alkanoyloxy- oder Furoyloxygruppe ist, R^{x2} ein Wasserstoffatom ist, einer der Reste R^{x3} und R^{x5} ein Wasserstoffatom ist und der andere der Reste R^{x3} und R^{x5} eine Hydroxyl-, Benzoyloxy-, C₁-C₆-Alkanoyloxy- oder Furoyloxygruppe ist und R^{x4} eine Cyanogruppe oder ein Halogenatom ist, mit der Maßgabe, daß mindestens einer der Reste R^{x1}, R^{x3} und R^{x5} eine Hydroxylgruppe ist und daß das Wasserstoffatom der einen durch R^{x1}, R^{x3} oder R^{x5} wiedergegebenen Hydroxylgruppe durch einen Rest 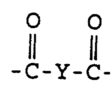 ersetzt ist, in dem Y die vorstehend angegebene Bedeutung hat, und R¹ eine Benzylgruppe, die gegebenenfalls einen Substituenten, ausgewählt aus einem Niederalkyl-, Niederalkoxy-, Halogen-, Carboxyl-, Niederalkoxycarbonyl- und Di(niederalkyl)aminorest am Phenylring aufweist, oder eine Benzylgruppe mit einem Niederalkylendioxyrest oder einer Phenylgruppe als Substituenten am Phenylring ist.

16. Verbindung nach Anspruch 15, in der R¹ eine gegebenenfalls mit einem Halogenatom substituierte Benzylgruppe ist.

17. Verbindung nach Anspruch 1, nämlich 3-[3-(6-Benzoyloxy-3-cyano-2-pyridyloxycarbonyl)benzoyl] -3'-O-benzyl-2'-deoxy-5-fluoruridin.

18. Verbindung nach Anspruch 1, ausgewählt aus 3-[4-(6-Benzoyloxy-3-cyano-2-pyridyloxycarbonyl)-3-furoyl]-3'-O-benzyl-2'-deoxy-5-fluoruridin, 3-[3-(6-Benzoyloxy-3-cyano-2-pyridyloxycarbonyl)benzoyl]-3 -O-benzyl-2'-deoxy-5'-O-acetyl-5-fluoruridin, 3-[3-(6-Benzoyloxy-3-cyano-2-pyridyloxycarbonyl)benzoyl]-3'-O-(p-fluorbenzyl)-2'-deoxy-5-fluoruridin und 3-[3-(6-Benzoyloxy-3-cyano-2-pyridyloxycarbonyl)benzoyl] -3,-O-(o-chlorbenzyl)-2'-deoxy-5-fluoruridin.

19. Verbindung nach Anspruch 1, ausgewählt aus 3-[3-(6-Benzoyloxy-3-chlor-2-pyridyloxycarbonyl)benzoyl]-3'-O-(p-chlorbenzyl)-2'-deoxy-5-fluoruridin, 3-[3-(6-Berzoyloxy-3-chlor-2-pyridyloxycarbonyl)berzoyl] -3'-O-benzyl-2'-deoxy-5-fluoruridin, 3-[3-(4-Benzoyloxy-5-chlor-2-pyridyloxycarbonyl)benzoyl]-3'-O-benzyl-2'-deoxy-5-fluoruridin, 3-[3-(4-Acetyloxy-5-chlor-2-pyridyloxycarbonyl)-benzoyl]-3'-O-benzyl-2'-deoxy-5-fluoruridin und 3-[3-(4-Furoyloxy-5-chlor-2-pyridyloxycarbonyl)benzoyl]-3'-O-benzyl-2'-deoxy-5-fluoruridin.

20. Verbindung der Formel (1a) gemäß Anspruch 1 zur Verwendung bei der Behandlung von Krebs.

21. Arzneimittel umfassend eine Verbindung der Formel (la) gemäß Anspruch 1 und einen pharmazeutisch verträglichen Träger.

22. Verbindung nach Anspruch 1, in der R³ ein durch die Formel 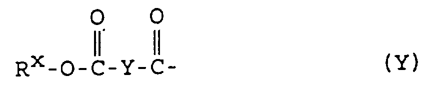 wiedergegebener Acylrest ist, in der R^{x} und Y die in Anspruch 2 angegebene Bedeutung haben.

23. Verbindung nach Anspruch 22, in der einer der Reste R¹ und R ein Acylrest, ausgewählt aus den unter den Punkten (i) bis (viii) von Anspruch 2 definierten Resten, ist.

24. Verbindung nach Anspruch 23, in der R¹ ein Phenylniederalkylrest, der gegebenenfalls einen Substituenten, ausgewählt aus Niederalkyl-, Niederalkoxy-, Halogen-, Carboxyl-, Niederalkoxycarbonyl- und Di(niederalkyl)aminoresten am Phenylring aufweist, ein Phenylniederalkylrest mit einem Niederalkylendioxyrest oder einer Phenylgruppe als Substituenten am Phenylring, ein Phenylniederalkenyl- oder Naphthylniederalkylrest ist.

25. Verbindung nach Anspruch 23, in der R ein Phenylniederalkylrest, der gegebenenfalls einen Substituenten, ausgewählt aus Niederalkyl-, Niederalkoxy-, Halogen-, Carboxyl-, Niederalkoxycarbonyl- und Di(niederalkyl)aminoresten am Phenylring aufweist, ein Phenylniederalkylrest mit einem Niederalkylendioxyrest oder einer Phenylgruppe als Substituenten am Phenylring, ein Phenylniederalkenyl- oder Naphthylniederalkylrest ist.

26. Verbindung, ausgewählt aus 3'-O-(4-Brombenzyl)-2'-deoxy-5-fluoruridin, 3'-O-(4-Chlorbenzyl)-2'-deoxy-5-fluoruridin und 3'-O-(4-Chlorbenzyl)-5'-O-acetyl-2'-deoxy-5-fluoruridin.

## Revendications

1. Composé représenté par la formule: 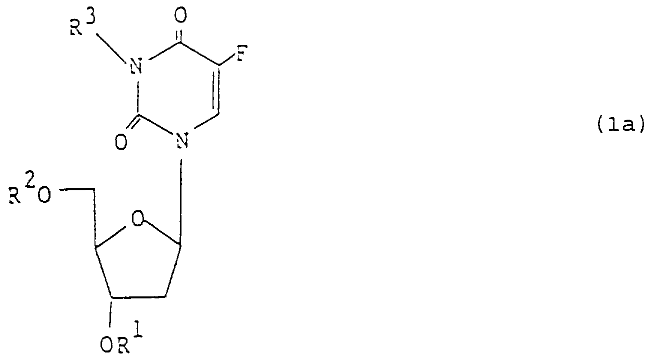 dans laquelle l'un des groupes R¹ ou R est un groupe phénylalkyle inférieur ayant éventuellement un substituant choisi au sein du groupe comprenant un groupe alkyle inférieur, un groupe alcoxy inférieur, un atome d'halogène, un groupe carboxyle, un groupe alcoxycarbonyle inférieur, et un groupe dialkylamino inférieur sur le cycle phényle, un groupe phénylalkyle inférieur comportant un groupe alkylènedioxy ou un groupe phényle comme substituant sur le cycle phényle, un groupe phénylalkényle inférieur, ou un groupe naphtylalkyle inférieur, l'autre des groupes R¹ ou R est un atome d'hydrogène ou un groupe acyle, et R³ est un atome d'hydrogène, un groupe acyle,ou un groupe tétrahydrofuranyle, à condition qu'au moins un des résidus R¹, R, et R³ soit un groupe acyle représenté par la formule : 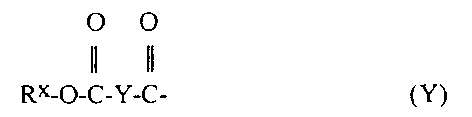 dans laquelle R^{x} représente des groupes pyridyle éventuellement substitués par 1 à 4 substituants choisis dans le groupe comprenant le groupe hydroxy, le groupe oxo, l'atome d'halogène, le groupe amino, le groupe carboxyle, le groupe cyano, le groupe nitro, le groupe carbamoyle, le groupe alkylcarbamoyle inférieur, le groupe carboxy(alkyle inférieur)-carbamoyle, le groupe (alcoxycarbonyle inférieur)-(alkyle inférieur)carbamoyle, le groupe phénylcarbamoyle éventuellement substitué par 1 à 3 substituants choisis au sein du groupe comprenant l'atome d'halogène, le groupe alcoxy inférieur, et le groupe alkyle inférieur sur le cycle phényle, le groupe alkyle inférieur, le groupe alkényle inférieur, le groupe alcoxycarbonyle inférieur, le groupe tétrahydrofuranyle, le groupe tétrahydropyranyle, le groupe (alcoxy inférieur)alkyle inférieur, le groupe (alkylthio inférieur)alkyle inférieur, le groupe phényl(alcoxy inférieur)alkyle inférieur, le groupe phtalidyle, et le groupe acyloxy, et Y est un groupe arylène.

2. Composé selon la revendication 1, dans lequel le groupe acyle représenté par R¹, R, et R³, est choisi au sein du groupe comprenant :
(i) les groupes alcanoyle C₁-C₂₀, éventuellement substitués par le substituant choisi au sein du groupe comprenant l'atome d'halogène, le groupe hydroxy, le groupe alcoxy inférieur, le groupe aryloxy, le groupe aryle substitué ou non substitué, le groupe phénylalcoxycarbonyle inférieur, et le groupe alkylcarbamoyle inférieur;
(ii) les groupes arylcarbonyle éventuellement substitués par un groupe alkylènedioxy inférieur, ou par 1 à 3 substituants choisis au sein du groupe comprenant l'atome d'halogène, le groupe alkyle inférieur, le groupe alcoxy inférieur, le groupe carboxy, le groupe alcoxycarbonyle inférieur, le groupe nitro, le groupe cyano, le groupe phénylalcoxycarbonyle inférieur, le groupe hydroxy, le groupe guanidyle, le groupe phénylalcoxy inférieur, le groupe amino, et le groupe amino substitué par un groupe alkyle inférieur;
(iii) les groupes carbonyle hétérocycliques non saturés à 5 ou 6 atomes, comportant un atome d'azote, un atome de soufre, ou un atome d'oxygène comme hétéroatome;
(iv) les résidus d'esters d'acide carbonique, tels que les groupes aryloxycarbonyle, les groupes alcoxycarbonyle à chaîne droite ou ramifiée, ou cycliques ;
(v) les groupes cycloalkylcarbonyle substitués ou non substitués;
(vi) les groupes alkénylcarbonyle inférieurs, ou alkynylcarbonyle inférieurs;
(vii) les groupes alkényloxycarbonyle inférieurs, ou alkynyloxycarbonyle inférieurs;
(viii) les groupes pyridyloxycarbonylarylénecarbonyle représentés par la formule : 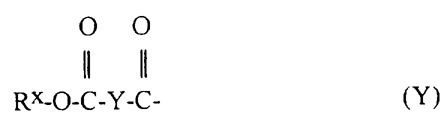 dans laquelle R^{x} représente des groupes pyridyle éventuellement substitués par 1 à 4 substituants choisis dans le groupe comprenant le groupe hydroxy, le groupe oxo, l'atome d'halogène, le groupe amino, le groupe carboxy, le groupe cyano, le groupe nitro, le groupe carbamoyle, le groupe alkylcarbamoyle inférieur, le groupe carboxyalkylcarbamoyle inférieur, le groupe (alcoxycarbonyle inférieur)alkylcarbamoyle inférieur, le groupe phénylcarbamoyle éventuellement substitué par 1 à 3 substituants choisis au sein du groupe comprenant l'atome d'halogène, le groupe alcoxy inférieur, et le groupe alkyle inférieur sur le cycle phényle, le groupe alkyle inférieur, le groupe alkényle inférieur, le groupe alcoxycarbonyle inférieur, le groupe tétrahydrofuranyle, le groupe tétrahydropyranyle, le groupe (alcoxy inférieur)alkyle inférieur, le groupe (alkylthio inférieur)alkyle inférieur, le groupe phényl(alcoxy inférieur)-alkyle inférieur, le groupe phtalidyle, et le groupe acyloxy comportant dans l'entité acyle, les groupes acyle définis plus haut dans les rubriques (i) à (vii), et Y est un groupe arylène.

3. Composé selon la revendication 2, dans lequel R¹ est un groupe phénylalkyle inférieur ayant éventuellement un substituant choisi au sein du groupe comprenant le groupe alkyle inférieur, le groupe alcoxy inférieur, l'atome d'halogène, le groupe carboxyle, le groupe alcoxycarbonyle inférieur, et le groupe diaminoalkyle inférieur sur le cycle phényle, un groupe phénylalkyle inférieur comportant un groupe alkylènedioxy inférieur ou phényle en tant que substituant sur le cycle phényle, un groupe phénylalkényle inférieur, ou un groupe napthylalkyle inférieur.

4. Composé selon la revendication 2, dans lequel R est un groupe phénylalkyle inférieur ayant éventuellement un substituant choisi au sein du groupe comprenant le groupe alkyle inférieur, le groupe alcoxy inférieur, l'atome d'halogène, le groupe carboxyle, le groupe alcoxycarbonyle inférieur, et le groupe diaminoalkyle inférieur sur le cycle phényle, un groupe phénylalkyle inférieur comportant un groupe alkylènedioxy inférieur ou phényle en tant que substituant sur le cycle phényle, un groupe phénylalkényle inférieur, ou un groupe napthylalkyle inférieur.

5. Composé selon la revendication 3, dans lequel R³ est un groupe acyle choisi au sein du groupe comprenant ceux définis plus haut dans les rubriques (i) à (viii).

6. Composé selon la revendication 3, dans lequel R³ est un atome d'hydrogène, ou un groupe tétrahydrofuranyle.

7. Composé selon la revendication 4 dans lequel R³ est un groupe acyle représenté par la formule : 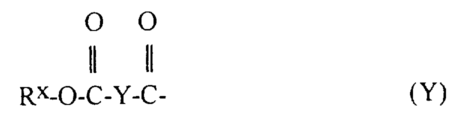 dans laquelle R^{x} et Y sont tels que définis plus haut.

8. Composé selon la revendication 5, dans lequel R³ est un groupe acyle choisi au sein du groupe comprenant ceux définis plus haut sous les rubriques (i) à (vii).

9. Composé selon la revendication 8, dans lequel R¹ est un groupe benzyle ayant éventuellement un substituant choisi au sein du groupe comprenant le groupe alkyle inférieur, le groupe alcoxy inférieur, l'atome d'halogène, le groupe carboxyle, le groupe alcoxycarbonyle inférieur, et le groupe dialkylamino inférieur sur le cycle phényle, ou un groupe benzyle ayant un groupe alkylénedioxy inférieur ou phényle en tant que substituant du cycle phényle.

10. Composé selon la revendication 5, dans lequel R³ est un groupe acyle représenté par la formule : 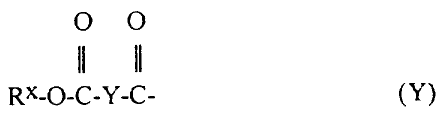 dans laquelle R^{x} représente un groupe pyridyle éventuellement substitués par 1 à 4 substituants choisis dans le groupe comprenant le groupe hydroxy, le groupe oxo, l'atome d'halogène, le groupe amino, le groupe carboxyle, le groupe cyano, le groupe nitro, le groupe carbamoyle, le groupe alkylcarbamoyle inférieur, le groupe carboxy(alkyle inférieur)-carbamoyle, le groupe (alcoxycarbonyle inférieur)-(alkyle inférieur)carbamoyle, le groupe phénylcarbamoyle éventuellement substitué par 1 à 3 substituants choisis au sein du groupe comprenant l'atome d'halogène, le groupe alcoxy inférieur, et le groupe alkyle inférieur sur le cycle phényle, le groupe alkyle inférieur, le groupe alkényle inférieur, le groupe alcoxycarbonyle inférieur, le groupe tétrahydrofuranyle, le groupe tétrahydropyranyle, le groupe (alcoxy inférieur)alkyle inférieur, le groupe (alkylthio inférieur)alkyle inférieur, le groupe phényl(alcoxy inférieur)alkyle inférieur, le groupe phtalidyle, et le groupe acyloxy comportant dans l'entité acyle, un acyle défini plus haut sous les rubriques (i) à (vii) , et Y est un groupe arylène.

11. Composé selon la revendication 10, dans lequel R est un atome d'hydrogène.

12. Composé selon la revendication 10, dans lequel R est un groupe acyle choisi au sein du groupe comprenant ceux définis plus haut sous les rubriques (i) à (vii).

13. Composé selon la revendication 11 ou la revendication 12, dans lequel le groupe 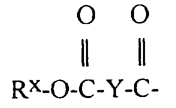 est un groupe représenté par la formule : 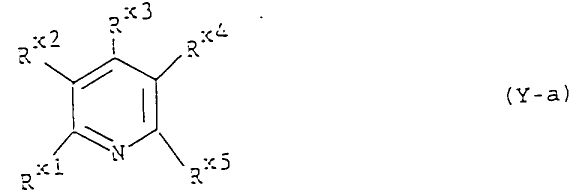 dans laquelle R^{x1} est un groupe hydroxy ou acyloxy ; R^{x2} et R^{x4} sont chacun un atome d'hydrogène, un halogène, un groupe amino, carboxyle, carbamoyle, cyano, nitro, alkyle inférieur, alkényle inférieur, ou alcoxycarbonyle inférieur; R^{x3} et R^{x5} sont chacun un atome d'hydrogène, ou un groupe hydroxy ou acyloxy; lorsque l'un au moins de R^{x1}, R^{x3}, et R^{x5} est un groupe hydroxy libre, la structure en position 1 du cycle pyridine peut être 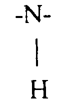 en raison de la tautomérie céto-énol, ledit atome d'hydrogène lié à l'atome d'azote étant éventuellement substitué par un substituant choisi au sein du groupe comprenant les groupes alkyle inférieur, tétrahydrofuranyle, tétrahydropyranyle, (alcoxy inférieur)alkyle inférieur, phtalidyle, carbamoyle, (alcoxycarbonyl inférieur)alkylcarbamoyle inférieur, phényl(alcoxy inférieur)alkyle inférieur, phénylcarbamoyle pouvant avoir 1 à 3 substituants choisis au sein du groupe comprenant l'halogène, le groupe alcoxy inférieur et alkyle inférieur sur le cycle phényle, alkylcarbamoyle inférieur, le groupe carboxy(alkyle inférieur)carbalnoyle, (alkylthio inférieur)alkyle inférieur, et alkényle inférieur, à condition que l'un au moins de R^{x1}, R^{x3}, et R^{x5}, représente un groupe hydroxy, et que l'hydrogène d'un groupe hydroxyle représenté par R^{x1}, R^{x3}, ou R^{x5}, soit substitué par un groupe 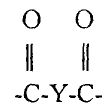 dans lequel Y est tel que défini plus haut.

14. Composé selon la revendication 13, dans lequel le groupe 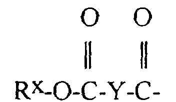 est un groupe représenté par la formule : 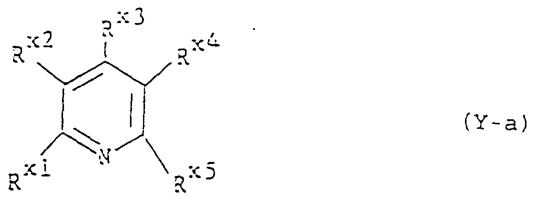 dans laquelle R^{x1} est un groupe hydroxy ou acyloxy ; R^{x2} et R^{x4} sont chacun un atome d'hydrogène, un halogène, un groupe amino, carboxyle, carbamoyle, cyano, nitro, alkyle inférieur, alkényle inférieur, ou alcoxycarbonyle inférieur; R^{x3} et R^{x5} sont chacun un atome d'hydrogène, ou un groupe hydroxy ou acyloxy; à condition que l'un au moins de R^{x1}, R^{x3}, et R^{x5} représente un groupe hydroxy, et que l'hydrogène d'un groupe hydroxyle représenté par R^{x1}, R^{x3}, ou R^{x5}, soit substitué par un groupe 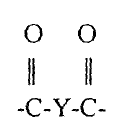 dans lequel Y est tel que défini plus haut.

15. Composé selon la revendication 14, dans lequel R^{x1} est un groupe hydroxy, benzoyloxy, alcanoyl C₁-C₆ oxy, ou furoyloxy, R^{x2} est un atome d'hydrogène, l'un des groupes R^{x3} ou R^{x5} est un atome d'hydrogène, et l'autre des groupes R^{x3} ou R^{x5} est un groupe hydroxy, benzoyloxy, alcanoyl C₁-C₆ oxy, ou furoyloxy, et R^{x4} est un groupe cyano ou un atome d'halogène, à condition qu'au moins un de R^{x1}, R^{x3}, ou R^{x5}, soit un groupe hydroxy, et que l'hydrogène d'un groupe hydroxyle représenté par R^{x1}, R^{x3}, ou R^{x5}, soit substitué par un groupe 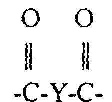 dans lequel Y est tel que défini plus haut; et R¹ est un groupe benzyle ayant éventuellement un substituant choisi au sein du groupe comprenant un groupe alkyle inférieur, un groupe alcoxy inférieur, un atome d'halogène, un groupe carboxyle, un groupe alcoxycarbonyle inférieur, et un groupe dialkylamino inférieur sur le cycle phényle, ou un groupe benzyle ayant comme substituant sur le cycle phényle, un groupe alkylènedioxy inférieur, ou un groupe phényle.

16. Composé selon la revendication 15, dans lequel R¹ est un groupe benzyle éventuellement substitué par un atome d'halogène.

17. Composé selon la revendication 1, qui est le 3-[3-(6-benzoyloxy-3-cyano-2-pyridyloxycarbonyl )benzoyl]-3'-O-benzyl-2'-désoxy-5-fluorouridine.

18. Composé selon la revendication 1, qui est choisi au sein du groupe comprenant:
- la 3-[4-(6-benzoyloxy-3-cyano-2-pyridyloxycarbonyl)-3-furoyl]-3'-O-benzyl-2'-désoxy-5-fluorouridine,
- la 3-[3-(6-benzoyloxy-3-cyano-2-pyridyloxycarbonyl)benzoylj-3'-O-benzyl-2'-désoxy-5'-O-acétyl-5-fluorouridine.
- la 3-[3-(6-benzoyloxy-3-cyano-2-pyridyloxycarbonyl)benzoyl]-3'-O-(p-fluorobenzyl)-2'-désoxy-5-fluorouridine, et
- la 3-[3-(6-benzoyloxy-3-cyano-2-pyridyloxycarbonyl)benzoyl]-3'-O-(o-chlorobenzyl)-2'-désoxy-5-fluorouridine.

19. Composé selon la revendication 1, qui est choisi au sein du groupe comprenant
- la 3-[3-(6-benzoyloxy-3-chloro-2-pyridyloxycarbonyl)benzoyl]-3'-O-(p-chlorobenzyl)-2'-désoxy-5-fluorouridine,
- la 3-[3-(6-benzoyloxy-3-chloro-2-pyridyloxycarbonyl)benzoyl]-3'-O-benzyl-2'-désoxy-5-fluorouridine
- la 3-[3-(4-benzoyloxy-5-chloro-2-pyridyloxycarbonyl )benzoyl]-3'-O-benzyl-2'-désoxy-5-fluoroundine,
- la 3-[3-(4-acétyloxy-5-chloro-2-pyridyloxycarbonyl)benzoyl]-3'-O-benzyl-2'-désoxy-5-fluorouridine, et
- la 3-[3-(4-furoyloxy-5-chloro-2-pyridyloxycarbonyl)benzoyl]-3'-O-benzyl-2'-désoxy-5-fluorouridine.

20. Composé ayant la formule (la) selon la revendication 1, pour l'utilisation dans le traitement du cancer.

21. Composition pharmaceutique comprenant un composé ayant la formule (1a) selon la revendication 1, et un véhicule pharmaceutiquement acceptable.

22. Composé selon la revendication 1, dans lequel R³ est un groupe acyle représenté par la formule: 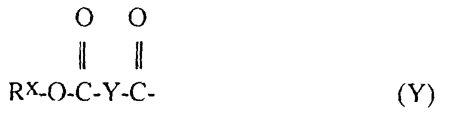 dans laquelle R^{x} et Y sont tels que définis dans la revendication 2.

23. Composé selon la revendication 22 dans lequel un des groupes R¹ ou R est un groupe acyle choisi au sein du groupe comprenant ceux définis sous les rubriques (i) à (viii) de la revendication 2.

24. Composé selon la revendication 23 dans lequel R¹ est un groupe phénylalkyle inférieur ayant éventuellement un substituant choisi au sein du groupe comprenant un groupe alkyle inférieur, un groupe alcoxy inférieur, un atome d'halogène, un groupe carboxyle, un groupe alcoxycarbonyle inférieur, et un groupe dialkylamino inférieur sur le cycle phényle, un groupe phénylalkyle inférieur ayant un groupe alkylènedioxy inférieur ou phényle comme substituant sur le cycle phényle, un groupe phénylalkényle inférieur, ou un groupe naphtylalkyle inférieur.

25. Composé selon la revendication 23, dans lequel R est un groupe phénylalkyle inférieur ayant éventuellement un substituant choisi au sein du groupe comprenant un groupe alkyle inférieur, un groupe alcoxy inférieur, un atome d'halogène, un groupe carboxyle, un groupe alcoxycarbonyle inférieur, et un groupe dialkylamino inférieur sur le cycle phényle, un groupe phénylalkyle inférieur ayant un groupe alkylènedioxy inférieur ou phényle comme substituant sur le cycle phényle, un groupe phénylalkényle inférieur, ou naphtylalkyle inférieur.

26. Composé choisi au sein du groupe comprenant la 3'-O-(4-bromobenzyl)-2'-désoxy-5-fluorouridine, la 3'-O-(4-chlorobenzyl)-2'-désoxy-5-fluorouridine, et la 3'-O-(4-chlorobenzyl)-5'-O-acétyl-2'-désoxy-5-fluorouridine.
